# EUROPEAN PATENT APPLICATION

(11) **EP 1 236 468 A1**
(43) Date of publication of application: **04.09.2002**
(21) Application number: 02002612.6
(22) Date of filing: 05.02.2002
(51) Int. Cl.: A61K 31/18, A61K 31/255, A61K 31/27

(54) **Sulfonylaminocarbonyl derivatives for the treatment of nuclear factor-kappa B mediated diseases and disorders**

(30) Priority: 12.02.2001 US 268203 P
(71) Applicant: WARNER-LAMBERT COMPANY, Morris Plains New Jersey 07950 (US)
(72) Inventor: Cornicelli, Joseph Anthony, Ann Arbor, Michigan 48108 (US); Karathanasis, Sotirios K., Saline, Michigan 48176 (US)
(74) Representative: Mansmann, Ivo

(57) **Abstract**

The present invention provides a method of treating a disease or a disorder responsive to inhibition of nuclear factor-κB transcription factors comprising administering to a patient in need thereof a sulfonylaminocarbonyl derivative, or a pharmaceutically acceptable salt thereof. The methods of the present invention are useful for treating, for example, rheumatoid arthritis, osteoarthritis, an autoimmune disease, psoriasis, asthma, a cardiovascular disease, an acute coronary syndrome, congestive heart failure, Alzheimer's disease, multiple sclerosis, cancer, type II diabetes, metabolic syndrome X, or inflammatory bowel disease.

## Description

### FIELD OF THE INVENTION

The present invention provides methods of treating a disease or a disorder responsive to inhibition of nuclear factor-κB transcription factors, comprising administering to patients in need thereof a sulfonylaminocarbonyl derivative, or a pharmaceutically acceptable salt thereof.

### BACKGROUND OF THE INVENTION

Inhibition of nuclear factor-kappa B ("NF-κB") transcription factor-mediated activity would provide valuable methods of treating a disease or a disorder afflicting millions of people worldwide. This is so because NF-κB mediates transcription of a large number of genes involved in the production of pro-inflammatory cytokines and other biomolecules intimately involved in the etiology of many diseases and disorders for which no completely effective treatment is available. Noteworthy among the diseases and disorders thought to be responsive to the inhibition of NF-κB are rheumatoid arthritis and osteoarthritis, autoimmune diseases, psoriasis, asthma, cardiovascular diseases such as, for example, atherosclerosis, acute coronary syndromes including myocardial infarction and unstable angina, and congestive heart failure, Alzheimer's disease, multiple sclerosis, cancer, type II diabetes, metabolic syndrome X, and inflammatory bowel disease ("IBD"). These diseases and disorders are among the most prevalent in society today and cause untold suffering and death.

Current methods of treatment of the above mentioned diseases and disorders are unsatisfactory, as they typically require surgical alteration or removal of the affected body part or the use of pharmaceuticals that treat symptoms, without stopping or, ideally, reversing the underlying disease process. For example, while there are many marketed agents that modify risk factors for atherosclerosis (e.g., reduction of plasma lipids and anti-hypertensive agents), there are no therapies that directly modify the atherosclerosis process itself. Further, more than 60% of all coronary artery disease cannot be explained on the basis of traditional risk factors alone.

One explanation for the lack of success of current treatments for the above-mentioned diseases is that multiple gene products are probably involved in each disease or disorder. Typical drug therapies target only one of these gene products. Also, many of the current drugs used to treat these diseases and disorders exhibit undesirable side effects such as, for example, the gastric ulceration observed with many nonsteroidal anti-inflammatory drugs ("NSAIDs") used to treat arthritis. Disadvantages of surgical methods of treating these diseases and disorders include the use of highly invasive procedures that cause pain, scarring, and sometimes infection.

In contrast, inhibition of NF-κB is potentially capable of halting, and even reversing, the progression of the underlying diseases and disorders mentioned above. Inhibitors of NF-κB are effective by virtue of their ability to prevent, block, and even halt a common key step in the activation of the genes involved in the production of a number of mediators of these diseases and disorders. In other words, NF-κB inhibitors work upstream to inhibit the production of multiple pro-inflammatory mediators, whereas traditional drug treatment regimens are less effective, perhaps because they work downstream and typically target only one of these mediators.

Nuclear factor-κB is a family of heterogeneous protein dimers that act as sequence-specific transcription factors in the activation of a large number of genes in response to inflammation, viral or bacterial infections, or other biological diseases and disorders requiring rapid reprogramming of gene expression. NF-κB is normally found sequestered in the cytoplasm in an inactive form bound to an inhibitory protein, namely the inhibitor of κB ("IκB"). IκB is thus bound with NF-κB to form an NF-κB-IκB complex, but NF-κB is rapidly converted to an active form via signaling processes that are still being elucidated.

NF-κB is found in virtually all cell types including T-lymphocytes, monocytes, macrophages, endothelial cells, and smooth muscle cells. In response to a stimulus such as, for example, an inflammatory cytokine, a reactive oxygen intermediate, or a lipopolysaccharide from a microorganism, the IκB component of the NF-κB―IκB complex is cleaved via a process comprising the sequential steps of phosphorylation, polyubiquitinylation, and degradation. Degradation of the modified IκB protein exposes the nuclear localization sequence on NF-κB, allowing translocation of NF-κB to the nucleus of the cell, where it binds to its target gene to initiate transcription.

Among the genes to which NF-κB binds in order to initiate transcription are genes expressing pro-inflammatory cytokines. These pro-inflammatory cytokines include tumor necrosis factor-alpha ("TNF-α"), interleukin-1 ("IL-1"), IL-6, IL-8, intercellular adhesion molecule-1 ("ICAM-1"), vascular cell adhesion molecule-1 ("VCAM-1"), E-selectin, monocyte chemotactic protein-1 ("MCP-1"), inducible nitric oxide synthase, tissue factor, and cyclooxygenase-2 ("COX-2"). The result of the transcriptional activation of genes expressing pro-inflammatory cytokines is a tissue-localized production of these cytokines, and the beginning or exacerbation of an inflammatory process in the affected tissue.

The present invention provides a method of using a sulfonylaminocarbonyl derivative, or a pharmaceutically acceptable salt thereof, to treat diseases and disorders known to be responsive to the inhibition of NF-κB.

The following United States patents disclose methods of using certain sulfonylaminocarbonyl derivatives as inhibitors of the enzyme acyl-coenzyme A:cholesterol acyltransferase (ACAT) for treating hypercholesterolemia and atherosclerosis:
United States Patent Number 5,245,068 and its Divisional 5,384,328;
United States Patent Number 5,214,206 and its Divisional 5,288,757;
United States Patent Number 5,254,715 and its Divisional 5,336,690;
United States Patent Number 5,198,466 and its Divisional 5,364,882;
United States Patent Number 5,491,172 and its Divisional 5,633,287; and
United States Patent Number 5,254,589 and its Continuation 5,981,595.

United States Patent Number 6,093,744 discloses methods of using certain sulfonylaminocarbonyl derivatives as ACAT inhibitors for regulating plasma cholesterol levels and lowering serum or plasma Lp(a) levels, and for treating hypercholesterolemia, atherosclerosis, peripheral vascular diseases, and restenosis.

United States Patent Number 6,117,909 discloses methods of using certain sulfonylaminocarbonyl derivatives as ACAT inhibitors for lowering serum or plasma Lp(a) levels, and treating cerebrovascular diseases, including stroke, peripheral vascular diseases, and restenosis.

United States Patent Number 6,124,309 and its Divisional Patent Numbers 6,143,755 and 6,093,719 disclose methods of using a sulfonylaminocarbonyl derivative as an ACAT inhibitor in combination with an HMG-CoA reductase inhibitor for restoring endogenous vascular endothelium-dependent activities including improving the normal dilation capacity of the endothelium, inducing vasodilation to modulate vascular tone and blood flow, decreasing the adherent properties of the blood vessel walls, and decreasing the coagulation of platelets, and for treating myocardial infarction and acute ischemic syndromes including angina pectoris, coronary artery disease, hypertension, cerebrovascular accidents, transient ischemic attacks, chronic obstructive pulmonary disease, chronic hypoxic lung disease, pulmonary hypertension, renal hypertension, chronic renal disease, microvascular complications of diabetes, and vaso-occlusive complications of sickle cell anemia.

As NF-κB is involved in the initiation and progression of inflammatory disease, a screening assay which provides a method for rapidly screening large numbers of compounds in vitro for their ability to inhibit NF-κB mediated transcription of a gene would be a valuable tool. Such a screening assay would be an important step in the pursuit of compounds to treat diseases responsive to inhibition of NF-κB.

We have now discovered the ability of certain sulfonylaminocarbonyl derivatives to inhibit NF-κB mediated transcription. Accordingly, the present invention provides a method of treating a disease or a disorder responsive to inhibition of NF-κB, comprising administering to patients in need thereof a sulfonylaminocarbonyl derivative, or a pharmaceutically acceptable salt thereof. All that is needed to practice the present invention is to administer to said patients a sulfonylaminocarbonyl derivative, or a pharmaceutically acceptable salt thereof, from 1 to 6 times daily for the treatment of rheumatoid arthritis, osteoarthritis, autoimmune diseases, psoriasis, asthma, cardiovascular diseases such as, for example, atherosclerosis, acute coronary syndromes including myocardial infarction and unstable angina, and congestive heart failure, Alzheimer's disease, multiple sclerosis, cancer, type II diabetes, metabolic syndrome X, and inflammatory bowel disease. Determination of a proper dosage and form of administration of a sulfonylaminocarbonyl derivative, or a pharmaceutically acceptable salt thereof, for use in the method of the present invention is well within the abilities of one of ordinary skill in the pharmaceutical and medical arts.

### SUMMARY OF THE INVENTION

The present invention provides a method of treating a disease or a disorder responsive to inhibition of nuclear factor-κB transcription factors, comprising administering to a patient in need thereof a sulfonylaminocarbonyl derivative, or a pharmaceutically acceptable salt thereof.

The sulfonylaminocarbonyl derivatives disclosed in United States Patent Number 5,491,172 and its Divisional 5,633,287, which are both hereby incorporated herein by reference, are useful in the present invention. Thus, one embodiment of the present invention is a method of treating a disease or a disorder responsive to inhibition of nuclear factor-κB transcription factors, comprising administering to a patient in need thereof a sulfonylaminocarbonyl derivative of Formula I 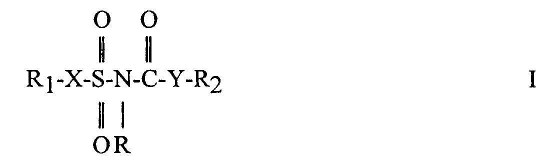 or a pharmaceutically acceptable salt thereof, wherein:
X and Y are selected from oxygen, sulfur and (CR'R")ₙ, wherein n is an integer of from 1 to 4 and R' and R" are each independently hydrogen, alkyl, alkoxy, halogen, hydroxy, acyloxy, cycloalkyl, phenyl optionally substituted or R' and R" together form a spirocycloalkyl or a carbonyl; with the proviso at least one of X and Y is -(CR'R")ₙ- and with the further proviso when X and Y are both (CR'R")ₙ and R' and R" are hydrogen and n is one, R₁ and R₂ are aryl;
R is hydrogen, a straight or branched alkyl of from 1 to 8 carbon atoms or benzyl;
R₁ and R₂ are each independently selected from:
   (a) phenyl or phenoxy each of which is unsubstituted or is substituted with from 1 to 5 substituents selected from:
      phenyl,
      an alkyl group having from 1 to 6 carbon atoms and which is straight or branched,
      an alkoxy group having from 1 to 6 carbon atoms and which is straight or branched;
      phenoxy,
      hydroxy,
      fluorine,
      chlorine,
      bromine,
      nitro,
      trifluoromethyl,
      -COOH,
      -COOalkyl wherein alkyl has from 1 to 4 carbon atoms and is straight or branched, and
      -(CH₂)ₚNR₃R₄, wherein p is zero or one, and each of R₃ and R₄ is selected from hydrogen or a straight or branched alkyl group having 1 to 4 carbon atoms;

   (b) 1- or 2-naphthyl unsubstituted or substituted with from 1 to 3 substituents selected from:
      phenyl,
      an alkyl group having from 1 to 6 carbon atoms and which is straight or branched,
      an alkoxy group having from 1 to 6 carbon atoms and which is straight or branched;
      hydroxy,
      phenoxy,
      fluorine,
      chlorine,
      bromine,
      nitro,
      trifluoromethyl,
      -COOH,
      -COOalkyl, wherein alkyl has from 1 to 4 carbon atoms and is straight or branched,
      -(CH₂)ₚNR₃R₄, wherein p, R₃ and R₄ have the meanings defined above;

   (c) arylalkyl;
   (d) a straight or branched alkyl chain having from 1 to 20 carbon atoms and which is saturated or contains from 1 to 3 double bonds; and
   (e) adamantyl or a cycloalkyl group wherein the cycloalkyl moiety has from 3 to 6 carbon atoms; with the provisos:
      (i) where X is (CH₂)ₙ, Y is oxygen, and R₁ is a substituted phenyl, then R₂ is a substituted phenyl;
      (ii) where Y is oxygen, X is (CH₂)ₙ, R₂ is phenyl or naphthyl, then R₁ is not a straight or branched alkyl chain; and
      (iii) the following compounds are excluded: 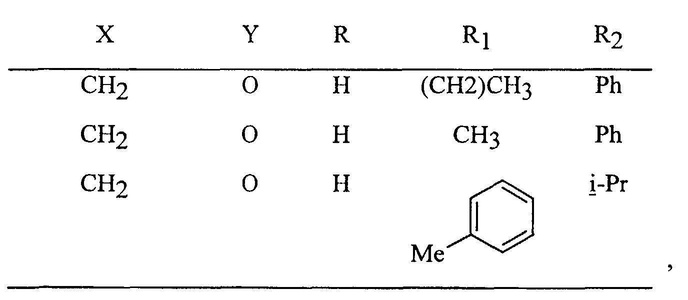 with the further proviso that compounds selected from the group consisting of:

Sulfamic acid [1-oxo-3-[2,4,6-tris(1-methylethyl)phenyl]propyl]-2,6-bis(1-methylethyl)phenyl ester,
Sulfamic acid [fluoro[2,4,6-tris(1-methylethyl)phenyl]acetyl]-2,6-bis(1-methylethyl)phenyl ester, and
Sulfamic acid [[2,4,6-tris(1-methylethyl)phenyl]acetyl]-2,6-bis(phenyl)phenyl ester are excluded.

Preferred is a method of treating a disease or a disorder responsive to inhibition of nuclear factor-κB transcription factors, comprising administering to a patient in need thereof a sulfonylaminocarbonyl derivative of Formula I:
wherein R₁ is phenyl or is phenyl disubstituted in the 2,6-positions;
wherein R₂ is phenyl or is phenyl disubstituted in the 2,6-positions;
wherein each of R₁ and R₂ is phenyl;
wherein each phenyl is disubstituted in the 2,6-position;
wherein R₁ is phenyl disubstituted in the 2,6-positions and R₂ is phenyl trisubstituted in the 2,4,6-positions;
wherein R₁ is 2,6-bis(1-methylethyl)phenyl and R₂ is 2,6-bis(1-methylethyl)phenyl or 2,4,6-tris(1-methylethyl)phenyl; or
wherein one of R₁ and R₂ is the group 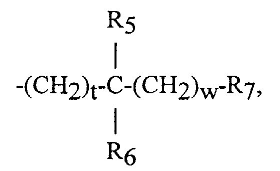
wherein t is zero or 1 to 4; w is zero or 1 to 4 with the proviso that the sum of t and w is not greater than 5; R₅ and R₆ are each independently selected from hydrogen or alkyl having from 1 to 6 carbon atoms, or when R₅ is hydrogen, R₆ can be selected from the groups defined for R₇; and R₇ is phenyl or phenyl substituted with from 1 to 3 substituents selected from a straight or branched alkyl group having from 1 to 6 carbon atoms, straight or branched alkoxy group having from 1 to 6 carbon atoms, phenoxy, hydroxy, fluorine, chlorine, bromine, nitro, trifluoromethyl, -COOH, COOalkyl wherein alkyl has from 1 to 4 carbon atoms, or -(CH₂)ₚNR₃R₄ wherein P, R₃ and R₄ have the meanings defined above.

Preferred is a method of treating a disease or a disorder responsive to inhibition of nuclear factor-κB transcription factors, comprising administering to a patient in need thereof a sulfonylaminocarbonyl derivative of Formula I, or a pharmaceutically acceptable salt thereof, wherein:
X is oxygen, sulfur or (CR'R")n;
Y is oxygen, sulfur or (CR'R")n, with the proviso that at least one of X or Y is (CR'R")n wherein n is an integer of from 1 to 4 and R' and R" are each independently hydrogen, straight or branched alkyl of from 1 to 6 carbons, optionally substituted phenyl, halogen, hydroxy, alkoxy, acyloxy, cycloalkyl, or R' and R" taken together form a carbonyl or a spirocycloalkyl group of from 3 to 10 carbons;
R is hydrogen;
R₁ is phenyl optionally substituted, straight or branched alkyl of from 1 to 10 carbon atoms, cycloalkyl of from 3 to 10 carbon atoms; and
R₂ is phenyl optionally substituted, straight or branched alkyl of from 1 to 10 carbon atoms, cycloalkyl of from 3 to 8 carbon atoms, phenoxy optionally substituted with the proviso that only if X is (CR'R")ₙ can R₁ be optionally substituted phenoxy and only if Y is (CR'R")ₙ can R₂ be optionally substituted phenoxy, and with the further proviso that at least one of R₁ and R₂ is optionally substituted phenyl or phenoxy.

Preferred is a method of treating a disease or a disorder responsive to inhibition of nuclear factor-κB transcription factors, comprising administering to a patient in need thereof a sulfonylaminocarbonyl derivative of Formula I, or a pharmaceutically acceptable salt thereof, wherein:
X is oxygen;
Y is (CR'R")ₙ wherein n is an integer of from 1 to 2;
R is hydrogen;
R₁ is optionally substituted phenyl;
R₂ is optionally substituted phenyl or phenoxy, straight or branched alkyl of from 1 to 10 carbons, or cycloalkyl of from 3 to 10 carbons; and
R' and R" are each independently hydrogen, straight or branched alkyl of from 1 to 6 carbons, optionally substituted phenyl, halogen, hydroxy, alkoxy, acyloxy, cycloalkyl, or R' and R" taken together form a carbonyl or a spirocycloalkyl.

Preferred is a method of treating a disease or a disorder responsive to inhibition of nuclear factor-κB transcription factors, comprising administering to a patient in need thereof a sulfonylaminocarbonyl derivative of Formula I, or a pharmaceutically acceptable salt thereof, wherein one of R₁ and R₂ is phenyl; and more preferably wherein one of R₁ and R₂ is substituted phenyl; or still more preferably wherein one of R₁ and R₂ is phenyl disubstituted in the 2,6-positions.

More preferred is a method of treating a disease or a disorder responsive to inhibition of nuclear factor-κB transcription factors, comprising administering to a patient in need thereof a sulfonylaminocarbonyl derivative of Formula I, or a pharmaceutically acceptable salt thereof, wherein both R₁ and R₂ are phenyl disubstituted in the 2,6-positions.

In another more preferred embodiment, the method uses a sulfonylaminocarbonyl derivative of Formula I, or a pharmaceutically acceptable salt thereof, wherein R₁ is phenyl disubstituted in the 2,6-positions and R₂ is phenyl trisubstituted in the 2,4,6-positions.

Preferred is a method of treating a disease or a disorder responsive to inhibition of nuclear factor-κB transcription factors, comprising administering to a patient in need thereof a sulfonylaminocarbonyl derivative of Formula I, or a pharmaceutically acceptable salt thereof, selected from:
(1,2,3,4-Tetrahydro-naphthalene-2-carbonyl)-sulfamic acid 2,6-diisopropyl-phenyl ester;
[Bis-(4-chloro-phenyl)-acetyl]-sulfamic acid 2,6-diisopropyl-phenyl ester;
(Bromo-phenyl-acetyl)-sulfamic acid 2,6-diisopropyl-phenyl ester;
[(2,4,6-Triisopropyl-phenyl)-acetyl]-sulfamic acid 4-hydroxy-2,6-diisopropyl-phenyl ester;
Methyl-[(2,4,6-triisopropyl-phenyl)-acetyl]-sulfamic acid 2,6-diisopropyl-phenyl ester;
[(2,4,6-Triisopropyl-phenyl)-acetyl]-sulfamic acid 2,6-diisopropyl-4-nitrophenyl ester;
[(2,4,6-Triisopropyl-phenyl)-acetyl]-sulfamic acid 4-fluoro-2,6-diisopropyl-phenyl ester;
[(2,4,6-Triisopropyl-phenyl)-acetyl]-sulfamic acid 2,6-dimethoxy-phenyl ester;
[(2,4,6-Triisopropyl-phenyl)-acetyl]-sulfamic acid 4-amino-2,6-diisopropyl-phenyl ester;
[(2,4,6-Triisopropyl-phenyl)-acetyl]-sulfamic acid 2,4,6-trimethoxy-phenyl ester;
[(2,4,6-Triisopropyl-phenyl)-acetyl]-sulfamic acid 4-tert-butyl-2,6-diisopropyl-phenyl ester;
[(2,4,6-Triisopropyl-phenyl)-acetyl]-sulfamic acid 4-acetyl-2-isopropylphenyl ester;
[(2,4,6-Triisopropyl-phenyl)-acetyl]-sulfamic acid 2,6-diisopropyl-4-methoxy-phenyl ester;
[(2,4,6-Triisopropyl-phenyl)-acetyl]-sulfamic acid 2,6-dichloro-phenyl ester;
[(2,4,6-Triisopropyl-phenyl)-acetyl]-sulfamic acid dodecyl ester;
[(2,4,6-Triisopropyl-phenyl)-acetyl]-sulfamic acid 4-bromo-2,6-diisopropyl-phenyl ester;
[(2,4,6-Triisopropyl-phenyl)-acetyl]-sulfamic acid 2,6-diisopropyl-4-methyl-phenyl ester;
[1-(4-Dimethylamino-phenyl)-cyclopentanecarbonyl]-sulfamic acid 2,6-diisopropyl-phenyl ester;
[1-(4-Nitro-phenyl)-cyclopentanecarbonyl]-sulfamic acid 2,6-diisopropyl-phenyl ester;
3,5-Diisopropyl-4-{[(2,4,6-triisopropyl-phenyl)-acetyl]sulfamoyloxy}-benzoic acid methyl ester;
Sulfamic acid (phenylacetyl)-2,6-bis(1-methylethyl)phenyl ester;
Sulfamic acid[[2,4,6-tris(1-methylethyl)phenyl]acetyl]-2,6-bis(1-methyl-ethyl)phenyl ester;
Sulfamic acid[[2,6-bis(1-methylethyl)phenyl]acetyl]-2,6-bis(1-methyl-ethyl)phenyl ester;
Sulfamic acid [[2,4,6-tris(1-methylethyl)phenyl]acetyl-2,4,6-tris(1-methyl-ethyl)phenyl ester;
Sulfamic acid[[2,6-bis(1-methylethyl)phenyl]acetyl]-2,4,6-tris( 1-methyl-ethyl)phenyl ester;
Sulfamic acid[adamantaneacetyl]-2,6-bis[1-methylethyl)phenyl ester,
Sulfamic acid[[2,6-bis(1-methylethyl)phenyl]acetyl]-2,6-bis( 1-methyl-ethyl)phenyl ester-sodium salt;
Sulfamic acid[[2,4,6-tris(1-methylethyl)phenyl]acetyl]-2,6-bis(1-methyl-ethyl)phenyl ester-sodium salt;
Sulfamic acid (decanoyl)-2,6-bis-(1-methylethyl)phenyl ester;
Sulfamic acid (dodecanoyl)-2,6-bis-(1-methylethyl)phenyl ester;
2,6-Bis(1-methylethyl)-N-[[[2,4,6-tris( 1-methylethyl)phenyl]methyl]-sulfonyl]benzeneacetamide;
2,6-Bis(1-methylethyl)-N-[[[2,4,6-tris( 1-methylethyl)phenyl]methyl]-sulfonyl]benzeneacetamide-sodium salt;
2,6-Bis(1-methylethyl)phenyl[[[2,4,6-tris( 1-methylethyl)phenyl]methyl]-sulfonyl]carbamate;
2,6-Bis(1-methylethyl)phenyl[[[2,4,6-tris( 1-methylethyl)phenyl]methyl]-sulfonyl]carbamate-sodium salt;
Sulfamic acid (1-oxo-3,3-diphenylpropyl)-2,6-bis(1-methylethyl)phenyl ester;
Sulfamic acid [2,6-dichlorophenyl(acetyl)]-2,6-bis(1-methylethyl)phenyl ester,
Sulfamic acid [2,6-dichlorophenyl(acetyl)]-2,6-bis(1-methylethyl)phenyl ester-sodium salt;
Sulfamic acid trans-[(2-phenylcyclopropyl)carbonyl]-2,6-bis(1-methylethyl)phenyl ester;
Sulfamic acid [2,5-dimethoxyphenyl(acetyl)]-2,6-bis(1-methylethyl)-phenyl ester;
Sulfamic acid [2,4,6-trimethoxyphenyl(acetyl)]-2,6-bis(1-methylethyl)-phenyl ester;
Sulfamic acid [2,4,6-trimethylphenyl(acetyl)]-2,6-bis(1-methylethyl)-phenyl ester;
Sulfamic acid [2-thiophenyl(acetyl)]-2,6-bis(1-methylethyl)phenyl ester;
Sulfamic acid [3-thiophenyl(acetyl)]-2,6-bis(1-methylethyl)phenyl ester;
Sulfamic acid [2-methoxyphenyl(acetyl)]-2,6-bis(1-methylethyl)phenyl ester;
Sulfamic acid (oxophenylacetyl)-2,6-bis(1-methylethyl)phenyl ester;
Sulfamic acid [2-trifluoromethylphenyl(acetyl)]-2,6-bis(1-methylethyl)-phenyl ester;
Sulfamic acid (1-oxo-2-phenylpropyl)-2,6-bis(1-methylethyl)phenyl ester;
Sulfamic acid (cyclopentylphenyl-acetyl)-2,6-bis(1-methylethyl)phenyl ester;
Sulfamic acid (cyclohexylacetyl)-2,6-bis(1-methylethyl)phenyl ester;
Sulfamic acid (diphenylacetyl)-2,6-bis(1-methylethyl)phenyl ester;
Sulfamic acid (triphenylacetyl)-2,6-bis(1-methylethyl)phenyl ester;
Sulfamic acid [(1-phenylcyclopentyl)carbonyl]-2,6-bis(1-methylethyl)-phenyl ester;
Sulfamic acid (3-methyl-1-oxo-2-phenylpentyl)-2,6-bis(1-methylethyl)phenyl ester;
Sulfamic acid (1-oxo-2-phenylbutyl)-2,6-bis(1-methylethyl)phenyl ester;
Sulfamic acid (cyclohexylphenyl-acetyl)-2,6-bis(1-methylethyl)phenyl ester;
Sulfamic acid (1-oxo-2,2-diphenylpropyl)-2,6-bis(1-methylethyl)phenyl ester;
Sulfamic acid [(9H-fluoren-9-yl)carbonyl]-2,6-bis(1-methylethyl)phenyl ester;
Sulfamic acid (1-oxo-3-phenylpropyl)-2,6-bis(1-methylethyl)phenyl ester;
Sulfamic acid [1-oxo-3-[2,4,6-tris(1-methylethyl)phenyl]-2-propenyl]-2,6-bis(1-methylethyl)phenyl ester;
Sulfamic acid [(acetyloxy)[2,4,6-tris(1-methylethyl)phenyl]acetyl]-2,6-bis(1-methylethyl)phenyl ester;
Sulfamic acid [hydroxy[2,4,6-tris(1-methylethyl)phenyl]acetyl]-2,6-bis(1-methylethyl)phenyl ester;
Sulfamic acid (3-methyl-1-oxo-2-phenylpentyl)-2,6-bis(1-methylethyl)phenyl ester sodium salt;
Sulfamic acid [[2,4,6-tris(1-methylethyl)phenoxy]acetyl]-2,6-bis(1-methylethyl)phenyl ester; and
Sulfamic acid [[2,6-bis(1-methylethyl)phenoxy]acetyl]-2,6-bis(1-methylethyl)phenyl ester.

More preferred is a method of treating a disease or a disorder responsive to inhibition of nuclear factor-κB transcription factors, comprising administering to a patient in need thereof a sulfonylaminocarbonyl derivative of Formula I named sulfamic acid [[2,4,6-tris(1-methylethyl)phenyl]acetyl-2,6-bis(1-methylethyl)phenyl ester, or a pharmaceutically acceptable salt thereof.

Still more preferred is a method of treating a disease or a disorder responsive to inhibition of nuclear factor-κB transcription factors, comprising administering to a patient in need thereof a sulfonylaminocarbonyl derivative of Formula I named sulfamic acid [[2,4,6-tris(1-methylethyl)phenyl]acetyl-2,6-bis(1-methylethyl)phenyl ester.

The sulfonylaminocarbonyl derivatives disclosed in United States Patent Number 6,093,744, which is hereby incorporated herein by reference, are also useful in the present invention. Thus, another embodiment of the present invention is a method of treating a disease or a disorder responsive to inhibition of nuclear factor-κB transcription factors, comprising administering to a patient in need thereof a sulfonylaminocarbonyl derivative of Formula II 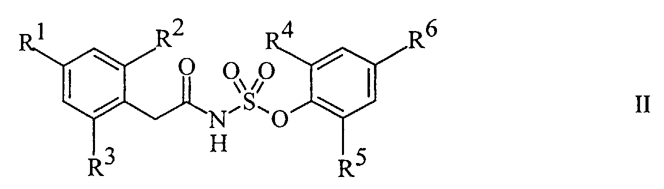 or a pharmaceutically acceptable salt thereof, wherein:
R¹ is hydrogen, alkyl, or alkoxy;
R² to R⁵ are alkyl, alkoxy, or unsubstituted or substituted phenyl; and
R⁶ is -CN,
   -(CH₂)₀₋₁-NR⁷R⁸,
   -O-(CH₂)₁₋₁₀-Z, wherein Z is -NR⁹R¹⁰, OR¹, or CO₂R¹,
   -OC(=O)R¹¹,
   -SR¹¹,
   -SCN,
   -S(CH₂)₁₋₁₀Z,
   -S(O)₁₋₂R¹², wherein R¹² is hydroxy, alkoxy, alkyl, (CH₂)₁₋₁₀Z or NR⁷R⁸,
   -C(=O)XR¹¹, or
   -CH₂-R¹³, wherein R¹³ is (CH₂)₀₋₅-Y-(CH₂)₀₋₅Z, or alkyl of from 1 to 20 carbons with from 1-3 double bonds, which alkyl is optionally substituted by one or more moieties selected from -CN, NO₂, halogen, OR¹, NR⁹R¹⁰, and CO₂R¹;
wherein R⁷ and R⁸ are each independently selected from:
- hydrogen, at least one of R⁷ and R⁸ is other than hydrogen;
- (CH₂)₁₋₁₀Z, wherein Z is as defined above and R⁹ and R¹⁰ are each independently selected from hydrogen, alkyl, and unsubstituted or substituted phenyl, or
R⁹ and R¹⁰ are taken together with the nitrogen to which they are attached to form a ring selected from: 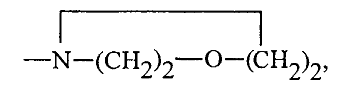 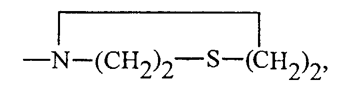 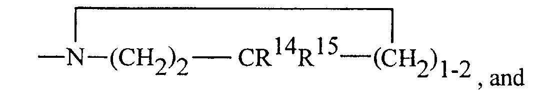 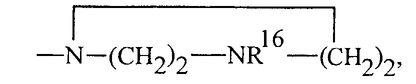 wherein R¹⁴, R¹⁵, and R¹⁶ are each independently selected from hydrogen, alkyl, and unsubstituted or substituted phenyl;
- C(=Q)XR¹¹, wherein X is a bond or NH wherein Q is O or S, R¹¹ is hydrogen, alkyl, unsubstituted or substituted phenyl;
- (CH₂)₀₋₅-Y-(CH₂)₀₋₅Z, wherein Z is as defined above and Y is phenyl or a bond;
- C(=O)-CR¹⁷R¹⁸Z;
- C(=O)NHCR¹⁷R¹⁸Z, wherein R¹⁷ and R¹⁸ are each independently hydrogen, alkyl, phenyl, substituted phenyl, or the side chain of a naturally occurring amino acid;
- S(O)₁₋₂R¹⁹, wherein R¹⁹ is alkyl, unsubstituted or substituted phenyl, naphthyl, or a heteroaromatic ring, or NR⁹R¹⁰; or
R⁷ and R⁸ are taken together with the nitrogen to which they are attached to form a ring selected from: 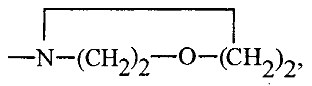 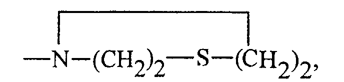 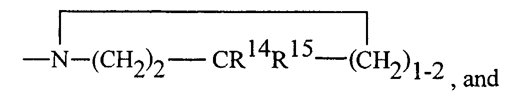 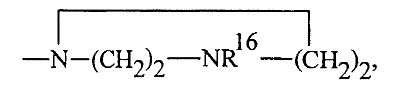 wherein R¹⁴, R¹⁵, and R¹⁶ are as above, with the proviso that compounds selected from:
[(2,4,6-Triisopropyl-phenyl)-acetyl]-sulfamic acid 4-formyl-2,6-diisopropyl-phenyl ester;
[(2,4,6-Triisopropyl-phenyl)-acetyl]-sulfamic acid 4-(2-cyano-vinyl)-2,6-diisopropyl-phenyl ester;
[(2,4,6-Triisopropyl-phenyl)-acetyl]-sulfamic acid 2,6-diisopropyl-4-(4-methyl-piperazin-1-ylmethyl)-phenyl ester, dihydrochloride;
[(2,4,6-Triisopropyl-phenyl)-acetyl]-sulfamic acid 4-[bis-(2-hydroxyethyl)-amino]-2,6-diisopropyl-phenyl ester;
[(2,4,6-Triisopropyl-phenyl)-acetyl]-sulfamic acid 2,6-diisopropyl-4-(3-phenyl-thioureido]-phenyl ester; and
[(2,4,6-Triisopropyl-phenyl)-acetyl]-sulfamic acid 2,6-diisopropyl-4-sulfamoyl-phenyl ester are excluded.

Preferred is a method of treating a disease or a disorder responsive to inhibition of nuclear factor-κB transcription factors, comprising administering to a patient in need thereof a sulfonylaminocarbonyl derivative of Formula II, or a pharmaceutically acceptable salt thereof, wherein:
R¹ is hydrogen or alkyl of from 1 to 4 carbon atoms;
R² to R⁵ are each alkyl of from 1 to 4 carbon atoms; and
R⁶ is -NR⁷R⁸ wherein R⁷ and R⁸ are each independently selected from:
   hydrogen, at least one of R⁷ and R⁸ is not hydrogen,
   -(CH₂)₁₋₁₀Z,
   -C(=Q)XR¹¹, or
   -S(O)₁₋₂R¹⁹.

More preferred is a method of treating a disease or a disorder responsive to inhibition of nuclear factor-κB transcription factors, comprising administering to a patient in need thereof a sulfonylaminocarbonyl derivative of Formula II, or a pharmaceutically acceptable salt thereof, wherein:
R⁷ is hydrogen and
R⁸ is -C(=O)CR¹⁷R¹⁸Z wherein Z is NH₂ where one of R¹⁷ and R¹⁸ is the side chain of a naturally occurring amino acid and the other is hydrogen.

Also preferred is a method of treating a disease or a disorder responsive to inhibition of nuclear factor-κB transcription factors, comprising administering to a patient in need thereof a sulfonylaminocarbonyl derivative of Formula II, or a pharmaceutically acceptable salt thereof, wherein:
R¹ is hydrogen or alkyl of from 1 to 4 carbon atoms;
R² to R⁵ are each alkyl of from 1 to 4 carbon atoms; and
R⁶ is NR⁷R⁸, wherein R⁷ and R⁸ taken together with the nitrogen to which they are attached to form a ring selected from the group consisting of: 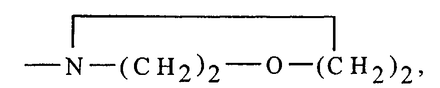 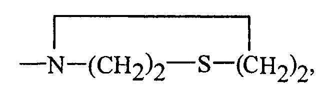 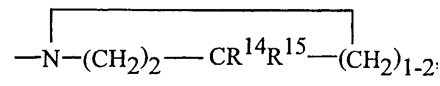 wherein R¹⁴ and R¹⁵ are each independently selected from hydrogen, alkyl, and phenyl; and 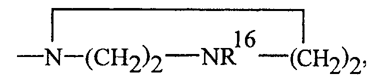 wherein R¹⁶ is hydrogen, alkyl, or phenyl.

Also preferred is a method of treating a disease or a disorder responsive to inhibition of nuclear factor-κB transcription factors, comprising administering to a patient in need thereof a sulfonylaminocarbonyl derivative of Formula II, or a pharmaceutically acceptable salt thereof, wherein:
R¹ is hydrogen or alkyl of from 1 to 4 carbon atoms;
R² to R⁵ are each alkyl of from 1 to 4; and
R⁶ is NR⁷R⁸, wherein one of R⁷ and R⁸ is hydrogen and the other is S(O)₁₋₂R¹⁹
wherein R¹⁹ is alkyl, unsubstituted or substituted phenyl, naphthyl, or a heteroaromatic ring.

More preferred is the invention method using a compound of Formula II, or a pharmaceutically acceptable salt thereof, wherein:
R¹ is hydrogen or alkyl of from 1 to 4 carbons;
R² to R⁵ are alkyl of from 1 to 4 carbons; and
R⁶ is -C(=O)XR¹¹ or -CH₂R¹³ wherein X, R¹¹, and R¹³ are as defined above for Formula II.

Also preferred is a method of treating a disease or a disorder responsive to inhibition of nuclear factor-κB transcription factors, comprising administering to a patient in need thereof a sulfonylaminocarbonyl derivative of Formula II, or a pharmaceutically acceptable salt thereof, wherein:
R¹ is hydrogen or alkyl of from 1 to 4 carbon atoms;
R² to R⁵ are alkyl of from 1 to 4 carbon atoms; and
R⁶ is -O-(CH₂)₁₋₁₀Z,
   -O-C(=O)R¹¹,
   -SH,
   -SCN,
   -S(CH₂)₁₋₁₀Z, or
   -S(O)₁₋₂R¹² wherein Z, R¹¹, and R¹² are as defined above for Formula II.

Also preferred is a method of treating a disease or a disorder responsive to inhibition of nuclear factor-κB transcription factors, comprising administering to a patient in need thereof a sulfonylaminocarbonyl derivative of Formula II, or a pharmaceutically acceptable salt thereof, wherein:
R¹ is hydrogen or alkyl of from 1 to 4 carbon atoms;
R² to R⁵ are alkyl of from 1 to 4 carbon atoms; and
R⁶ is O(CH₂)₁₋₁₀NR⁹R¹⁰ wherein R⁹ and R¹⁰ are as defined above for Formula II.

Especially preferred is a method of treating a disease or a disorder responsive to inhibition of nuclear factor-κB transcription factors, comprising administering to a patient in need thereof a sulfonylaminocarbonyl derivative of Formula II, or a pharmaceutically acceptable salt thereof, selected from:
6-(3,5-Diisopropyl-4-{[(2,4,6-triisopropyl-phenyl)-acetyl]sulfamoyloxy}-phenyl)-hexanoic acid ethyl ester;
3-[3-(3,5-Diisopropyl-4-{[(2,4,6-triisopropyl-phenyl)-acetyl]sulfamoyloxy}-phenyl)-ureido]-propionic acid ethyl ester;
{[4-(1-Hydroxy-1-methyl-ethyl)-2,6-diisopropyl-phenyl]-acetyl} -sulfamic acid 2,6-diisopropyl-phenyl ester;
[2-(2,4,6-Triisopropyl-phenyl)-acetyl]-sulfamic acid 4-((S)-2-amino-4-methyl-pentanoylamino)-2,6-diisopropyl-phenyl ester; compound with trifluoro-acetic acid;
[(2,4,6-Triisopropyl-phenyl)-acetyl]-sulfamic acid 4-(3-tert-butyl-ureido)-2,6-diisopropyl-phenyl ester;
[2-(2,4,6-Triisopropyl-phenyl)-acetyl]-sulfamic acid 4-(3-amino-propionylamino)-2,6-diisopropyl-phenyl ester; compound with trifluoro-acetic acid;
[(2,4,6-Triisopropyl-phenyl)-acetyl]-sulfamic acid 4-(2-cyano-vinyl)-2,6-diisopropyl-phenyl ester;
[2-(2,4,6-Triisopropyl-phenyl)-acetyl]-sulfamic acid 4-((S)-2-amino-3-hydroxy-propionylamino)-2,6-diisopropyl-phenyl ester; compound with trifluoro-acetic acid;
[2-(2,4,6-Triisopropyl-phenyl)-acetyl]-sulfamic acid 4-((S)-2-amino-4-carbamoyl-butyrylamino)-2,6-diisopropyl-phenyl ester; compound with trifluoro-acetic acid;
[2-(2,4,6-Triisopropyl-phenyl)-acetyl]-sulfamic acid 4-((S)-2-amino-3-methyl-butyrylamino)-2,6-diisopropyl-phenyl ester; compound with trifluoro-acetic acid;
[(2,4,6-Triisopropyl-phenyl)-acetyl]-sulfamic acid 4-[3-(3,5-dichlorophenyl)-thioureido]-2,6-diisopropyl-phenyl ester;
(S)-[5-tert-Butoxycarbonylamino-5-(3,5-diisopropyl-4-{[(2,4,6-triisopropyl-phenyl)-acetyl]sulfamoyloxy}-phenylcarbamoyl)-pentyl]-carbamic acid tert-butyl ester;
(S)-[(2,4,6-Triisopropyl-phenyl)-acetyl]-sulfamic acid 4-(2,6-diaminohexanoylamino)-2,6-diisopropyl-phenyl ester dihydrochloride;
[(2,4,6-Triisopropyl-phenyl)-acetyl]-sulfamic acid 4-(2-t-butoxycarbonylamino-acetylamino)-2,6-diisopropyl-phenyl ester;
[(2,4,6-Triisopropyl-phenyl)-acetyl]-sulfamic acid 4-(2-amino-acetylamino)-2,6-diisopropyl-phenyl ester;
[(2,4,6-Triisopropyl-phenyl)-acetyl]-sulfamic acid 4-(2-t-butoxycarbonylamino-4-methylsulfanyl-butyrylamino)-2,6-diisopropyl-phenyl ester;
[(2,4,6-Triisopropyl-phenyl)-acetyl]-sulfamic acid 4-(2-amino-4-methylsulfanyl-butyrylamino)-2,6-diisopropyl-phenyl ester trifluoroacetate;
3-[3-(3,5-Diisopropyl-4-{[(2,4,6-triisopropyl-phenyl)-acetyl]sulfamoyloxy}-phenyl)-ureido]-propionic acid ethyl ester;
3-[3-(3,5-Diisopropyl-4-{[(2,4,6-triisopropyl-phenyl)-acetyl]sulfamoyloxy}-phenyl)-ureido]-propionic acid;
[(2,4,6-Triisopropyl-phenyl)-acetyl]-sulfamic acid 4-[2-amino-3-(1H-indol-3-yl)-propionylamino]-2,6-diisopropyl-phenyl ester;
[(2,4,6-Triisopropyl-phenyl)-acetyl]-sulfamic acid 4-(5-amino-pentanoylamino)-2,6-diisopropyl-phenyl ester trifluoroacetate(1:1)(salt);
(D)-[(2,4,6-Triisopropyl-phenyl)-acetyl]-sulfamic acid 4-(2-amino-propionylamino)-2,6-diisopropyl-phenyl ester trifluoroacetate(1:1)(salt);
(L)-[(2,4,6-Triisopropyl-phenyl)-acetyl]-sulfamic acid 4-(2-amino-propionylamino)-2,6-diisopropyl-phenyl ester;
[(2,4,6-Triisopropyl-phenyl)-acetyl]-sulfamic acid 4-(2-amino-2-methyl-propionylamino)-2,6-diisopropyl-phenyl ester;
[(2,4,6-Triisopropyl-phenyl)-acetyl]-sulfamic acid 4-(3-dimethylamino-propoxy)-2,6-diisopropyl-phenyl ester;
[(2,4,6-Triisopropyl-phenyl)-acetyl]-sulfamic acid 4-(3-dimethylamino-propoxy)-2,6-diisopropyl-phenyl ester hydrochloride salt;
[(2,4,6-Triisopropyl-phenyl)-acetyl]-sulfamic acid 4-(3-amino-propoxy)-2,6-diisopropyl-phenyl ester hydrochloride salt;
[(2,4,6-Triisopropyl-phenyl)-acetyl]-sulfamic acid 2,6-diisopropyl-4-thiocyanato-phenyl ester;
[(2,4,6-Triisopropyl-phenyl)-acetyl]-sulfamic acid 4-cyano-2,6-diisopropyl-phenyl ester;
[(2,4,6-Triisopropyl-phenyl)-acetyl]-sulfamic acid 4-[(2-amino-acetylamino)-methyl]-2,6-diisopropyl-phenyl ester;
[(2,4,6-Triisopropyl-phenyl)-acetyl]-sulfamic acid 4-(benzylamino-methyl)-2,6-diisopropyl-phenyl ester mono hydrochloride;
[(2,4,6-Triisopropyl-phenyl)-acetyl]-sulfamic acid 4-carbamoyl-2,6-diisopropyl-phenyl ester;
[(2,4,6-Triisopropyl-phenyl)-acetyl]-sulfamic acid 4-hydroxymethyl-2,6-diisopropyl-phenyl ester;
[(2,4,6-Triisopropyl-phenyl)-acetyl]-sulfamic acid 4-acetylamino-2,6-diisopropyl-phenyl ester;
[(2,4,6-Triisopropyl-phenyl)-acetyl]-sulfamic acid 4-(2-hydroxyethylamino)-2,6-diisopropyl-phenyl ester;
[(2,4,6-Triisopropyl-phenyl)-acetyl]-sulfamic acid 4-[3-(2,6-diisopropylphenyl)-ureido]-2,6-diisopropyl-phenyl ester;
[(2,4,6-Triisopropyl-phenyl)-acetyl]-sulfamic acid 2,6-diisopropyl-4-(3-phenyl-ureido]-phenyl ester;
[(2,4,6-Triisopropyl-phenyl)-acetyl]-sulfamic acid 2,6-diisopropyl-4-(thiophene-2-sulfonylamino)-phenyl ester;
[(2,4,6-Triisopropyl-phenyl)-acetyl]-sulfamic acid 4-(5- dimethylaminonaphthalene-1-sulfonylamino)- 2,6-diisopropyl-phenyl ester;
[(2,4,6-Triisopropyl-phenyl)-acetyl]-sulfamic acid 2,6-diisopropyl-4-methanesulfonylamino-phenyl ester;
6-(3,5-Diisopropyl-4-{[(2,4,6-triisopropyl-phenyl-acetyl]sulfamoyloxy}-phenyl)-hexanoic acid ethyl ester; and
6-(3,5-Diisopropyl-4-{[(2,4,6-triisopropyl-phenyl-acetyl]sulfamoyloxy}-phenyl)-hexanoic acid.

Also preferred is a method of treating a disease or a disorder responsive to inhibition of nuclear factor-κB transcription factors, comprising administering to a patient in need thereof a sulfonylaminocarbonyl derivative or a pharmaceutically acceptable salt thereof selected from:
(9H-Xanthene-9-carbonyl)-sulfamic acid 2,6-diisopropyl-phenyl ester;
((E)-2-Methyl-3-phenyl-acryloyl)-sulfamic acid 2,6-diisopropyl-phenyl ester; and
(2-Oxo-2H-chromene-3-carbonyl)-sulfamic acid 2,6-diisopropyl-phenyl ester.

The sulfonylaminocarbonyl derivatives disclosed in United States Patent No. 5,254,715 and its divisional 5,336,690, which are both hereby incorporated herein by reference, are also useful in the present invention. Thus, another embodiment of the present invention is a method of treating a disease or a disorder responsive to inhibition of nuclear factor-κB transcription factors, comprising administering to a patient in need thereof a sulfonylaminocarbonyl derivative selected from:
Carbamic acid, [(phenylamino)sulfonyl]-, 2,6-bis(1-methylethyl)phenyl ester;
Carbamic acid, [(phenylamino)sulfonyl]-, 2,6-bis(1,1-dimethylethyl)-4-hydroxyphenyl ester;
Carbamic acid, [(phenylamino)sulfonyl]-, 2,6-bis(1,1-dimethylethyl)-phenyl ester;
Carbamic acid, [(didecylamino)sulfonyl]-, 2,6-bis(1,1-dimethylethyl)-4-methylphenyl ester;
Carbamic acid, [[bis(1-methylethyl)amino]sulfonyl]-, 2,6-bis(1-methylethyl)phenyl ester;
Carbamic acid, [(dipentylamino)sulfonyl]-, 2,6-bis(1-methylethyl)phenyl ester;
Carbamic acid, [[(diphenylmethyl)amino]sulfonyl]methyl-, 2,6-bis(1,1-dimethylethyl)phenyl ester;
DL-Tryptophan, ]a/-methyl-N-[[[(tricyclo[3.3.1.1^{3,7}]dec-2-yloxy)carbonyl]amino]sulfonyl]-, methyl ester;
Carbamic acid, sulfonylbis-, bis[2,6-bis(1-methylethyl)phenyl] ester;
Carbamic acid, [[[2-(phenylmethyl)phenyl]amino]sulfonyl]-, 2,6-bis(1,1-dimethylethyl)phenyl ester;
Methyl[[2,6-bis(1-methylethyl)phenyl amino]sulfonyl]carbamate;
Dodecyl[[2,6-bis(1-methylethyl)phenyl] amino]sulfonyl]carbamate;
2,6-Bis( 1,1-dimethylethyl)-4-methoxyphenyl[[(2,2-diphenylethyl)amino]-sulfonyl] carbamate;
2,6-Bis(1,1-dimethylethyl)-4-methoxy phenyl [[[2,6-bis(1-methylethyl)-phenyl]amino]sulfonyl] carbamate;
2,6-Bis(1,1-dimethylethyl)phenyl-[[(diphenylmethyl)amino]-sulfonyl]carbamate;
2,6-Bis(1,1-dimethylethyl)phenyl [[[2,6-bis(1-methylethyl)phenyl]amino]-sulfonyl] carbamate;
2,6-Bis(1,1-dimethylethyl)phenyl [[(2,2-diphenylethyl)amino]sulfonyl]-carbamate;
2,6-Bis(1,1-dimethylethyl)phenyl [[bis(phenylmethyl)amino]sulfonyl]-carbamate;
2,6-bis( 1-methylethyl)phenyl[(diphenyl-amino)sulfonyl]carbamate;
2,6-Bis( 1-methylethyl)phenyl[(dibutyl-amino)sulfonyl]carbamate;
2,6-Bis(1-methylethyl)phenyl[[bis(phenyl-methyl)amino]sulfonyl]-carbamate;
2,6-Bis(1-methylethyl)phenyl[( 1H-benzimidazol-2-ylamino)sulfonyl]-carbamate;
2,6-Bis( 1-methylethyl)phenyl[[2,2-diphenylethyl)amino]sulfonyl]-carbamate;
2,6-Bis(1-methylethyl)phenyl[[[2,6-bis( 1-methylethyl)phenyl]amino]-sulfonyl]carbamate;
2,6-Bis(1-methylethyl)phenyl[ [(diphenyl-methyl)amino] sulfonyl]-carbamate;
2,6-Bis( 1,1-dimethylethyl)-4-methyl-phenyl[[(diphenylmethyl)amino]-sulfonyl]carbamate;
2,6-Bis( 1,1-dimethylethyl)-4-methyl-phenyl[[[bis(2,6-bis( 1-methylethyl)-phenyl]amino] sulfonyl] carbamate;
2,6-Bis( 1,1-dimethylethyl)-4-methyl-phenyl[[(2,2-diphenylethyl)amino]-sulfonyl]-carbamate;
2,6-Bis(1,1-dimethylethyl)-4-methyl-phenyl [(dibutylamino)sulfonyl]-carbamate;
2,6-Bis(1,1-dimethylethyl)-4-methyl-phenyl [(dipentylamino)sulfonyl]-carbamate;
2,6-Bis( 1,1-dimethylethyl)-4-methyl-phenyl [[bis( 1-methylethyl)amino]-sulfonyl]carbamate;
2,6-Bis( 1,1-dimethylethyl)-4-methyl-phenyl[(dihexylamino)sulfonyl]-carbamate;
2,6-Bis( 1,1-dimethylethyl)-4-methyl-phenyl [(hexylamino)sulfonyl]-carbamate;
2,6-Bis(1,1-dimethylethyl)-4-methyl-phenyl[[methyl(2-phenylethyl)-amino] sulfonyl]carbamate;
2,6-Bis(1,1-dimethylethyl)-4-methyl-phenyl[[[bis-3-(dimethylamino)-propyl]amino]-sulfonyl]carbamate;
2,6-Bis(1,1-dimethylethyl)-4-methyl-phenyl[(methyl octyl amino)-sulfonyl]carbamate;
2,6-Bis(1,1-dimethylethyl)-4-methyl-[[bis[(tetrahydro-2-furanyl)methyl]-amino]sulfonyl]carbamate;
2,6-Bis(1,1-dimethylethyl)-4-methyl-phenyl[(dioctylamino)sulfonyl]-carbamate;
2,6-Bis(1,1-dimethylethyl)-4-methyl-phenyl[[[methyl 2-(2-pyridinyl)-ethyl]amino]sulfonyl]carbamate; hydrochloride salt,
2,6-Bis(1,1-dimethylethyl)-4-methyl-phenyl[[[methyl 2-(2-pyridinyl)-ethyl]amino]-sulfonyl]carbamate, sodium salt,
2,6-Bis(1,1-dimethylethyl)-4-methyl-phenyl[(dodecylamino)sulfonyl]-carbamate;
2,6-Bis(1-methylethyl)phenyl[[bis(1-methylethyl)amino]sulfonyl]-carbamate;
2,6-Bis(1-methylethyl)phenyl[[( 1-methylethyl)phenylmethyl)amino]-sulfonyl]carbamate;
2,6-Bis(1-methylethyl)phenyl[(hexyl-amino)sulfonyl]carbamate;
2,6-Bis(1-methylethyl)phenyl[(dioctyl-amino)sulfonyl]carbamate;
2,6-Bis(1-methylethyl)phenyl[[cyclo-hexyl( 1-methylethyl)amino]-sulfonyl]carbamate;
2,6-Bis(1-methylethyl)phenyl[(methyl-octylamino)sulfonyl]carbamate;
2,6-Bis(1-methylethyl)phenyl[(dihexyl-amino)sulfonyl]carbamate;
Dodecyl[[(2,4,6-trimethoxyphenyl)amino]-sulfonyl]carbamate;
2,6-Bis(1-methylethyl)phenyl ester(4-morpholinylsulfonyl)carbamic acid,
2,6-Bis(1-methylethyl)phenyl ester(1-piperidinylsulfonyl)carbamic acid;
2,6-Bis(1-methylethyl)phenyl ester(1-pyrrolidinylsulfonyl)carbamic acid;
2,6-Bis(1-methylethyl)phenyl ester[(2,3-dihydro- 1H-indol-1-yl)sulfonyl]-carbamic acid;
2,6-Bis( 1-methylethyl)phenyl[(dibutylamino)sulfonyl]carbamate monosodium salt; and
2,6-Bis( 1,1-dimethylethyl)phenyl[[(diphenylmethyl)amino]sulfonyl]-methyl carbamate.

The sulfonylaminocarbonyl derivatives disclosed in United States Patent No. 5,214,206 and its divisional 5,288,757, which are both hereby incorporated herein by reference, are also useful in the present invention. Thus, another embodiment of the present invention is a method of treating a disease or a disorder responsive to inhibition of nuclear factor-κB transcription factors, comprising administering to a patient in need thereof a sulfonylaminocarbonyl derivative selected from:
Urea, N-[2,6-bis(1-methylethyl)phenyl]-N'-[(dipropylamino)sulfonyl]-;
Urea, N-(2,2-dimethyl-4-phenyl-1,3-dioxan-5-yl)-N'-[[(tricyclo[3.3.1.1^{3,7}]dec-1-ylmethyl)amino]sulfonyl]-, (4S-*cis*)-;
Urea, N-(2,2-dimethyl-4-phenyl-1,3-dioxan-5-yl)-N'-[[(2,2-dimethyl-4-phenyl-1,3-dioxan-5-yl)amino]sulfonyl]-, stereoisomer;
N-[2,6-bis(1-methylethyl)phenyl]-N'-[[bis( 1-methylethyl)amino]-sulfonyl]urea;
N-[2,6-bis( 1-methylethyl)phenyl]-N'-[[(diphenylmethyl)amino]-sulfonyl]urea;
N-[2,6-bis(1-methylethyl)phenyl]-N'-[(diphenylamino)sulfonyl]urea;
N-[2,6-bis(1-methylethyl)phenyl]-N'-[(dibutylamino)sulfonyl]urea;
N-[[[2,6-bis(1-methylethyl)phenyl] amino]-sulfonyl]-N'-(diphenylmethyl)-urea;
N-[2,6-bis(1-methylethyl)phenyl]-N'-[[[2,6-bis(1-methylethyl)phenyl]-amino]sulfonyl]urea;
N-[2,6-bis(1-methylethyl)phenyl]-N'-[[(2,2-diphenylethyl)amino]-sulfonyl]urea;
N-[2,6-bis(1-methylethyl)phenyl]-N'-[(9H-fluoren-9-ylamino)sulfonyl]-urea;
N-[2,6-bis(1-methylethyl)phenyl]-N'-[[bis(phenylmethyl)amino]sulfonyl]-urea;
N-[2,6-bis( 1-methylethyl)phenyl]-N'-[[(1-methylethyl)(phenylmethyl)-amino] sulfonyl] urea;
N-[2,6-bis(1-methylethyl)phenyl)-N'-[(dioctylamino)sulfonyl]urea;
N-[2,6-bis( 1-methylethyl)phenyl]-N'-[(4-phenyl- 1-piperidinyl)-sulfonyl]urea;
N-[2,6-bis(1-methylethyl)phenyl]-N'-[(dihexylamino)sulfonyl]urea;
N-[[bis[3-(dimethylamino)propyl]amino]-sulfonyl]-N'-[2,6-bis(1-methylethyl)phenyl] urea;
N-[2,6-bis( 1-methylethyl)phenyl]-N'-[(hexylamino)sulfonyl]urea;
N-[2,6-bis( 1-methylethyl)phenyl]-N'-[[bis-[(tetrahydro-2-furanyl)methyl]amino]sulfonyl]-urea;
N-[2,6-bis(1-methylethyl)phenyl]-N'-[(diethylamino)sulfonyl]urea;
N-[2,6-bis(1-methylethyl)phenyl]-N'-[(methyloctyl amino)sulfonyl]urea;
N-[2,6-bis( 1-methylethyl)phenyl]-N'-[[cyclohexyl( 1-methylethyl)amino]-sulfonyl]urea;
N-[2,6-bis(1-methylethyl)phenyl]-N'-[(dipentylamino)sulfonyl]urea;
N-[2,6-bis(1-methylethyl)phenyl]-N'-[[bis(2-methylpropyl)amino]-sulfonyl]urea;
N-[2,6-bis( 1-methylethyl)phenyl]-N'-[[ethyl(2-propenyl)amino]-sulfonyl]urea;
N-[[bis(3-methylbutyl)amino] sulfonyl]-N'-[2,6-bis( 1-methylethyl)-phenyl]urea;
N-[2,6-bis( 1-methylethyl)phenyl)-N'-[(didecylamino)sulfonyl]urea;
N-[2,6-bis( 1-methylethyl)phenyl]-N'-[(didodecylamino)sulfamoyl]urea;
N-[2,6-bis(1-methylethyl)phenyl]-N'-[(diisopropylamino)sulfonyl]urea;
N-[2,6-bis(1-methylethyl)phenyl]-N'-[(dicyclohexylamino)sulfonyl]urea;
N-[2,6-bis( 1-methylethyl)phenyl]-N'-[(methyloctadecylamino)-sulfonyl]urea;
N-[2,6-bis(1-methylethyl)phenyl]-N'-[(di-2-propenylamino)sulfonyl]urea;
N-[2,6-bis( 1-methylethyl)phenyl]-N'-[[( 1;1-dimethylethyl)(1-methylethyl)amino]sulfonyl]-urea;
N-[2,6-bis(1 -methylethyl)phenyl]-N'-[[bis(1-methylpropyl)amino]-sulfonyl]urea;
N-[2,6-bis( 1-methylethyl)phenyl]-N'-[(methyltetradecylamino)-sulfonyl]urea;
N-[2,6-bis(1-methylethyl)phenyl]-N'-(1-pyrrolidinylsulfonyl) urea;
N-[2,6-bis(1-methylethyl)phenyl]-N'-(1-piperidinylsulfonyl) urea;
N'-[[[2,6-bis(1-methylethyl)phenyl]amino]sulfonyl]-N;N-bis(phenylmethyl) urea;
N-[2,6-bis(1-methylethyl)phenyl]-N'-[(dibutylamino)sulfonyl]urea; monosodium salt; and
N'-[2,6-bis(1-methylethyl)phenyl]-N-methyl-[(dibutylamino)sulfonyl]urea.

The sulfonylaminocarbonyl derivatives disclosed in United States patent no. 5,198,466 and its divisional 5,364,882, which are both hereby incorporated herein by reference, are also useful in the present invention. Thus, another embodiment of the present invention is a method of treating a disease or a disorder responsive to inhibition of nuclear factor-κB transcription factors, comprising administering to a patient in need thereof a sulfonylaminocarbonyl derivative selected from:
Sulfamic acid, [[[2,4,6-tris(1-methylethyl)phenyl]amino]-carbonyl]-, 2,6-bis(1-methylethyl)phenyl ester;
Sulfamic acid, [[[[1-[4-(dimethylamino)phenyl]cyclopentyl]methyl]-amino]carbonyl]-, 2,6-bis(1-methylethyl)phenyl ester;
(2,3-Dihydro-indole- 1-carbonyl)-sulfamic acid 2,6-diisopropyl-phenyl ester;
Sulfamic acid, [[(triphenylmethyl)amino]carbonyl]-, 2,6-bis(1-methylethyl)phenyl ester;
Octadecyl [[[2,6-bis(1-methylethyl)phenyl]-amino]carbonyl]sulfamate;
Dodecyl-N-[[[2,6-bis(1-methylethyl)phenyl]-amino]carbonyl]sulfamate;
Decyl [[[2,6-bis(1-methylethyl)phenyl]amino]carbonyl]sulfamate;
(±) 1-Methylheptyl [[[2,6-bis(1-methylethyl)phenyl]amino]carbonyl]-sulfamate;
2,6-Bis(1-methylethyl)phenyl [[[2,6-bis(1-methylethyl)phenyl]amino]-carbonyl]sulfamate;
(±) 1-Methylundecyl [[[2,6-bis(1-methylethyl)phenyl]amino]carbonyl]-sulfamate; and
Dodecyl [[[2,6-bis(1-methylethyl)phenyl]amino]carbonyl]sulfamate, sodium salt.

The sulfonylaminocarbonyl derivatives disclosed in United States Patent No. 5,245,068 and its divisional 5,384,328, which are both hereby incorporated herein by reference, are also useful in the present invention. Thus, another embodiment of the present invention is a method of treating a disease or a disorder responsive to inhibition of nuclear factor-κB transcription factors, comprising administering to a patient in need thereof a sulfonylaminocarbonyl derivative selected from:
Carbamic acid, [(dodecyloxy)sulfonyl]-, dodecyl ester;
Carbamic acid, [(dodecyloxy)sulfonyl]-, [1,1':3',1"-terphenyl]-2'-yl ester;
Carbamothioic acid, [(dodecyloxy)sulfonyl]-, S-[2,6-bis(1-methylethyl)-phenyl] ester;
Carbamic acid, (phenoxysulfonyl)-, 2,6-bis(1-methylethyl)phenyl ester;
Carbamic acid, [(2,6-dimethylphenoxy)sulfonyl]-, 2,6-bis(1-methylethyl)-phenyl ester;
Carbamic acid, [[2,6-bis(1,1-dimethylethyl)phenoxy]sulfonyl]-, 2,6-bis(1,1-dimethylethyl)phenyl ester;
Carbamic acid, [[2,6-bis(1,1-dimethylethyl)phenoxy]sulfonyl]-, 2,6-bis(1-methylethyl)phenyl ester;
Carbamic acid, [(2,6-difluorophenoxy)sulfonyl]-, 2,6-bis(1-methylethyl)-phenyl ester;
Carbamic acid, [(hexadecyloxy)sulfonyl]-, 2,6-bis(1-methylethyl)phenyl ester;
Carbamic acid, [[2,6-bis(1-methylethyl)phenoxy]sulfonyl]-, 2,6-dimethoxyphenyl ester;
Carbamic acid, [[2,6-bis(1-methylethyl)phenoxy]sulfonyl]-, 1-methylheptyl ester;
Carbamic acid, [[2,6-bis(1-methylethyl)phenoxy]sulfonyl]-, 2,6-bis(1-methylethyl)-4-nitrophenyl ester;
Carbamic acid, [[2,6-bis(1-methylethyl)phenoxy]sulfonyl]-, 1,2-ethanediyl ester;
Carbamic acid, [[2,6-bis(1-methylethyl)phenoxy]sulfonyl]-, 1,2,3-propanetriyl ester;
Carbamic acid, [[2,6-bis(1-methylethyl)phenoxy]sulfonyl]-, 4-bromo-2,6-bis(1-methylethyl)phenyl ester;
Carbamic acid, [[2,6-bis(1-methylethyl)phenoxy]sulfonyl]-, [1,1':3',1"-terphenyl]-2'-yl ester;
Carbamic acid, [[2,6-bis(1-methylethyl)phenoxy]sulfonyl]-, 2,6-bis( 1,1dimethylethyl)-4-methoxyphenyl ester;
Carbamic acid, [[2,6-bis(1-methylethyl)phenoxy]sulfonyl]-, 4-fluoro-2,3,5,6-tetrakis(1-methylethyl)phenyl ester;
Carbamic acid, [[2,6-bis(1-methylethyl)phenoxy]sulfonyl]-, 4-chloro-2,6-bis(1-methylethyl)phenyl ester;
Stigmasta-5,22-dien-3-ol, [[2,6-bis(1-methylethyl)phenoxy]sulfonyl]-carbamate, (3α)-;
Carbamic acid, [[2,6-bis(1-methylethyl)phenoxy]sulfonyl]-, 2,6-bis(1,1-dimethylethyl)-4-methylphenyl ester;
Stigmastan-3-ol, [[2,6-bis(1-methylethyl)phenoxy]sulfonyl]carbamate, (3α)-;
Carbamic acid, [[2,6-bis(1-methylethyl)phenoxy]sulfonyl]-, 4-methoxy-2,6-bis( 1-methylethyl)phenyl ester;
Carbamic acid, [[2,6-bis(1-methylethyl)phenoxy]sulfonyl]-, 2,4,6-tris(1-methylethyl)phenyl ester;
Carbamic acid, [[2,4,6-tris(1-methylethyl)phenoxy]sulfonyl]-, 2,6-bis(1-methylethyl)phenyl ester;
Carbamic acid, [[2,4,6-tris(1-methylethyl)phenoxy]sulfonyl]-, 2,4,6-tris(1-methylethyl)phenyl ester;
Carbamic acid, [[2,6-bis(1-methylethyl)phenoxy]sulfonyl]-, 2,4,6-tris(1,1-dimethylethyl)phenyl ester;
Carbamic acid, [[2,6-bis(1-methylethyl)phenoxy]sulfonyl]-, 4-[[3,5-bis( 1,1-dimethylethyl)-4-hydroxyphenyl]dithio]-2,6-bis(1,1-dimethylethyl)phenyl ester;
Carbamic acid, [[2,6-bis(1-methylethyl)phenoxy]sulfonyl]-, 2,4-bis(1-methylethyl)phenyl ester;
Carbamic acid, [[2,6-bis(1-methylethyl)phenoxy]sulfonyl]-, 4-[(dimethylamino)methyl]-2,6-bis(1-methylethyl)phenyl ester;
Carbamic acid, [[2,6-bis(1-methylethyl)phenoxy]sulfonyl]-, tricyclo[3.3.1.1^{3,7}]dec-2-yl ester;
Carbamic acid, [[2,6-bis(1-methylethyl)phenoxy]sulfonyl]-, 4-hydroxy-2,6-bis(1-methylethyl)phenyl ester;
Carbamic acid, [[2,6-bis(1-methylethyl)phenoxy]sulfonyl]-, cyclohexyl ester;
Carbamic acid, [[2,6-bis(1-methylethyl)phenoxy]sulfonyl]-, 3,3',5,5'-tetrakis(1-methylethyl)[1,1'-biphenyl]-4,4'-diyl ester;
Carbamic acid, [[4-hydroxy-2,6-bis(1-methylethyl)phenoxy]sulfonyl]-, 2,6-bis(1-methylethyl)phenyl ester;
Carbamic acid, [[2,6-bis(1-methylethyl)phenoxy]sulfonyl]-, tricyclo[3.3.1.1^{3,7}]dec-1-yl ester;
Carbamic acid, [[2,6-bis(1-methylethyl)phenoxy]sulfonyl]-, 2-(1,1-dimethylethyl)-6-methylphenyl ester;
Carbamic acid, [[2,6-bis(1-methylethyl)phenoxy]sulfonyl]-, 5-methyl-2-(1-methylethyl)cyclohexyl ester;
Carbamothioic acid, [[2,6-bis(1-methylethyl)phenoxy]sulfonyl]-, *S*-[2,6-bis(1-methylethyl)phenyl] ester;
Carbamic acid, [[2,6-bis(1-methylethyl)phenoxy]sulfonyl]-, (2,6-diethylphenyl)methyl ester;
Carbamic acid, [[2,6-bis(1-methylethyl)phenoxy]sulfonyl]-, (2*S*,6*S*)-2,6-bis(1-methylethyl)cyclohexyl ester;
Carbamic acid, [[2,6-bis(1-methylethyl)phenoxy]sulfonyl]-, 4-(1,1-dimethylethyl)-2,6-(1-methylethyl)phenyl ester;
Carbamic acid, [[2,6-bis(1-methylethyl)phenoxy]sulfonyl]-, 4-fluorophenyl ester;
Carbamic acid, [[2,6-bis(1-methylethyl)phenoxy]sulfonyl]-, 2,4-difluorophenyl ester;
Carbamic acid, [[2,6-bis(1-methylethyl)phenoxy]sulfonyl]-, pentafluorophenyl ester;
Carbamic acid, [[2,6-bis(1-methylethyl)phenoxy]sulfonyl]-, 2,6-difluorophenyl ester;
Carbamic acid, [[2,6-bis(1-methylethyl)phenoxy]sulfonyl]-, (2*R*,6*S*)-2,6-bis(1-methylethyl)cyclohexyl ester;
Carbamic acid, [[2,6-bis(1-methylethyl)phenoxy]sulfonyl]-, 2,3,5,6-tetramethylphenyl ester;
Carbamic acid, [[2,6-bis(1-methylethyl)phenoxy]sulfonyl]-, 3-pyridinyl ester;
Carbamic acid, [[2,6-bis(1-methylethyl)phenoxy]sulfonyl]-, 2,6-dimethylphenyl ester;
Carbamic acid, [[2,6-bis(1-methylethyl)phenoxy]sulfonyl]-, 4-acetyl-2,6-bis(1-methylethyl)phenyl ester;
Carbamic acid, [[2,6-bis(1-methylethyl)phenoxy]sulfonyl]-, 4-fluoro-2,6-bis(1-methylethyl)phenyl ester;
2,6-Bis(1-methylethyl)phenyl[[2,6-bis(1-methylethyl)phenoxy]-sulfonyl]-carbamate;
2,6-Bis(1,1-dimethylethyl)-4-methylphenyl (phenoxysulfonyl)carbamate;
2,6-Bis(1,1-dimethylethyl)-4-methylphenyl[(hexyloxy)sulfonyl]carbamate;
2,6-Bis(1,1-dimethylethyl)-4-methylphenyl[(dodecyloxy-sulfonyl]-carbamate;
Dodecyl [[2,6-bis(1-methylethyl)phenoxy]-sulfonyl]carbamate;
Methyl[[2,6-bis(1-methylethyl)phenoxy]-sulfonyl]carbamate;
2,6-Bis( 1-methylethyl)phenyl[(hexyloxy)-sulfonyl]carbamate;
2,6-Bis(1-methylethyl)phenyl](dodecyloxy)-sulfonyl]carbamate; and
2,6-Bis(1,1-dimethylethyl)phenyl[[2,6-bis(1-methylethyl)phenoxy]-sulfonyl]carbamate.

The sulfonylaminocarbonyl derivatives disclosed in United States Patent No. 5,254,589 and its continuation 5,981,595, which are both hereby incorporated herein by reference, are also useful in the present invention. Thus, another embodiment of the present invention is a method of treating a disease or a disorder responsive to inhibition of nuclear factor-κB transcription factors, comprising administering to a patient in need thereof a sulfonylaminocarbonyl derivative selected from:
N-[2,6-bis(1-methylethyl)phenyl]-N'-(6-ethoxy-2-benzothiazolyl)-sulfonyl]-urea;
N-[2,6-bis(1-methylethyl)phenyl]-N'-(2-octadecylsulfonyl)urea;
N-[2,4,6-triemthoxyphenyl]-N'-(2-octadecylsulfonyl)urea;
N-[2,6-bis(1-methylethyl)phenyl]-N'-(tetradecylsulfonyl)urea;
N-[2,6-bis(1-methylethyl)phenyl]-N'-methyl-N'-(tetradecylsulfonyl)urea;
N-[2,6-bis(1-methylethyl)phenyl]-N'-(dodecylsulfonyl)urea;
N-[2,6-bis(1-methylethyl)phenyl]-N'-(hexadecylsulfonyl)urea;
N-[2,6-bis(1-methylethyl)phenyl]-N'-methyl-N'-(dodecylsulfonyl)urea;
N-[2,6-bis(1-methylethyl)phenyl]-N'-(tridecylsulfonyl)urea;
N-[2,4,6-trimethoxyphenyl]-N'-(hexadecylsulfonyl)urea;
N-[2,6-bis(1-methylethyl)phenyl]-N'-(2-methyl-2-pentadecylsulfonyl)urea;
N-2,6-bis(1-methylethyl)phenyl-N'-(dodecylsulfonyl)urea;
N-[2,6-bis(1-methylethyl)phenyl]-N'-( 1-phenyl-1-tetradecylsulfonyl)urea;
N-[2,6-bis(1-methylethyl)phenyl]-N'-(1-phenyl-1-nonylsulfonyl)urea; and
N-[2,6-bis(1-methylethyl)phenyl]-N'-(2-decylsulfonyl)urea.

The following sulfonylaminocarbonyl derivatives are excluded from use in the methods of the present invention:
Urea, N-[2,6-bis(1-methylethyl)phenyl]-N'-[(dimethylamino)sulfonyl]-;
Sulfamic acid, [[[2,6-bis(1-methylethyl)phenyl]amino]carbonyl]-, hexyl ester;
Urea, N-[2,6-bis(1-methylethyl)phenyl]-N'-[[methyl(2-phenylethyl)-amino]sulfonyl]-;
Carbamic acid, [(4-methyl-1-piperazinyl)sulfonyl]-, 2,6-bis(1-methylethyl)phenyl ester, monohydrochloride;
Urea, N-[2,6-bis(1-methylethyl)phenyl]-N'-[(butylmethylamino)sulfonyl]-;
Urea, N-[2,6-bis(1-methylethyl)phenyl]-N'-[[(1-methylethyl)amino]-sulfonyl]-;
Urea, N-[2,6-bis(1-methylethyl)phenyl]-N'-[(butylethylamino)sulfonyl]-;
Carbamic acid, [[[2,6-bis(1-methylethyl)phenyl]amino]sulfonyl]-, [1,1':3',1"-terphenyl]-2'-yl ester;
Carbamic acid, [(2,6-dimethoxyphenoxy)sulfonyl]-, 2,6-bis(1-methylethyl)phenyl ester;
Carbamic acid, [(2,4-difluorophenoxy)sulfonyl]-, 2,6-bis(1-methylethyl)-phenyl ester;
Carbamic acid, [(2,4,6-trimethoxyphenoxy)sulfonyl]-, 2,6-bis(1-methylethyl)phenyl ester;
Carbamic acid, [(2,6-dimethoxyphenoxy)sulfonyl]-, 2,6-dimethoxyphenyl ester;
Carbamic acid, [[2,6-bis(1-methylethyl)phenoxy]sulfonyl]methyl-, 2,6-bis(1-methylethyl)phenyl ester;
Carbamic acid, [[2,6-bis(1-methylethyl)phenoxy]sulfonyl]-, ethyl ester, sodium salt;
[3-(2,4,6-Triisopropyl-phenyl)-propionyl)-sulfamic acid 2,6-diisopropyl-phenyl ester;
[Fluoro-(2,4,6-triisopropyl-phenyl)-acetyl]-sulfamic acid 2,6-diisopropyl-phenyl ester;
[(2,4,6-Triisopropyl-phenyl)-acetyl]-sulfamic acid [1,1';3',1"]terphenyl-2'-yl ester;
Carbamic acid, [[2,6-bis(1-methylethyl)phenoxy]sulfonyl]-, 4-chlorophenyl ester;
Carbamic acid, [[2,6-bis(1-methylethyl)phenoxy]sulfonyl]-, (3-pyridinyl)methyl ester;
[(2,4,6-Triisopropyl-phenyl)-acetyl]-sulfamic acid 4-chloro-2,6-diisopropyl-phenyl ester;
[(2,4,6-Triisopropyl-phenyl)-acetyl]-sulfamic acid 2,6-diisopropyl-4-(3-phenyl-thioureido)-phenyl ester;
[(2,4,6-Triisopropyl-phenyl)-acetyl]-sulfamic acid 4-formyl-2,6-diisopropyl-phenyl ester;
[2-(2,4,6-Triisopropyl-phenyl)-acetyl]-sulfamic acid 4-((R)-2-amino-4-methyl-pentanoylamino)-2,6-diisopropyl-phenyl ester; compound with trifluoro-acetic acid;
[(2,4,6-Triisopropyl-phenyl)-acetyl]-sulfamic acid 4-[bis-(2-hydroxyethyl)-amino]-2,6-diisopropyl-phenyl ester;
[(2,4,6-Triisopropyl-phenyl)-acetyl]-sulfamic acid 2,6-diisopropyl-4-sulfamoyl-phenyl ester;
Benzeneacetamide, N-[[[2,6-bis(1-methylethyl)phenyl]amino]sulfonyl]-2,4,6-tris( 1-methylethyl)-;
[2-(2,4,6-Triisopropyl-phenyl)-acetyl]-sulfamic acid 2,6-diisopropyl-4-(4-methyl-piperazin-1-ylmethyl)-phenyl ester; compound with generic inorganic neutral component;
[2-(2,4,6-Triisopropyl-phenyl)-acetyl]-sulfamic acid 4-aminomethyl-2,6-diisopropyl-phenyl ester; compound with generic inorganic neutral component;
[2-(2,4,6-Triisopropyl-phenyl)-acetyl]-sulfamic acid 4-((S)-2-amino-3-phenyl-propionylamino)-2,6-diisopropyl-phenyl ester; compound with trifluoro-acetic acid;
[2-(2,4,6-Triisopropyl-phenyl)-acetyl]-sulfamic acid 4-((S)-2-amino-3-methyl-pentanoylamino)-2,6-diisopropyl-phenyl ester; compound with trifluoro-acetic acid;
(2,3-Diphenyl-acryloyl)-sulfamic acid 3,4-dichloro-phenyl ester;
(4-Phenyl-but-3-enoyl)-sulfamic acid 2,6-diisopropyl-phenyl ester;
N-[2,6-bis(1-methylethyl)phenyl]-N'-phenylmethyl-N'-(tetradecylsulfonyl)urea;
N-[2,6-bis(1-methylethyl)phenyl]-N'-(octylsulfonyl)urea;
N-(2,4-difluorophenyl)-N'-(tetradecylsulfonyl)urea;
N-[2,6-bis( 1-methylethyl)phenyl]-N'-(decylsulfonyl)urea;
N-[2,6-bis(1-methylethyl)phenyl]-N'-(2-pentadecylsulfonyl)urea;
N-[2,6-bis(1-methylethyl)phenyl)-N'-[[6-(2,3-dihydro-1,3-dioxo-1H-isoindol-2-yl)hexyl]sulfonyl]urea;
N-[[[2,6-bis(1-methylethyl)phenyl]amino-carbonyl]-14-heptacosanesulfonamide; and
N-[2,4,6-trimethoxyphenyl]-N'-(tetradecylsulfonyl)urea.

Another embodiment of the invention is a method of inhibiting NF-κB transcription factors in an animal, comprising administering to the animal an NF-κB inhibiting amount of a sulfonylaminocarbonyl derivative, or a pharmaceutically acceptable salt thereof.

Another invention embodiment is use of a sulfonylaminocarbonyl derivative, or a pharmaceutically acceptable salt thereof, in the manufacture of a medicament for the treatment of a disease or a disorder responsive to inhibition of nuclear factor-κB transcription factors.

Another invention embodiment is a pharmaceutical composition, comprising a nuclear factor-κB transcription factor inhibiting amount of a sulfonylaminocarbonyl derivative, or a pharmaceutically acceptable salt thereof.

Another embodiment of the present invention is a method of treating a disease or a disorder responsive to inhibition of nuclear factor-κB transcription factors, comprising administering to a patient in need thereof a sulfonylaminocarbonyl derivative, or a pharmaceutically acceptable salt thereof,
wherein the disease or disorder being treated is rheumatoid arthritis, osteoarthritis, an autoimmune disease, psoriasis, asthma, a cardiovascular disease, an acute coronary syndrome, congestive heart failure, Alzheimer's disease, multiple sclerosis, cancer, type II diabetes, metabolic syndrome X, or inflammatory bowel disease.

Preferred is a method of treating a disease or a disorder responsive to inhibition of nuclear factor-κB transcription factors, comprising administering to a patient in need thereof a sulfonylaminocarbonyl derivative, or a pharmaceutically acceptable salt thereof, wherein the disease or disorder being treated is rheumatoid arthritis, osteoarthritis, systemic lupus erythematosus, Grave's disease, myasthenia gravis, insulin resistance, autoimmune hemolytic anemia, scleroderma with anti-collagen antibodies (Abs), pernicious anemia, diabetes mellitus, psoriasis, asthma, atherosclerosis, myocardial infarction, unstable angina, congestive heart failure, Alzheimer's disease, multiple sclerosis, cancer, type II diabetes, metabolic syndrome X, or inflammatory bowel disease.

More preferred is a method of treating a disease or a disorder responsive to inhibition of nuclear factor-κB transcription factors, comprising administering to a patient in need thereof a sulfonylaminocarbonyl derivative, or a pharmaceutically acceptable salt thereof, wherein the disease or disorder being treated is rheumatoid arthritis.

Also more preferred is a method of treating a disease or a disorder responsive to inhibition of nuclear factor-κB transcription factors, comprising administering to a patient in need thereof a sulfonylaminocarbonyl derivative, or a pharmaceutically acceptable salt thereof, wherein the disease or disorder being treated is osteoarthritis.

Also more preferred is a method of treating a disease or a disorder responsive to inhibition of nuclear factor-κB transcription factors, comprising administering to a patient in need thereof a sulfonylaminocarbonyl derivative, or a pharmaceutically acceptable salt thereof, wherein the disease or disorder being treated is insulin resistance.

Also more preferred is a method of treating a disease or a disorder responsive to inhibition of nuclear factor-κB transcription factors, comprising administering to a patient in need thereof a sulfonylaminocarbonyl derivative, or a pharmaceutically acceptable salt thereof, wherein the disease or disorder being treated is asthma.

Also more preferred is a method of treating a disease or a disorder responsive to inhibition of nuclear factor-κB transcription factors, comprising administering to a patient in need thereof a sulfonylaminocarbonyl derivative, or a pharmaceutically acceptable salt thereof, wherein the disease or disorder being treated is atherosclerosis.

Also more preferred is a method of treating a disease or a disorder responsive to inhibition of nuclear factor-κB transcription factors, comprising administering to a patient in need thereof a sulfonylaminocarbonyl derivative, or a pharmaceutically acceptable salt thereof, wherein the disease or disorder being treated is myocardial infarction.

Also more preferred is a method of treating a disease or a disorder responsive to inhibition of nuclear factor-κB transcription factors, comprising administering to a patient in need thereof a sulfonylaminocarbonyl derivative, or a pharmaceutically acceptable salt thereof, wherein the disease or disorder being treated is unstable angina.

Also more preferred is a method of treating a disease or a disorder responsive to inhibition of nuclear factor-κB transcription factors, comprising administering to a patient in need thereof a sulfonylaminocarbonyl derivative, or a pharmaceutically acceptable salt thereof, wherein the disease or disorder being treated is congestive heart failure.

Also more preferred is a method of treating a disease or a disorder responsive to inhibition of nuclear factor-κB transcription factors, comprising administering to a patient in need thereof a sulfonylaminocarbonyl derivative, or a pharmaceutically acceptable salt thereof, wherein the disease or disorder being treated is Alzheimer's disease.

Also more preferred is a method of treating a disease or a disorder responsive to inhibition of nuclear factor-κB transcription factors, comprising administering to a patient in need thereof a sulfonylaminocarbonyl derivative, or a pharmaceutically acceptable salt thereof, wherein the disease or disorder being treated is cancer.

Also more preferred is a method of treating a disease or a disorder responsive to inhibition of nuclear factor-κB transcription factors, comprising administering to a patient in need thereof a sulfonylaminocarbonyl derivative, or a pharmaceutically acceptable salt thereof, wherein the disease or disorder being treated is inflammatory bowel disease.

Also more preferred is a method of treating a disease or a disorder responsive to inhibition of nuclear factor-κB transcription factors, comprising administering to a patient in need thereof a sulfonylaminocarbonyl derivative, or a pharmaceutically acceptable salt thereof, wherein the disease or disorder being treated is multiple sclerosis.

Also more preferred is the invention method of treating a disease or a disorder responsive to inhibition of nuclear factor-κB transcription factors, comprising administering to a patient in need thereof a sulfonylaminocarbonyl derivative, or a pharmaceutically acceptable salt thereof, wherein the disease or disorder being treated is type II diabetes.

Also more preferred is the invention method of treating a disease or a disorder responsive to inhibition of nuclear factor-κB transcription factors, comprising administering to a patient in need thereof a sulfonylaminocarbonyl derivative, or a pharmaceutically acceptable salt thereof, wherein the disease or disorder being treated is metabolic syndrome X.

Another embodiment of the present invention is a method for screening compounds in vitro for their ability to inhibit NF-κB mediated transcription of a gene, comprising analyzing an assay mixture containing stimulated NF-κB using fluorescence detection.

Preferred is a method for screening compounds in vitro for their ability to inhibit NF-κB mediated transcription of a gene, comprising analyzing an assay mixture containing stimulated NF-κB using fluorescence detection wherein the assay is a cell-based assay.

Also preferred is a method for screening compounds in vitro for their ability to inhibit NF-κB mediated transcription of a gene, comprising analyzing an assay mixture containing stimulated NF-κB using fluorescence detection, wherein the assay is a cell-based assay and is performed in high throughput screening mode.

More preferred is a method for screening compounds in vitro for their ability to inhibit NF-κB mediated transcription of a gene, comprising analyzing an assay mixture containing stimulated NF-κB using fluorescence detection, the assay comprising:
Step a) Stably transfecting into cells an NF-κB binding site and a plasmid vector containing cDNA for an enzyme capable of cleaving a nonfluorescent substrate to produce a fluorescent cleavage product, an enzyme capable of cleaving a fluorescent substrate to produce a nonfluorescent cleavage product, or an enzyme capable of cleaving a fluorescent substrate to produce a fluorescent cleavage product;
Step b) Plating the cells of Step a) in media;
Step c) Incubating the mixture of plated cells of Step b);
Step d) Stimulating the cells of Step c) with a cytokine or a mixture of a cytokine and a compound being tested for NF-κB inhibition;
Step e) Adding a fluorescent disclosing reagent to the stimulated cells of Step d); and
Step f) Analyzing the mixture of Step e) by fluorescence detection.

Still more preferred is a method for screening compounds in vitro for their ability to inhibit NF-κB mediated transcription of a gene, comprising analyzing an assay mixture containing stimulated NF-κB using fluorescence detection, the assay comprising:
Step a) Stably transfecting into cells an NF-κB binding site and a plasmid vector containing cDNA for an enzyme capable of cleaving a nonfluorescent substrate to produce a fluorescent cleavage product, an enzyme capable of cleaving a fluorescent substrate to produce a nonfluorescent cleavage product, or an enzyme capable of cleaving a fluorescent substrate to produce a fluorescent cleavage product;
Step b) Plating the cells of Step a) in media;
Step c) Incubating the mixture of plated cells of Step b);
Step d) Stimulating the cells of Step c) with a cytokine or a mixture of a cytokine and a compound being tested for NF-κB inhibition;
Step e) Adding a fluorescent disclosing reagent to the stimulated cells of Step d); and
Step f) Analyzing the mixture of Step e) by fluorescence detection,
wherein:
the cells undergoing transfection in Step a) are ECV-304 cells;
the cDNA being transfected in Step a) codes for β-lactamase;
the NF-κB binding site being transfected in Step a) is an HIV NF-κB binding site;
the cytokine employed in Step c) is TNF-α or IL-1β; or
the fluorescent disclosing reagent employed in Step e) is a CCF2 dye.

### DETAILED DESCRIPTION OF THE INVENTION

As discussed above, the present invention provides a method of treating a disease or a disorder responsive to inhibition of nuclear factor-κB transciption factors comprising administering to patients in need thereof a sulfonylaminocarbonyl derivative, or a pharmaceutically acceptable salt thereof.

While as mentioned above, some of the sulfonylaminocarbonyl derivatives useful in the methods of the present invention are also inhibitors of the enzyme ACAT, and accordingly have demonstrated serum and plasma cholesterol and Lp(a) regulating activities in vivo, no connection exists between these activities and the ability of the sulfonylaminocarbonyl derivatives to inhibit NF-κB mediated transcription and thereby treat diseases and disorders responsive to inhibition of NF-κB.

In Formula I above, illustrative examples of straight or branched saturated hydrocarbon chains having from 1 to 20 carbon atoms include methyl, ethyl, n-propyl, isopropyl, n-butyl, iso-butyl, tert-butyl, n-pentyl, isopentyl, n-hexyl, n-heptyl, n-octyl, n-undecyl, n-dodecyl, n-hexadecyl, 2,2-dimethyldodecyl, 2-tetradecyl, and n-octadecyl groups.

Illustrative examples of straight or branched hydrocarbon chains having from 1 to 20 carbon atoms and having from 1 to 3 double bonds include ethenyl, 2-propenyl, 2-butenyl, 3-pentenyl, 2-octenyl, 5-nonenyl, 4-undecenyl, 5-heptadecenyl, 3-octadecenyl, 9-octadecenyl, 2,2-dimethyl-11-eicosenyl, 9,12-octadecadienyl, and hexadecenyl.

Straight or branched alkoxy groups having from 1 to 6 carbon atoms include, for example, methoxy, ethoxy, n-propoxy, t-butoxy, and pentyloxy.

Illustrative examples of straight or branched alkyl groups having from 1 to 6 carbon atoms as used in Formula I include methyl, ethyl, n-propyl, isopropyl, n-pentyl, n-butyl, and tert-butyl.

Illustrative examples of cycloalkyl groups, as used in Formula I, include cyclopentyl, cyclohexyl, cyclooctyl, tetrahydronaphthyl, and 1- or 2-adamantyl.

Spirocycloalkyl groups are, for example, spirocyclopropyl, spirocyclobutyl, spirocyclopentyl, and spirocyclohexyl.

Illustrative examples of arylalkyl groups are: benzyl, phenethyl, 3-phenylpropyl, 2-phenylpropyl, 4-phenylbutyl, 2-phenylbutyl, 3-phenylbutyl, benzhydryl, 2,2-diphenylethyl, and 3,3-diphenylpropyl.

In Formula II above, illustrative examples of straight or branched carbon chains having from 1 to 10 carbon atoms include methyl, ethyl, n-propyl, isopropyl, n-butyl, isobutyl, tert-butyl, n-pentyl, isopentyl, n-hexyl, n-heptyl, and n-octyl.

Alkoxy means straight or branched groups having from 1 to 6 carbon atoms include, for example, methoxy, ethoxy, n-propoxy, t-butoxy, and pentyloxy.

The natural (essential) amino acids are: valine, leucine, isoleucine, threonine, methionine, phenylalanine, tryptophan, lysine, alanine, aginine, aspartic acid, cysteine, glutamic acid, glycine, histidine, proline, serine, tyrosine, asparagine, and glutamine.

Preferred natural amino acids are: valine, leucine, isoleucine, threonine, lysine, alanine, glycine, serine, asparagine, and glutamine.

Phenyl, naphthyl, and heteroaromatic rings are unsubstituted or substituted by from 1 to 5 substituents selected from alkyl of from 1 to 6 carbons, alkoxy, halogen, nitro, cyano, carboxylic acids and alkyl esters, amino, and hydroxyl.

Heteroaromatic rings are, for example, 2-, 3-, or 4-pyridinyl; 2-, 4-, or 5-pyrimidinyl; 2- or 3-thienyl; isoquinolines, quinolines, pyrroles, indoles, and thiazoles.

The phrase "sulfonylaminocarbonyl derivative" means a compound with one of the following substructure motifs:

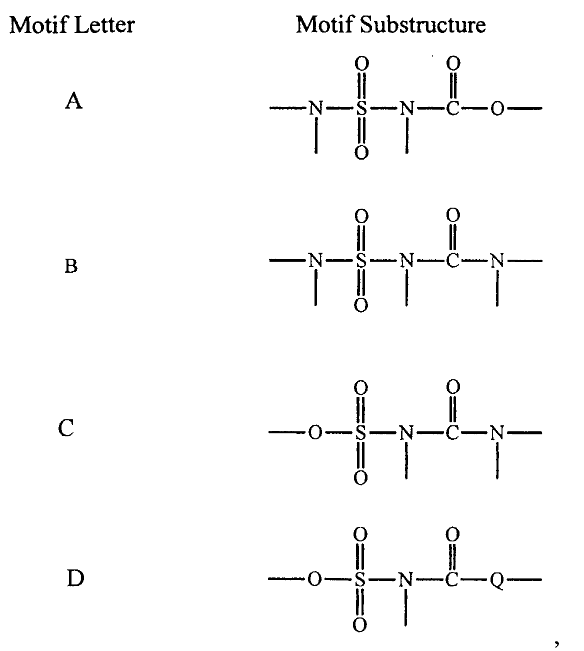

wherein Q is O or S

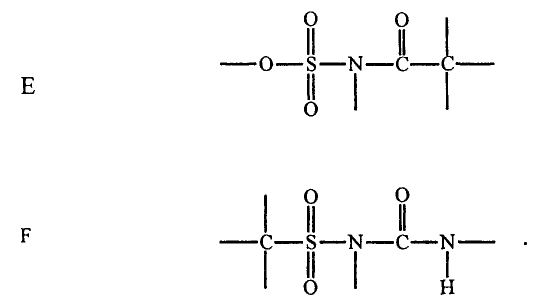

United States Patent No. 5,254,715 and its divisional 5,336,690 describe sulfonylaminocarbonyl derivatives with substructure motif A.

United States Patent No. 5,214,206 and its divisional 5,288,757 describe sulfonylaminocarbonyl derivatives with substructure motif B.

United States Patent No. 5,198,466 and its divisional 5,364,882 describe sulfonylaminocarbonyl derivatives with substructure motif C.

United States Patent No. 5,245,068 and its divisional 5,384,328 describe sulfonylaminocarbonyl derivatives with substructure motif D.

United States Patent No. 5,491,172 and its divisional 5,633,287, and United States Patent No. 6,093,744 describe sulfonylaminocarbonyl derivatives with substructure motif E.

United States Patent No. 5,254,589 and its continuation 5,981,595 describe sulfonylaminocarbonyl derivatives with substructure motif F.

The phrase "NF-κB inhibiting amount" means an amount of a sulfonylaminocarbonyl derivative, or a pharmaceutically acceptable salt thereof, sufficient to inhibit a NF-κB transcription factor in a particular animal or animal population. For example in a human or other mammal, an NF-κB inhibiting amount can be determined experimentally in a laboratory setting by measuring NF-κB activity in vitro according to the methods described below. Alternatively, an NF-κB inhibiting amount can be determined in vivo in an animal being treated by measuring disease-modifying affects in the conventional way. In a clinical setting, an NF-κB inhibiting amount may be determined according to the guidelines of the United States Food and Drug Administration, or equivalent foreign agency, for the particular NF-κB transcription factor being inhibited and patient being treated.

The phrase "autoimmune disease" means the diseases classified as "Highly probable" or "Probable" in TABLE 20-3. PUTATIVE AUTOIMMUNE DISORDERS of The Merck Manual of Diagnosis and Therapy, 16^{th} edition, Robert Berkow ed., Merck Research Laboratories, Rahway, New Jersey, 1992, page 340, which is hereby incorporated herein by reference. Diseases classified as highly probable include, to name a few, systemic lupus erythematosus, Grave's disease, myasthenia gravis, insulin resistance, and autoimmune hemolytic anemia. Diseases classified as probable include, to name a few, rheumatoid arthritis, scleroderma with anti-collagen antibodies (Abs), pernicious anemia, and some cases of diabetes mellitus.

Examples of a cardiovascular disease include, but are not limited to, atherosclerosis and acute coronary syndrome.

Examples of an acute coronary syndrome include, but are not limited to, myocardial infarction and unstable angina.

The term "patient" means a mammal, including a human, cat, dog, sheep, pig, horse, and cow.

The term "animal" means a mammal, including a human, cat, dog, sheep, cow, horse, pig, rat, mouse, guinea pig, rabbit, monkey, and transgenic variants thereof.

The term "comprising," which is synonymous with the terms "including," "containing," or "characterized by," is inclusive or open-ended and does not exclude additional, unrecited elements or method steps from the scope of the invention that follows.

The phrase "consisting of" is closed-ended and excludes any element, step, or ingredient not specified in the description of the invention that follows.

The phrase "consisting essentially of" limits the scope of the invention that follows to the specified elements or steps and those further elements or steps that do not materially affect the basic and novel characteristics of the invention.

Some of the compounds useful in the present invention may have chiral centers, in which case all stereoisomers thereof, both individual stereoisomers and mixtures of enantiomers or diastereomers, are included within the scope of the sulfonylaminocarbonyl derivatives useful in the present invention.

Some of the compounds useful in the present invention are capable of further forming nontoxic pharmaceutically acceptable acid-addition and/or base salts. All of these forms are within the scope of the compounds useful in the present invention.

For example, pharmaceutically acceptable acid addition salts of the compounds useful in the present invention include nontoxic salts derived from inorganic acids such as hydrochloric, nitric, phosphoric, sulfuric, hydrobromic, hydriodic, hydrofluoric, phosphorous, and the like, as well as the salts derived from organic acids, such as aliphatic mono- and dicarboxylic acids, phenyl-substituted alkanoic acids, hydroxy alkanoic acids, alkanedioic acids, aromatic acids, aliphatic and aromatic sulfonic acids, etc. Such salts thus include sulfate, pyrosulfate, bisulfate, sulfite, bisulfite, nitrate, phosphate, monohydrogenphosphate, dihyrogenphosphate, metaphosphate, pyrophosphate, chloride, bromide, iodide, acetate, trifluoroacetate, propionate, caprylate, isobutyrate, oxalate, malonate, succinates suberate, sebacate, fumarate, maleate, mandelate, benzoate, chlorobenzoate, methylbenzoate, dinitrobenzoate, phthalate, benzenesulfonate, toluenesulfonate, phenylacetate, citrate, lactate, maleate, tartrate, methanesulfonate, and the like. Also contemplated are salts of amino acids such as arginate and the like and gluconate, galacturonate (see, for example, Berge S.M., et al., "Pharmaceutical Salts," *Journal of Pharmaceutical Science,* 1977;66:1-19).

Acid addition salts of the compounds useful in the present invention that contain a basic functional group are prepared by contacting the free base form of the sulfonylaminocarbonyl derivative with a sufficient amount of the desired acid, which amount is usually 1 molar equivalent, to produce the salt in the conventional manner.

Pharmaceutically acceptable base salts of the compounds useful in the present invention are formed with metal cations such as, for example, alkali and alkaline earth metal cations, or amines such as, for example, organic amines. Examples of metals used as cations are sodium, potassium, magnesium, calcium, and the like. Examples of suitable amines are N,N-dibenzylethylenediamine, chloroprocaine, choline, diethanolamine, dicyclohexylamine, ethylenediamine, N-methylglucamine, and procaine (see, for example, Berge, supra., 1977).

Base salts of the compounds useful in the present invention that contain an acidic functional group are prepared by contacting the free acid form of the sulfonylaminocarbonyl derivative with a sufficient amount of the desired base, which amount is usually 1 molar equivalent, to produce the salt in the conventional manner.

Certain of the compounds useful in the present invention can exist in unsolvated forms as well as solvated forms, including hydrated forms. In general, the solvated forms, including hydrated forms, are equivalent to unsolvated forms and are encompassed within the scope of the compounds useful in the present invention.

Examples of sulfonylaminocarbonyl derivatives useful in the present invention are found below. The examples are for illustration purposes, and are not to be construed as limiting the scope of the invention in any respect.

### EXAMPLE 1

Carbamic acid, [[(diphenylmethyl)amino]sulfonyl]-, 2,6-bis(1-methylethyl)phenyl ester

### EXAMPLE 2

Carbamic acid, [[(diphenylmethyl)amino]sulfonyl]-, 2,6-bis(1,1-dimethylethyl)phenyl ester

### EXAMPLE 3

Carbamic acid, [[(diphenylmethyl)amino]sulfonyl]-, 2,6-bis(1,1-dimethylethyl)-4-methylphenyl ester

### EXAMPLE 4

Carbamic acid, [[[2,6-bis(1-methylethyl)phenyl]amino]sulfonyl]-, 2,6-bis(1-methylethyl)phenyl ester

### EXAMPLE 5

Carbamic acid, [[[2,6-bis(1-methylethyl)phenyl]amino]sulfonyl]-, 2,6-bis(1,1-dimethylethyl)phenyl ester

### EXAMPLE 6

Carbamic acid, [[[2,6-bis(1-methylethyl)phenyl]amino]sulfonyl]-, 2,6-bis(1,1-dimethylethyl)-4-methylphenyl ester

### EXAMPLE 7

Urea, N-[2,6-bis(1-methylethyl)phenyl]-N'-[[[2,6-bis(1-methylethyl)phenyl]-amino]sulfonyl]-

### EXAMPLE 8

Urea, N-[2,6-bis(1-methylethyl)phenyl]-N'-[[(diphenylmethyl)amino]-sulfonyl]-

### EXAMPLE 9

Urea, N-[2,6-bis(1-methylethyl)phenyl]-N'-[[(2,2-diphenylethyl)amino]-sulfonyl]-

### EXAMPLE 10

Carbamic acid, [[(2,2-diphenylethyl)amino]sulfonyl]-, 2,6-bis(1-methylethyl)-phenyl ester

### EXAMPLE 11

Carbamic acid, [[(2,2-diphenylethyl)amino]sulfonyl]-, 2,6-bis(1,1-dimethylethyl)-phenyl ester

### EXAMPLE 12

Carbamic acid, [[(2,2-diphenylethyl)amino]sulfonyl]-, 2,6-bis(1,1-dimethylethyl)-4-methylphenyl ester

### EXAMPLE 13

Carbamic acid, [(phenylamino)sulfonyl]-, 2,6-bis(1-methylethyl)phenyl ester

### EXAMPLE 14

Carbamic acid, [(phenylamino)sulfonyl]-, 2,6-bis(1,1-dimethylethyl)-4-hydroxyphenyl ester

### EXAMPLE 15

Carbamic acid, [(phenylamino)sulfonyl]-, 2,6-bis(1,1-dimethylethyl)-phenyl ester

### EXAMPLE 16

Carbamic acid, [(1H-benzimdazol-2-ylamino)sulfonyl]-, 2,6-bis(1-methylethyl)-phenyl ester

### EXAMPLE 17

N-[2,6-bis(1-methylethyl)phenyl]-N'-[[bis(phenylmethyl)amino]-sulfonyl]-urea

### EXAMPLE 18

N-[[[2,6-bis(1-methylethyl)phenyl]amino]sulfonyl]-N'-(diphenylmethyl)-urea

### EXAMPLE 19

N-[2,6-bis(1-methylethyl)phenyl]-N'-[(9H-fluoren-9-ylamino)-sulfonyl]-urea

### EXAMPLE 20

N-[2,6-bis(1-methylethyl)phenyl-N'-[[bis( 1-methylethyl)amino]-sulfonyl]-urea

### EXAMPLE 21

N-[2,6-bis(1-methylethyl)phenyl]-N'-[(dibutylamino)sulfonyl]-urea

### EXAMPLE 22

N-[2,6-bis( 1-methylethyl)phenyl]-N'-[[(1-methylethyl)-(phenylmethyl)amino]-sulfonyl]-urea

### EXAMPLE 23

N-[2,6-bis(1-methylethyl)phenyl]-N'-[(dioctylamino)sulfonyl]-urea

### EXAMPLE 24

Carbamic acid, [[bis(phenylmethyl)amino]sulfonyl]-, 2,6-bis(1,1-dimethylethyl)-phenyl ester

### EXAMPLE 25

Carbamic acid, [[bis(phenylmethyl)amino]sulfonyl]-, 2,6-bis(1-methylethyl)-phenyl ester

### EXAMPLE 26

Carbamic acid, [(diphenylamino)sulfonyl]-, 2,6-bis(1-methylethyl)phenyl ester

### EXAMPLE 27

Carbamic acid, [(dibutylamino)sulfonyl]-, 2,6-bis(1-methylethyl)phenyl ester

### EXAMPLE 28

Carbamic acid, [[2,6-bis(1-methylethyl)phenoxy]sulfonyl]-, 2,6-bis(1-methylethyl)phenyl ester

### EXAMPLE 29

N'-[[[2,6-bis(1-methylethyl)phenyl]amino]sulfonyl]-N,N-bis(phenylmethyl)-urea

### EXAMPLE 30

Carbamic acid, [(dibutylamino)sulfonyl]-, 2,6-bis(1,1-dimethylethyl)-4-methylphenyl ester

### EXAMPLE 31

Carbamic acid, [(dipentylamino)sulfonyl]-, 2,6-bis(1,1-dimethylethyl)-4-methylphenyl ester

### EXAMPLE 32

Carbamic acid, [[bis(1-methylethyl)amino]sulfonyl]-, 2,6-bis(1,1-dimethylethyl)-4-methylphenyl ester

### EXAMPLE 33

Carbamic acid, [(dihexylamino)sulfonyl]-, 2,6-bis(1,1-dimethylethyl)-4-methylphenyl ester

### EXAMPLE 34

Carbamic acid, [(hexylamino)sulfonyl]-, 2,6-bis(1,1-dimethylethyl)-4-methylphenyl ester

### EXAMPLE 35

N-[2,6-bis( 1-methylethyl)phenyl]-N'-[(4-phenyl- 1-piperidinyl)-sulfonyl]-urea

### EXAMPLE 36

N-[2,6-bis(1-methylethyl)phenyl]-N'-[(dihexylamino)sulfonyl]-urea

### EXAMPLE 37

N-[[bis[3-(dimethylamino)propyl]amino]sulfonyl]-N'-[2,6-bis( 1-methylethyl)phenyl] -urea

### EXAMPLE 38

Carbamic acid, [[methyl(2-phenylethyl)amino]sulfonyl]-, 2,6-bis(1,1-dimethylethyl)-4-methylphenyl ester

### EXAMPLE 39

N-[2,6-bis(1-methylethyl)phenyl]-N'-[(hexylamino)sulfonyl]-urea

### EXAMPLE 40

Carbamic acid, [[bis[3-(dimethylamino)propyl]amino]sulfonyl]-, 2,6-bis(1,1-dimethylethyl)-4-methylphenyl ester

### EXAMPLE 41

N-[2,6-bis(1-methylethyl)phenyl]-N'-[[bis[(tetrahydro-2-furanyl)methyl]amino]sulfonyl]-urea, (=+/)-

### EXAMPLE 42

Carbamic acid, [[methyl[2-(2-pyridinyl)ethyl]amino]sulfonyl]-, 2,6-bis(1,1-dimethylethyl)-4-methylphenyl ester, monohydrochloride

### EXAMPLE 43

Carbamic acid, [(methyloctylamino)sulfonyl]-, 2,6-bis(1,1-dimethylethyl)-4-methylphenyl ester

### EXAMPLE 44

Urea, N-[2,6-bis(1-methylethyl)phenyl]-N'-[(diethylamino)sulfonyl]-

### EXAMPLE 45

Urea, N-[2,6-bis(1-methylethyl)phenyl]-N'-[(methyloctylamino)sulfonyl]-

### EXAMPLE 46

Carbamic acid, [(dioctylamino)sulfonyl]-, 2,6-bis(1,1-dimethylethyl)-4-methylphenyl ester

### EXAMPLE 47

Carbamic acid, [[(2,2-diphenylethyl)amino]sulfonyl]-, 2,6-bis(1,1-dimethylethyl)-4-methoxyphenyl ester

### EXAMPLE 48

Carbamic acid, (phenoxysulfonyl)-, 2,6-bis(1,1-dimethylethyl)-4-methylphenyl ester

### EXAMPLE 49

Carbamic acid, [(hexyloxy)sulfonyl]-, 2,6-bis(1,1-dimethylethyl)-4-methylphenyl ester

### EXAMPLE 50

Carbamic acid, [[[2,6-bis(1-methylethyl)phenyl]amino]sulfonyl]-, 2,6-bis(1,1-dimethylethyl)-4-methoxyphenyl ester

### EXAMPLE 51

Carbamic acid, [(dodecyloxy)sulfonyl]-, 2,6-bis(1,1-dimethylethyl)-4-methylphenyl ester

### EXAMPLE 52

Carbamic acid, [(didecylamino)sulfonyl]-, 2,6-bis(1,1-dimethylethyl)-4-methylphenyl ester

### EXAMPLE 53

Carbamic acid, [[[2,6-bis(1-methylethyl)phenyl]amino]sulfonyl]-, dodecyl ester

### EXAMPLE 54

Carbamic acid, [[[2,6-bis(1-methylethyl)phenyl]amino]sulfonyl]-, methyl ester

### EXAMPLE 55

Carbamic acid, [[2,6-bis(1-methylethyl)phenoxy]sulfonyl]-, dodecyl ester

### EXAMPLE 56

Carbamic acid, [[2,6-bis(1-methylethyl)phenoxy]sulfonyl]-, methyl ester

### EXAMPLE 57

Carbamic acid, [(hexyloxy)sulfonyl]-, 2,6-bis(1-methylethyl)phenyl ester

### EXAMPLE 58

Carbamic acid, (4-morpholinylsulfonyl)-, 2,6-bis(1-methylethyl)phenyl ester

### EXAMPLE 59

Carbamic acid, (1-piperidinylsulfonyl)-, 2,6-bis(1-methylethyl)phenyl ester

### EXAMPLE 60

Sulfamic acid, [[[2,6-bis(1-methylethyl)phenyl]amino]carbonyl]-, octadecyl ester

### EXAMPLE 61

Carbamic acid, [(dodecyloxy)sulfonyl]-, dodecyl ester

### EXAMPLE 62

Carbamic acid, [[bis(1-methylethyl)amino]sulfonyl]-, 2,6-bis(1-methylethyl)-phenyl ester

### EXAMPLE 63

Urea, N-[2,6-bis(1-methylethyl)phenyl]-N'-[[cyclohexyl(1-methylethyl)amino]-sulfonyl]-

### EXAMPLE 64

Urea, N-[2,6-bis(1-methylethyl)phenyl]-N'-[(dipentylamino)sulfonyl]-

### EXAMPLE 65

Carbamic acid, [[(1-methylethyl)phenylmethyl)amino]sulfonyl]-, 2,6-bis(1-methylethyl)phenyl ester

### EXAMPLE 66

Carbamic acid, (1-pyrrolidinylsulfonyl)-, 2,6-bis(1-methylethyl)phenyl ester

### EXAMPLE 67

Carbamic acid, [(hexylamino)sulfonyl]-, 2,6-bis(1-methylethyl)phenyl ester

### EXAMPLE 68

Urea, N-[2,6-bis( 1-methylethyl)phenyl]-N'-[[bis(2-methylpropyl)amino]-sulfonyl]-

### EXAMPLE 69

Sulfamic acid, [[[2,6-bis(1-methylethyl)phenyl]amino]carbonyl]-, dodecyl ester

### EXAMPLE 70

Urea, N-[2,6-bis(1-methylethyl)phenyl]-N'-(1-pyrrolidinylsulfonyl)-

### EXAMPLE 71

Urea, N-[2,6-bis(1-methylethyl)phenyl]-N'-(1-piperidinylsulfonyl)-

### EXAMPLE 72

Carbamic acid, [(2,3-dihydro-1H-indol-1-yl)sulfonyl]-, 2,6-bis(1-methylethyl)-phenyl ester

### EXAMPLE 73

Urea, N-[2,6-bis(1-methylethyl)phenyl]-N'-[[ethyl(2-propenyl)amino]-sulfonyl]-

### EXAMPLE 74

Urea, N-[[bis(3-methylbutyl)amino]sulfonyl]-N'-[2,6-bis(1-methylethyl) phenyl]-

### EXAMPLE 75

Urea, N-[2,6-bis(1-methylethyl)phenyl]-N'-[(didecylamino)sulfonyl]-

### EXAMPLE 76

Urea, N-[2,6-bis(1-methylethyl)phenyl]-N'-[(didodecylamino)sulfonyl]-

### EXAMPLE 77

Urea, N-[2,6-bis(1-methylethyl)phenyl]-N'-[(dipropylamino)sulfonyl]-

### EXAMPLE 78

Urea, N-[2,6-bis(1-methylethyl)phenyl]-N'-[(dicyclohexylamino)-sulfonyl]-

### EXAMPLE 79

Carbamic acid, [[(2,4,6-trimethoxyphenyl)amino]sulfonyl]-, dodecyl ester

### EXAMPLE 80

Urea, N-[2,6-bis(1-methylethyl)phenyl]-N'-[(methyloctadecylamino)-sulfonyl]-

### EXAMPLE 81

Urea, N-[2,6-bis(1-methylethyl)phenyl]-N'-[(di-2-propenylamino)-sulfonyl]-

### EXAMPLE 82

Urea, N-[2,6-bis(1-methylethyl)phenyl]-N'-[[(1,1-dimethylethyl)( 1-methylethyl)-amino]sulfonyl]-

### EXAMPLE 83

Urea, N-[2,6-bis(1-methylethyl)phenyl]-N'-[[bis(1-methylpropyl)-amino]-sulfonyl]-

### EXAMPLE 84

Urea, N-[2,6-bis(1-methylethyl)phenyl)-N'-[(methyltetradecylamino)-sulfonyl]-

### EXAMPLE 85

Carbamic acid, [(dioctylamino)sulfonyl]-, 2,6-bis(1-methylethyl)phenyl ester

### EXAMPLE 86

Carbamic acid, [[cyclohexyl(1-methylethyl)amino]sulfonyl]-, 2,6-bis(1-methylethyl)phenyl ester

### EXAMPLE 87

Carbamic acid, [(methyloctylamino)sulfonyl]-, 2,6-bis(1-methylethyl)-phenyl ester

### EXAMPLE 88

Carbamic acid, [(dihexylamino)sulfonyl]-, 2,6-bis(1-methylethyl)phenyl ester

### EXAMPLE 89

Carbamic acid, [(dipentylamino)sulfonyl]-, 2,6-bis(1-methylethyl)phenyl ester

### EXAMPLE 90

Sulfamic acid, [[[2,6-bis(1-methylethyl)phenyl]amino]carbonyl]-, decyl ester

### EXAMPLE 91

Carbamic acid, [[2,6-bis(1-methylethyl)phenoxy]sulfonyl]-, 2,6-bis(1,1-dimethylethyl)phenyl ester

### EXAMPLE 92

Carbamic acid, [(dodecyloxy)sulfonyl]-, [1,1':3',1"-terphenyl]-2'-yl ester

### EXAMPLE 93

Carbamothioic acid, [(dodecyloxy)sulfonyl]-, S-[2,6-bis(1-methylethyl)-phenyl] ester

### EXAMPLE 94

Sulfamic acid, [[[2,6-bis(1-methylethyl)phenyl]amino]carbonyl]-, 1-methylheptyl ester, (=+/)-

### EXAMPLE 95

Sulfamic acid, [[[2,6-bis(1-methylethyl)phenyl]amino]carbonyl]-, 2,6-bis(1-methylethyl)phenyl ester

### EXAMPLE 96

Carbamic acid, [[(diphenylmethyl)amino]sulfonyl]methyl-, 2,6-bis(1,1-dimethylethyl)phenyl ester

### EXAMPLE 97

Carbamic acid, (phenoxysulfonyl)-, 2,6-bis(1-methylethyl)phenyl ester

### EXAMPLE 98

Carbamic acid, [(2,6-dimethylphenoxy)sulfonyl]-, 2,6-bis(1-methylethyl)-phenyl ester

### EXAMPLE 99

Urea, N'-[2,6-bis(1-methylethyl)phenyl]-N-[(dibutylamino)sulfonyl]-N-methyl-

### EXAMPLE 100

Carbamic acid, [[2,6-bis(1,1-dimethylethyl)phenoxy]sulfonyl]-, 2,6-bis(1,1-dimethylethyl)phenyl ester

### EXAMPLE 101

DL-Tryptophan, ]a/-methyl-N-[[[(tricyclo[3.3.1.1^{3,7}]dec-2-yloxy)-carbonyl]amino]sulfonyl]-, methyl ester

### EXAMPLE 102

Carbamic acid, [[2,6-bis(1,1-dimethylethyl)phenoxy]sulfonyl]-, 2,6-bis(1-methylethyl)phenyl ester

### EXAMPLE 103

Carbamic acid, [(2,6-difluorophenoxy)sulfonyl]-, 2,6-bis(1-methylethyl)-phenyl ester

### EXAMPLE 104

Urea, N-(2,2-dimethyl-4-phenyl-1,3-dioxan-5-yl)-N'-[[(tricyclo[3.3.1.1^{3,7}]dec-1-ylmethyl)amino]sulfonyl]-, (4S-#cis/)-

### EXAMPLE 105

Urea, N-(2,2-dimethyl-4-phenyl-1,3-dioxan-5-yl)-N'-[[(2,2-dimethyl-4-phenyl-1,3-dioxan-5-yl)amino]sulfonyl]-, stereoisomer

### EXAMPLE 106

Sulfamic acid, (1-oxodecyl)-, 2,6-bis(1-methylethyl)phenyl ester

### EXAMPLE 107

Carbamic acid, [(hexadecyloxy)sulfonyl]-, 2,6-bis(1-methylethyl)phenyl ester

### EXAMPLE 108

Carbamic acid, [[2,6-bis(1-methylethyl)phenoxy]sulfonyl]-, 2,6-dimethoxyphenyl ester

### EXAMPLE 109

Carbamic acid, [[2,6-bis(1-methylethyl)phenoxy]sulfonyl]-, 1-methylheptyl ester

### EXAMPLE 110

Carbamic acid, [[2,6-bis(1-methylethyl)phenoxy]sulfonyl]-, 2,6-bis(1-methylethyl)-4-nitrophenyl ester

### EXAMPLE 111

Carbamic acid, [[2,6-bis(1-methylethyl)phenoxy]sulfonyl]-, 1,2-ethanediyl ester

### EXAMPLE 112

Carbamic acid, [[2,6-bis(1-methylethyl)phenoxy]sulfonyl]-, 1,2,3-propanetriyl ester

### EXAMPLE 113

Carbamic acid, [[2,6-bis(1-methylethyl)phenoxy]sulfonyl]-, 4-bromo-2,6-bis(1-methylethyl)phenyl ester

### EXAMPLE 114

Carbamic acid, [[2,6-bis(1-methylethyl)phenoxy]sulfonyl]-, [1,1':3',1"-terphenyl]-2'-yl ester

### EXAMPLE 115

Carbamic acid, [[2,6-bis(1-methylethyl)phenoxy]sulfonyl]-, 2,6-bis(1,1-dimethylethyl)-4-methoxyphenyl ester

### EXAMPLE 116

Carbamic acid, [[2,6-bis(1-methylethyl)phenoxy]sulfonyl]-, 4-fluoro-2,3,5,6-tetrakis(1-methylethyl)phenyl ester

### EXAMPLE 117

Carbamic acid, [[2,6-bis(1-methylethyl)phenoxy]sulfonyl]-, 4-chloro-2,6-bis(1-methylethyl)phenyl ester

### EXAMPLE 118

Stigmasta-5,22-dien-3-ol, [[2,6-bis(1-methylethyl)phenoxy]sulfonyl]-carbamate, (3α)-

### EXAMPLE 119

Carbamic acid, [[2,6-bis(1-methylethyl)phenoxy]sulfonyl]-, 2,6-bis(1,1-dimethylethyl)-4-methylphenyl ester

### EXAMPLE 120

Sulfamic acid, [[2,4,6-tris(1-methylethyl)phenyl]acetyl]-, 2,6-bis(1-methylethyl)phenyl ester

### EXAMPLE 121

Stigmastan-3-ol, [[2,6-bis(1-methylethyl)phenoxy]sulfonyl]-carbamate, (3α)-

### EXAMPLE 122

Sulfamic acid, [[2,6-bis(1-methylethyl)phenyl]acetyl]-, 2,6-bis(1-methylethyl)-phenyl ester

### EXAMPLE 123

Carbamic acid, [[2,6-bis(1-methylethyl)phenoxy]sulfonyl]-, 4-methoxy-2,6-bis(1-methylethyl)phenyl ester

### EXAMPLE 124

Carbamic acid, [[2,6-bis(1-methylethyl)phenoxy]sulfonyl]-, 2,4,6-tris(1-methylethyl)phenyl ester

### EXAMPLE 125

Carbamic acid, [[2,4,6-tris(1-methylethyl)phenoxy]sulfonyl]-, 2,6-bis(1-methylethyl)phenyl ester

### EXAMPLE 126

Carbamic acid, [[2,4,6-tris(1-methylethyl)phenoxy]sulfonyl]-,2,4,6-tris( 1-methylethyl)phenyl ester

### EXAMPLE 127

Carbamic acid, [[2,4,6-bis(1-methylethyl)phenoxy]sulfonyl]-, 2,4,6-tris(1,1-dimethylethyl)phenyl ester

### EXAMPLE 128

Carbamic acid, [[2,6-bis(1-methylethyl)phenoxy]sulfonyl]-, 4-[[3,5-bis(1,1-dimethylethyl)-4-hydroxyphenyl]dithio]-2,6-bis(1,1-dimethylethyl)phenyl ester

### EXAMPLE 129

Carbamic acid, [[2,6-bis(1-methylethyl)phenoxy]sulfonyl]-, 2,4-bis(1-methylethyl)phenyl ester

### EXAMPLE 130

Carbamic acid, [[2,6-bis(1-methylethyl)phenoxy]sulfonyl]-, 4-[(dimethylamino)-methyl]-2,6-bis(1-methylethyl)phenyl ester

### EXAMPLE 131

Carbamic acid, [[2,6-bis(1-methylethyl)phenoxy]sulfonyl]-, tricyclo[3.3.1.1^{3,7}]-dec-2-yl ester

### EXAMPLE 132

Carbamic acid, [[2,6-bis(1-methylethyl )phenoxy]sulfonyl]-, 4-hydroxy-2,6-bis(1-methylethyl)phenyl ester

### EXAMPLE 133

Carbamic acid, [[2,6-bis(1-methylethyl)phenoxy]sulfonyl]-, cyclohexyl ester

### EXAMPLE 134

Carbamic acid, [[2,6-bis(1-methylethyl)phenoxy]sulfonyl]-, 3,3',5,5'-tetrakis(1-methylethyl)[1,1'-biphenyl]-4,4'-diyl ester

### EXAMPLE 135

Carbamic acid, [[4-hydroxy-2,6-bis(1-methylethyl)phenoxy]sulfonyl]-, 2,6-bis(1-methylethyl)phenyl ester

### EXAMPLE 136

Carbamic acid, [[2,6-bis(1-methylethyl)phenoxy]sulfonyl]-, tricyclo[3.3.1.1^{3,7}]-dec-1-yl ester

### EXAMPLE 137

Carbamic acid, [[2,6-bis(1-methylethyl)phenoxy]sulfonyl]-, 2-(1,1-dimethylethyl)-6-methylphenyl ester

### EXAMPLE 138

Carbamic acid, [[2,6-bis(1-methylethyl)phenoxy]sulfonyl]-, 5-methyl-2-(1-methylethyl)cyclohexyl ester

### EXAMPLE 139

Carbamothioic acid, [[2,6-bis(1-methylethyl)phenoxy]sulfonyl]-, *S*-[2,6-bis(1-methylethyl)phenyl] ester

### EXAMPLE 140

Carbamic acid, [[2,6-bis(1-methylethyl)phenoxy]sulfonyl]-, (2,6-diethylphenyl)methyl ester

### EXAMPLE 141

Carbamic acid, sulfonylbis-, bis[2,6-bis(1-methylethyl)phenyl] ester

### EXAMPLE 142

Carbamic acid, [[2,6-bis(1-methylethyl)phenoxy]sulfonyl]-, (2*S*,6*S*)-2,6-bis(1-methylethyl)cyclohexyl ester

### EXAMPLE 143

Carbamic acid, [[2,6-bis(1-methylethyl)phenoxy]sulfonyl]-, 4-(1,1-dimethylethyl)-2,6-bis(1-methylethyl)phenyl ester

### EXAMPLE 144

(2-Phenyl-cyclopropanecarbonyl)-sulfamic acid 2,6-diisopropyl-phenyl ester

### EXAMPLE 145

[(2,5-Dimethoxy-phenyl)-acetyl]-sulfamic acid 2,6-diisopropyl-phenyl ester

### EXAMPLE 146

[(2,4,6-Trimethyl-phenyl)-acetyl]-sulfamic acid 2,6-diisopropyl-phenyl ester

### EXAMPLE 147

[(2,4,6-Trimethoxy-phenyl)-acetyl]-sulfamic acid 2,6-diisopropyl-phenyl ester

### EXAMPLE 148

(Thiophen-2-yl-acetyl)-sulfamic acid 2,6-diisopropyl-phenyl ester

### EXAMPLE 149

(Thiophen-3-yl-acetyl)-sulfamic acid 2,6-diisopropyl-phenyl ester

### EXAMPLE 150

Carbamic acid, [[2,6-bis(1-methylethyl)phenoxy]sulfonyl]-, 4-fluorophenyl ester

### EXAMPLE 151

Carbamic acid, [[2,6-bis(1-methylethyl)phenoxy]sulfonyl]-, 2,4-difluorophenyl ester

### EXAMPLE 152

Carbamic acid, [[2,6-bis(1-methylethyl)phenoxy]sulfonyl]-, pentafluorophenyl ester

### EXAMPLE 153

Carbamic acid, [[2,6-bis(1-methylethyl)phenoxy]sulfonyl]-, 2,6-difluorophenyl ester

### EXAMPLE 154

Acetic acid 2-(2,6-diisopropyl-phenoxysulfonylamino)-2-oxo-1-(2,4,6-triisopropyl-phenyl)-ethyl ester

### EXAMPLE 155

Cyclohexylacetyl-sulfamic acid 2,6-diisopropyl-phenyl ester

### EXAMPLE 156

[(2-Methoxy-phenyl)-acetyl]-sulfamic acid 2,6-diisopropyl-phenyl ester

### EXAMPLE 157

(Oxo-phenyl-acetyl)-sulfamic acid 2,6-diisopropyl-phenyl ester

### EXAMPLE 158

[(2-Trifluoromethyl-phenyl)-acetyl]-sulfamic acid 2,6-diisopropyl-phenyl ester

### EXAMPLE 159

(2-Phenyl-propionyl)-sulfamic acid 2,6-diisopropyl-phenyl ester

### EXAMPLE 160

Diphenylacetyl-sulfamic acid 2,6-diisopropyl-phenyl ester

### EXAMPLE 161

(Cyclopentyl-phenyl-acetyl)-sulfamic acid 2,6-diisopropyl-phenyl ester

### EXAMPLE 162

[Hydroxy-(2,4,6-triisopropyl-phenyl)-acetyl]-sulfamic acid 2,6-diisopropyl-phenyl ester

### EXAMPLE 163

Triphenylacetyl-sulfamic acid 2,6-diisopropyl-phenyl ester

### EXAMPLE 164

Carbamic acid, [[2,6-bis(1-methylethyl)phenoxy]sulfonyl]-, (2*R*,6*S*)-2,6-bis(1-methylethyl)cyclohexyl ester

### EXAMPLE 165

(1,2,3,4-Tetrahydro-naphthalene-2-carbonyl)-sulfamic acid 2,6-diisopropyl-phenyl ester

### EXAMPLE 166

Carbamic acid, [[2,6-bis(1-methylethyl)phenoxy]sulfonyl]-, 2,3,5,6-tetramethylphenyl ester

### EXAMPLE 167

(3-Methyl-2-phenyl-pentanoyl)-sulfamic acid 2,6-diisopropyl-phenyl ester

### EXAMPLE 168

(1-Phenyl-cyclopentanecarbonyl)-sulfamic acid 2,6-diisopropyl-phenyl ester

### EXAMPLE 169

(2-Phenyl-butyryl)-sulfamic acid 2,6-diisopropyl-phenyl ester

### EXAMPLE 170

(Cyclohexyl-phenyl-acetyl)-sulfamic acid 2,6-diisopropyl-phenyl ester

### EXAMPLE 171

(2,2-Diphenyl-propionyl)-sulfamic acid 2,6-diisopropyl-phenyl ester

### EXAMPLE 172

[Bis-(4-chloro-phenyl)-acetyl]-sulfamic acid 2,6-diisopropyl-phenyl ester

### EXAMPLE 173

(9H-Xanthene-9-carbonyl)-sulfamic acid 2,6-diisopropyl-phenyl ester

### EXAMPLE 174

(9H-Fluorene-9-carbonyl)-sulfamic acid 2,6-diisopropyl-phenyl ester

### EXAMPLE 175

(Bromo-phenyl-acetyl)-sulfamic acid 2,6-diisopropyl-phenyl ester

### EXAMPLE 176

(3-Phenyl-propionyl)-sulfamic acid 2,6-diisopropyl-phenyl ester

### EXAMPLE 177

Sulfamic acid, [[[2,4,6-tris(1-methylethyl)phenyl]amino]carbonyl]-, 2,6-bis(1-methylethyl)phenyl ester

### EXAMPLE 178

Carbamic acid, [[2,6-bis(1-methylethyl)phenoxy]sulfonyl]-, 3-pyridinyl ester

### EXAMPLE 179

Carbamic acid, [[2,6-bis(1-methylethyl)phenoxy]sulfonyl]-, 2,6-dimethylphenyl ester

### EXAMPLE 180

[(2,4,6-Triisopropyl-phenyl)-acetyl]-sulfamic acid 4-hydroxy-2,6-diisopropylphenyl ester

### EXAMPLE 181

Methyl-[(2,4,6-triisopropyl-phenyl)-acetyl]-sulfamic acid 2,6-diisopropyl-phenyl ester

### EXAMPLE 182

[(2,4,6-Triisopropyl-phenyl)-acetyl]-sulfamic acid 2,6-diisopropyl-4-nitro-phenyl ester

### EXAMPLE 183

Carbamic acid, [[2,6-bis(1-methylethyl)phenoxy]sulfonyl]-, 4-acetyl-2,6-bis(1-methylethyl)phenyl ester

### EXAMPLE 184

Carbamic acid, [[2,6-bis(1-methylethyl)phenoxy]sulfonyl]-, 4-fluoro-2,6-bis(1-methylethyl)phenyl ester

### EXAMPLE 185

[(2,4,6-Triisopropyl-phenyl)-acetyl]-sulfamic acid 4-fluoro-2,6-diisopropylphenyl ester

### EXAMPLE 186

[(2,4,6-Triisopropyl-phenyl)-acetyl]-sulfamic acid 2,6-dimethoxy-phenyl ester

### EXAMPLE 187

[(2,4,6-Triisopropyl-phenyl)-acetyl]-sulfamic acid 4-amino-2,6-diisopropylphenyl ester

### EXAMPLE 188

6-(3,5-Diisopropyl-4-{[(2,4,6-triisopropyl-phenyl)-acetyl]sulfamoyloxy}-phenyl)-hexanoic acid ethyl ester

### EXAMPLE 189

[(2,4,6-Triisopropyl-phenyl)-acetyl]-sulfamic acid 2,4,6-trimethoxy-phenyl ester

### EXAMPLE 190

[(2,4,6-Triisopropyl-phenyl)-acetyl]-sulfamic acid 4-tert-butyl-2,6-diisopropylphenyl ester

### EXAMPLE 191

[(2,4,6-Triisopropyl-phenyl)-acetyl]-sulfamic acid 4-acetyl-2-isopropyl-phenyl ester

### EXAMPLE 192

[(2,4,6-Triisopropyl-phenyl)-acetyl]-sulfamic acid 2,6-diisopropyl-4-methoxyphenyl ester

### EXAMPLE 193

[(2,4,6-Triisopropyl-phenyl)-acetyl]-sulfamic acid 2,6-dichloro-phenyl ester

### EXAMPLE 194

3-[3-(3,5-Diisopropyl-4-{[(2,4,6-triisopropyl-phenyl)-acetyl]-sulfamoyloxy}-phenyl)-ureido]-propionic acid ethyl ester

### EXAMPLE 195

[5-tert-Butoxycarbonylamino-5-(3,5-diisopropyl-4-{[(2,4,6-triisopropyl-phenyl)-acetyl]sulfamoyloxy}-phenylcarbamoyl)-pentyl]-carbamic acid tert-butyl ester

### EXAMPLE 196

[(2,4,6-Triisopropyl-phenyl)-acetyl]-sulfamic acid 4-acetylamino-2,6-diisopropylphenyl ester

### EXAMPLE 197

[2-(2,4,6-Triisopropyl-phenyl)-acetyl]-sulfamic acid 4-((S)-2,6-diaminohexanoylamino)-2,6-diisopropyl-phenyl ester; compound with generic inorganic neutral component

### EXAMPLE 198

[(3,5-Diisopropyl-4-{[(2,4,6-triisopropyl-phenyl)-acetyl]sulfamoyloxy}-phenylcarbamoyl)-methyl]-carbamic acid tert-butyl ester

### EXAMPLE 199

[(2,4,6-Triisopropyl-phenyl)-acetyl]-sulfamic acid dodecyl ester

### EXAMPLE 200

[(2,4,6-Triisopropyl-phenyl)-acetyl]-sulfamic acid 4-(2-amino-acetylamino)-2,6-diisopropyl-phenyl ester

### EXAMPLE 201

[2-(2,4,6-Triisopropyl-phenyl)-acetyl]-sulfamic acid 4-((S)-2-amino-4-methylsulfanyl-butyrylamino)-2,6-diisopropyl-phenyl ester

### EXAMPLE 202

{[4-(1-Hydroxy-1-methyl-ethyl)-2,6-diisopropyl-phenyl]-acetyl}-sulfamic acid 2,6-diisopropyl-phenyl ester

### EXAMPLE 203

[(2,4,6-Triisopropyl-phenyl)-acetyl]-sulfamic acid 4-bromo-2,6-diisopropylphenyl ester

### EXAMPLE 204

[(2,4,6-Triisopropyl-phenyl)-acetyl]-sulfamic acid 4-[2-amino-3-( 1H-indol-3-yl)-propionylamino]-2,6-diisopropyl-phenyl ester

### EXAMPLE 205

[(2,4,6-Triisopropyl-phenyl)-acetyl]-sulfamic acid 4-(3-dimethylamino-propoxy)-2,6-diisopropyl-phenyl ester

### EXAMPLE 206

[2-(2,4,6-Triisopropyl-phenyl)-acetyl]-sulfamic acid 4-((R)-2-amino-propionylamino)-2,6-diisopropyl-phenyl ester; compound with trifluoro-acetic acid

### EXAMPLE 207

[(2,4,6-Triisopropyl-phenyl)-acetyl]-sulfamic acid 4-(2-amino-2-methylpropionylamino)-2,6-diisopropyl-phenyl ester

### EXAMPLE 208

[2-(2,4,6-Triisopropyl-phenyl)-acetyl]-sulfamic acid 4-(3-amino-propoxy)-2,6-diisopropyl-phenyl ester

### EXAMPLE 209

[(2,4,6-Triisopropyl-phenyl)-acetyl]-sulfamic acid 2,6-diisopropyl-4-thiocyanatophenyl ester

### EXAMPLE 210

[(2,4,6-Triisopropyl-phenyl)-acetyl]-sulfamic acid 2,6-diisopropyl-4-methylphenyl ester

### EXAMPLE 211

[1-(4-Dimethylamino-phenyl)-cyclopentanecarbonyl]-sulfamic acid 2,6-diisopropyl-phenyl ester

### EXAMPLE 212

[(2,4,6-Triisopropyl-phenyl)-acetyl]-sulfamic acid 4-cyano-2,6-diisopropylphenyl ester

### EXAMPLE 213

[1-(4-Nitro-phenyl)-cyclopentanecarbonyl]-sulfamic acid 2,6-diisopropyl-phenyl ester

### EXAMPLE 214

[1-(3,5-Diisopropyl-4-{[(2,4,6-triisopropyl-phenyl)-acetyl]sulfamoyloxy}-phenylcarbamoyl)-3-methylsulfanyl-propyl]-carbamic acid tert-butyl ester

### EXAMPLE 215

[(2,4,6-Triisopropyl-phenyl)-acetyl]-sulfamic acid 4-[3-(2,6-diisopropyl-phenyl)-ureido]-2,6-diisopropyl-phenyl ester

### EXAMPLE 216

[2-(2,4,6-Triisopropyl-phenyl)-acetyl]-sulfamic acid 4-((S)-2-amino-4-methylpentanoylamino)-2,6-diisopropyl-phenyl ester; compound with trifluoro-acetic acid

### EXAMPLE 217

Sulfamic acid, [[[[1-[4-(dimethylamino)phenyl]cyclopenty]methyl]-amino]carbonyl]- 2,6-bis(1-methylethyl)phenyl ester

### EXAMPLE 218

[(2,4,6-Triisopropyl-phenyl)-acetyl]-sulfamic acid 2,6-diisopropyl-4-(3-phenylureido)-phenyl ester

### EXAMPLE 219

[(2,4,6-Triisopropyl-phenyl)-acetyl]-sulfamic acid 4-(3-tert-butyl-ureido)-2,6-diisopropyl-phenyl ester

### EXAMPLE 220

[2-(2,4,6-Triisopropyl-phenyl)-acetyl]-sulfamic acid 4-(3-amino-propionylamino)-2,6-diisopropyl-phenyl ester; compound with trifluoro-acetic acid

### EXAMPLE 221

Carbamic acid, [[[2-(phenylmethyl)phenyl]amino]sulfonyl]-, 2,6-bis(1,1-dimethylethyl)phenyl ester

### EXAMPLE 222

(2,3-Dihydro-indole-1-carbonyl)-sulfamic acid 2,6-diisopropyl-phenyl ester

### EXAMPLE 223

Sulfamic acid, [[(triphenylmethyl)amino]carbonyl]-, 2,6-bis(1-methylethyl)phenyl ester

### EXAMPLE 224

[(2,4,6-Triisopropyl-phenyl)-acetyl]-sulfamic acid 4-(2-cyano-vinyl)-2,6-diisopropyl-phenyl ester

### EXAMPLE 225

[(2,4,6-Triisopropyl-phenyl)-acetyl]-sulfamic acid 2,6-diisopropyl-4-(thiophene-2-sulfonylamino)-phenyl ester

### EXAMPLE 226

[(2,4,6-Triisopropyl-phenyl)-acetyl]-sulfamic acid 4-(5-dimethylaminonaphthalene-1-sulfonylamino)-2,6-diisopropyl-phenyl ester

### EXAMPLE 227

[(2,4,6-Triisopropyl-phenyl)-acetyl]-sulfamic acid 2,6-diisopropyl-4-methanesulfonylamino-phenyl ester

### EXAMPLE 228

3,5-Diisopropyl-4-{[(2,4,6-triisopropyl-phenyl)-acetyl]sulfamoyloxy}-benzoic acid methyl ester

### EXAMPLE 229

[2-(2,4,6-Triisopropyl-phenyl)-acetyl]-sulfamic acid 4-(benzylamino-methyl)-2,6-diisopropyl-phenyl ester; compound with generic inorganic neutral component

### EXAMPLE 230

[2-(2,4,6-Triisopropyl-phenyl)-acetyl]-sulfamic acid 4-((S)-2-amino-3-hydroxypropionylamino)-2,6-diisopropyl-phenyl ester; compound with trifluoro-acetic acid

### EXAMPLE 231

[2-(2,4,6-Triisopropyl-phenyl)-acetyl]-sulfamic acid 4-((S)-2-amino-4-carbamoylbutyrylamino)-2,6-diisopropyl-phenyl ester; compound with trifluoro-acetic acid

### EXAMPLE 232

[2-(2,4,6-Triisopropyl-phenyl)-acetyl]-sulfamic acid 4-((S)-2-amino-3-methylbutyrylamino)-2,6-diisopropyl-phenyl ester; compound with trifluoro-acetic acid

### EXAMPLE 233

[(2,4,6-Triisopropyl-phenyl)-acetyl]-sulfamic acid 4-hydroxymethyl-2,6-diisopropyl-phenyl ester

### EXAMPLE 234

[(2,4,6-Triisopropyl-phenyl)-acetyl]-sulfamic acid 4-carbamoyl-2,6-diisopropylphenyl ester

### EXAMPLE 235

[(2,4,6-Triisopropyl-phenyl)-acetyl]-sulfamic acid 4-[3-(3,5-dichloro-phenyl)-thioureido]-2,6-diisopropyl-phenyl ester

### EXAMPLE 236

((E)-2-Methyl-3-phenyl-acryloyl)-sulfamic acid 2,6-diisopropyl-phenyl ester

### EXAMPLE 237

(2-Oxo-2H-chromene-3-carbonyl)-sulfamic acid 2,6-diisopropyl-phenyl ester

### EXAMPLE 238

N-[2,6-bis(1-methylethyl)phenyl]-N'-(hexadecylsulfonyl)-urea

### EXAMPLE 239

N-[2,6-bis(1-methylethyl)phenyl]-N'-[(6-ethoxy-2-benzothiazolyl)sulfonyl]-urea

### EXAMPLE 240

N-[2,6-bis(1-methylethyl)phenyl]-N'-(tetradecylsulfonyl)-urea

### EXAMPLE 241

N'-[2,6-bis(1-methylethyl)phenyl]-N-methyl-N-(tetradecylsulfonyl)-urea

### EXAMPLE 242

N-[2,6-bis(1-methylethyl)phenyl]-N'-(tridecylsulfonyl)urea

### EXAMPLE 243

N-[2,6-bis(1-methylethyl)phenyl]-N'-(1-phenyl-1-nonylsulfonyl)urea

### EXAMPLE 244

N-[2,6-bis(1-methylethyl)phenyl]-N'-(2-decylsulfonyl)urea

### EXAMPLE 245

N-[2,6-bis(1-methylethyl)phenyl]-N'-(1-phenyl-1-tetradecylsulfonyl)urea

### EXAMPLE 246

N-[2,6-bis(1-methylethyl)phenyl]-N'-(2-octadecylsulfonyl)urea

### EXAMPLE 247

N-[2,4,6-trimethoxyphenyl]-N'-(2-octadecylsulfonyl)urea

### EXAMPLE 248

N-[2,6-bis(1-methylethyl)phenyl]-N'-(2-methyl-2-pentadecylsulfonyl)urea

### EXAMPLE 249

N-[2,4,6-trimethoxyphenyl]-N'-(2-methyl-2-pentadecylsulfonyl)urea

### EXAMPLE 250

Carbamic acid, [[[2,6-bis(1-methylethyl)phenyl]amino]sulfonyl]-, dodecyl ester

Sulfonylaminocarbonyl derivatives useful in the present invention may be identified using the methods described below.

### BIOLOGICAL METHODS

Introduction: Described below is an in vitro, cell-based, high throughput screening assay that reliably identifies inhibitors of NF-κB mediated transcription. While the assay described below utilized inhibitors which were sulfonylaminocarbonyl derivatives useful in the present invention, the assay may be used to screen for any inhibitor of NF-κB mediated transcription.

The assay takes advantage of AURORA (Aurora Bioscience Corporation, La Jolla, California) fluorescence technology. Endothelial cell vein-304 (ECV-304) cells, an endothelial-like immortalized cell type, may be stably transfected with a plasmid vector containing the cDNA for the enzyme β-lactamase (under the control of a basal promoter, Stratagene pNFκB-luc vector) and 5 copies of an human immunodeficiency virus-1 (HIV-1) NF-κB binding site. ECV-304 cells are described by Takahashi K. et al. in Spontaneous Transformation and Immobilization of Human Endothelial Cells, *In Vitro Cell. Dev*. *Biol.* 1990;25:265-274. Activation of NF-κB in ECV-304 by cytokines such as TNF-α or interleuken- 1 beta (IL-1β) results in the production of β-lactamase, which cleaves a green fluorescent substrate (excitation/emission wavelengths 395 nm/530 nm) to yield a blue fluorescent product (excitation/emission wavelengths 395 nm/460 nm). Visually, the uncleaved green fluorescent substrate is sequestered intracellularly and emits green fluorescence, while the cleaved product emits blue fluorescence. Fluorescence is quantitated spectrophotometrically, and the spectral intensity of blue fluorescence versus green fluorescence can be used to calculate the degree of activation of NF-κB.

The assay was performed as outlined here and described in detail below. ECV-304 cells permanently transfected with the NF-κB driven (β-lactamase gene (ECV-304 NF-κB.βlaZ) were plated in clear-bottom, black 96-well plates (1.25 × 10⁴ cells/well) in media-199 (M-199) media containing 2% fetal bovine serum (FBS). Approximately 18 hours later the cells were stimulated with either 10 ng/mL of TNF-α or 100 pg/mL of IL-1β, and incubated for 6 hours at 37°C in the presence or absence of a compound useful in the present invention. The AURORA fluorescent disclosing reagent was then added. After one additional hour, the plates were read in a fluorometer at the blue 395 nm/460 nm (excitation/emission) and green 395 nm/530 nm wavelengths. Then the blue/green emission ratio was calculated. Percent inhibition was calculated by comparing fluorescence in the presence of a sulfonylaminocarbonyl derivative useful in the present invention with fluorescence in the absence of said sulfonylaminocarbonyl derivative under conditions of maximum stimulation with TNF-α or IL-1β. An IC₅₀ for said sulfonylaminocarbonyl derivative was determined from a dose-response curve. This assay was designed to be optionally run in either high or low throughput screening modes.

Materials: ECV-304 cells were obtained from American Type Culture Collection (ATCC). Cytokines TNF-α and IL-1β were obtained from R&D Systems. Lipofectamine, M199, and penicillin/streptomycin 1000 U (P/S) were from GIBCO-BRL. Reagents A, B, and C are proprietary reagents from Aurora Technologies. The FBS is from Summit Technologies. The CCF2 dye was from Aurora Biosciences, La Jolla, California.

### Methods:

### (i) ECV-304 NF-κB.βlaZ Cell Line:

Five copies of the HIV-1 NF-κB binding site (5'-TGGGGACTTTCCGC-3') along with a TATA box were inserted into the EcorRI/BamHI sites of plasmid pMCRBlaZ to create plasmid p5xKBBlaZ, which procedure is illustrated in Scheme 1 below.

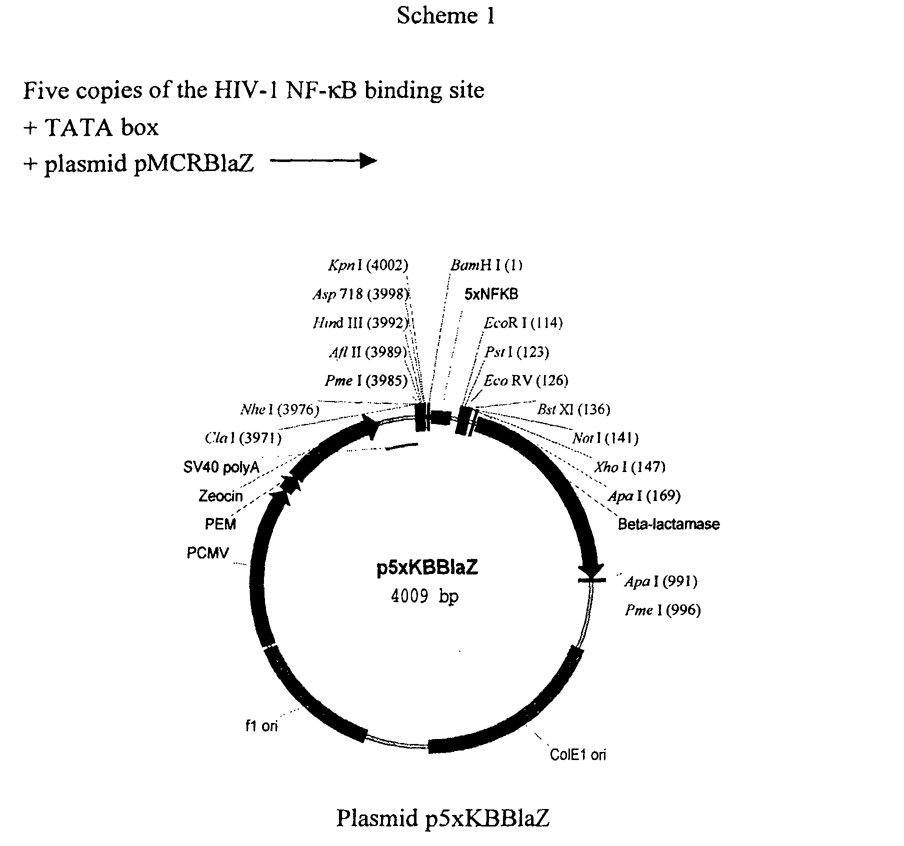

The parental plasmid, pMCRBlaZ, contains a multiple cloning site upstream of the β-lactamase gene as well as a Zeocin antibiotic resistance gene.

Plasmid p5xKBBlaZ was transfected into ECV-304 cells using lipofectamine and the well-known standard conditions found on the package insert. Cells were selected for antibiotic resistance with Zeocin for approximately 2 weeks. After the stable population had been expanded to a significant number of cells, it was stimulated with IL-1β and stained with AURORA CCF2 dye, a membrane permeant, intracellularly-trapped, fluorescent substrate. Cells that fluoresced blue (indicating a positive response of the NF-κB/β-lactamase reporter to stimulation by IL-1β) in the stimulated/stained population were then sorted by flow cytometry into a pool. The pool was expanded and stained with CCF2, and then the cells that fluoresced green from this unstimulated population (indicating a low background of the NF-κB/β-lactamase reporter construct) were cloned by flow cytometry after adding 1 cell per well in 96-well plates. Clones were allowed to expand. Each cloned cell line was then examined for fold stimulation after stimulation with IL-1β using a CCF2 reporter assay, and these stimulated cells were compared to unstimulated cells. The cloned cell line with the maximal fold induction (plate 1, row E, cell 8 or 1E8) was chosen for further assay development. This clone consistently showed the highest fluorescence signal to noise ratio upon stimulation with TNF-α or IL-1β.

### (ii) Assay Development:

The assay described herein was initially developed for high throughput screening purposes. As such, several factors were taken into consideration in the process. The assay was developed to minimize handling (i.e., no media changes, washes, etc.). Conditions were established to optimize the incubation period to 4 to 6 hours for logistical reasons. The assay was also optimized to its ability to tolerate the presence or absence of serum and the concentration of dimethylsulfoxide (DMSO) that could be used without interference of the fluorescence generated from the activation of NF-κB. Cytokine stability and optimal cell density were also optimized. Also, no difference in NF-κB stimulation in ECV-304 cells separated in lineage by 20 passages (Passages 5 and 25) was found.

### (iii) Regeants:

Cells: ECV-304 NF-κB β-Lactamase clone 1E8
Complete Media: M199, 10%FBS (nonheat inactivated), 1% P/S, 200 µg/mL Zeocin;
Assay Media: M199, 2% FBS, no antibiotics
Cell Culture: 0.5 × 106 cells/T 150 culture flask, 30 mL complete media, fed every other day; harvested Day 7, approximate yield 1.1 (107/flask)
Assay Seeding Density: 1.78 × 105/mL, 70 µL/well―96-well plate, (12,500 cells/well)
Quality Controls: The proteosome inhibitors MG132, MG262, and clastolactacystine β-lactone may be used, as these agents irreversibly inhibit the proteasomal breakdown of IkB (McCormicack, et al., *Journal of Biological Chemistry,* 1997;272(42):26103-26109 and Craui et al., *Journal of Biological Chemistry,* 1997;272(20):13437-13445.)
Inhibitors: Prepared stock solutions of aminosulfonyl derivatives at 10 mM concentration in DMSO;
   Diluted stock solutions in 96-well diluting plate as follows:
   a) Added 20 µL of stock solution into 180 µL of M199 = A,
   b) Added 20 µL A into 180 µL M199 = B,
   c) Added 10 µL of B into appropriate well in assay plate (the final concentration of drug is 10 µM), and
   (d) Diluted further for IC₅₀ determinations.
Activation Cytokines TNF-α and IL-1β:
   Stock solution of TNF-α: R&D Systems 210-TA, 10 µg was diluted into 2 mL of phosphate buffered saline (PBS) containing 0.1% bovine serum albumin (BSA) (concentration of TNF-α = 5 µg/mL);
   Diluted TNF-α stock solution 1:100: 90 µL of TNF-α stock solution was diluted into 9.0 mL of M199 media (concentration of TNF-α = 50 ng/mL); and
   Added 20 µL of the solution of TNF-α at 50 ng/mL to all wells except reagent control wells, and cell control wells (final concentration of TNF-α in well = 10 ng/mL).
   Stock solution of IL-1β: R&D Systems 203-LB, 5 µg was diluted into 1 mL of PBS containing 0.1% BSA (concentration of IL-1β= 5 µg/mL);
   Diluted the IL-1β stock solution 1:1000: 20 µL of IL-1β stock solution was diluted into 20 mL of M199 (concentration of IL-1β = 5 ng/mL);
   Diluted the 5 ng/mL solution of IL-1β 1:10: 0.5 mL of the 5 ng/mL solution IL-1β at a concentration of 5 ng/mL was diluted into 5.0 mL of M199 (concentration of IL-1β = 500 pg/mL); and
   Added 20 µL the solution of the 500 pg/mL IL-1β at to all wells except reagent control wells and cell control wells (final concentration IL-1β in well = 100 pg/mL).

### (iv) A High Throughput Screening Assay Procedure:

**Table 1.**

| Plate Map | | | | | | | | | | | | |
|---|---|---|---|---|---|---|---|---|---|---|---|---|
| | **1** | **2** | **3** | **4** | **5** | **6** | **7** | **8** | **9** | **10** | **11** | **12** |
| **A** | USC | T | O | O | O | O | O | O | O | O | O | B |
| **B** | USC | T | O | O | O | O | O | O | O | O | O | B |
| **C** | USC | T | O | O | O | O | O | O | O | O | O | B |
| **D** | USC | T | O | O | O | O | O | O | O | O | O | B |
| **E** | USC | T | O | O | O | O | O | O | O | O | O | B |
| **F** | USC | T | O | O | O | O | O | O | O | O | O | B |
| **G** | USC | MG | O | O | O | O | O | O | O | O | O | B |
| **H** | USC | MG | O | O | O | O | O | O | O | O | O | B |
| Column 1 = **USC**, Unstimulated cell control wells. | | | | | | | | | | | | |
| Column 2 A-F = **T**, Maximal activation control wells. | | | | | | | | | | | | |
| Column 2 G-H = **MG**, MG132 quality control. | | | | | | | | | | | | |
| Column 12 = **B**, Reagent background wells. | | | | | | | | | | | | |
| Columns 3-11 = **O**, Sulfonylaminocarbonyl derivatives in triplicate at a concentration of 10 µM (for screening purposes) or at varying concentrations for dose response studies (IC₅₀). | | | | | | | | | | | | |

Plates for the HTS are configured in an alternative format.

PM Day 1: Seeded cells at 0.178 × 10⁶/mL, 70 µL/well, in assay media AM Day 2:
a) DMSO Control: Added 10 µL of a 1% DMSO solution in M199 to the following wells (final concentration of DMSO in well = 0.1%): Added to B wells (reagent control: assay media, no cytokine, no cells), USC wells (unstimulated cell control: cells, assay media, no activation cytokine), and T wells (maximal activity: cells, assay media, cytokine);
b) Inhibitor: Added 10 µL of a sulfonylaminocarbonyl derivative at 10× desired final concentration to the following wells: Added to all O wells (unknowns: cells, assay media, cytokine) and MG wells (quality control: cells, assay media, cytokine). No inhibitor should be added to USC wells, B wells, or T wells.
c) Activation Cytokine: Added 20 µL of the 50 ng/mL solution of TNF-α (to give a 10 ng/mL final concentration of TNF-α) or 20 µL of the 500 pg/mL solution of IL-1β (to give a 100 pg/mL final concentration of IL-1β) to all T wells, O wells, and MG wells. No cytokine was added to USC wells or B wells.
d) Incubation after stimulation of cells with an activation cytokine, with or without a sulfonylaminocarbonyl derivative: 6 hours, 37°C, 5% CO₂ atmosphere
e) Preparation of AURORA CCF2 Fluorescence Disclosing Substrate Solutions:

Prepared four separate solutions of 2 mL each for each plate using the amounts recited in Table 2 below according to the following procedure.

Added Reagent A to 50-mL tube first, then added Reagent B. Mixed. Then added Reagent C. Mixed.

**Table 2.**

| Substrate Formulation Instructions | | |
|---|---|---|
| (Reagent A + Reagent B) | | + Reagent C |
| 6 µL | 60 µL | 1 mL |
| 24 µL | 240 µL | 4 mL |
| 48 µL | 480 µL | 8 mL |
| 60 µL | 600 µL | 10 mL |
| 2 mL/plate, 20 µL/well; make 2 mL extra | | |

Incubation after addition of the AURORA CCF2 fluorescence disclosing solution: 1 hour at room temperature in the dark

Spectrophotometric analysis: CYTOFLUOR (Millipore Corporation, Bedford, Massachusetts) 2 instruments using the following wavelengths in nanometers. Excite 395 Emit 460 (Blue) and Excite 395 Emit 530 (Green)

### (v) Calculation of Data

- BKG Blue (BB) =: Average reagent background blue emission.
- BKG Green (BG): = Average reagent background green emission.
- Corrected Blue (CB): = Subtract BB from all blue readings on plate.
- Corrected Green (CG) =: Subtract BG from all green readings on plate.
- Blue/Green Ratio (BGR) =: Divide CB by CG (CB/CG).
- BGRF =: Divide BGR by BGR of USC wells.
- Maximum Activity =: Average of BGRF of stimulated cells (TNF-α max or IL-1β max).
- % Inhibition of T Maximum Activity =: (100 - (average BGRF of unknown (cells + inhibitor)/TNF-α max or IL-β max) × 100).

Representative sulfonylaminocarbonyl derivatives useful in the present invention were tested at a concentration of 10 µM for the ability to inhibit NF-κB mediated transcription using the method described above, and the results are shown below in Table 3 in the column labeled "Percent inhibition at 10 µM."

**Table 3.**

| Inhibition of NF-κB Mediated Transcription (Page 1 of 9) | |
|---|---|
| Example No. | Percent Inhibition at 10 µM |
| 1 | 24.83 |
| 2 | 37.20 |
| 3 | 79.34 |
| 4 | 35.32 |
| 5 | 56.40 |
| 6 | 64.92 |
| 7 | 37.04 |
| 8 | <10^{a} |
| 9 | 33.15 |
| 10 | 39.74 |
| 11 | 50.11 |
| 12 | 57.02 |
| 13 | <10 |
| 14 | 13.84 |
| 15 | 12.56 |
| 16 | <10 |
| 17 | 43.76 |
| 18 | 35.92 |
| 19 | 20.24 |
| 20 | <10 |
| 21 | 29.41 |
| 22 | 20.17 |
| 23 | 38.67 |
| 24 | 30.97 |
| 25 | 33.24 |
| 26 | 32.80 |
| 27 | 26.15 |
| 28 | 30.20 |
| 29 | 38.49 |
| 30 | 52.05 |
| 31 | 74.02 |
| 32 | 20.33 |
| 33 | 58.63 |
| 34 | 24.30 |
| 35 | <10 |
| 36 | 51.23 |
| 37 | <10 |
| 38 | 31.49 |
| 39 | <10 |
| 40 | <10 |
| 41 | <10 |
| 42 | <10 |
| 43 | 70.23 |
| 44 | <10 |
| 45 | 53.50 |
| 46 | 34.79 |
| 47 | 41.89 |
| 48 | 13.77 |
| 49 | 22.93 |
| 50 | 39.06 |
| 51 | 47.24 |
| 52 | <10 |
| 53 | 61.31 |
| 54 | <10 |
| 55 | 55.07 |
| 56 | <10 |
| 57 | 20.38 |
| 58 | <10 |
| 59 | <10 |
| 60 | <10 |
| 61 | 27.45 |
| 62 | <10 |
| 63 | 23.03 |
| 64 | 55.44 |
| 65 | 16.44 |
| 66 | <10 |
| 67 | 13.82 |
| 68 | 34.32 |
| 69 | 75.49 |
| 70 | <10 |
| 71 | <10 |
| 72 | 14.11 |
| 73 | <10 |
| 74 | 37.58 |
| 75 | <10 |
| 76 | <10 |
| 77 | <10 |
| 78 | 40.90 |
| 79 | <10 |
| 80 | <10 |
| 81 | <10 |
| 82 | <10 |
| 83 | 10.58 |
| 84 | 44.82 |
| 85 | 44.99 |
| 86 | 16.00 |
| 87 | <10 |
| 88 | <10 |
| 89 | 31.94 |
| 90 | 13.55 |
| 91 | 39.69 |
| 92 | <10 |
| 93 | 14.51 |
| 94 | <10 |
| 95 | 33.07 |
| 96 | 10.70 |
| 97 | <10 |
| 98 | <10 |
| 99 | <10 |
| 100 | 50.03 |
| 101 | <10 |
| 102 | 20.58 |
| 103 | <10 |
| 104 | <10 |
| 105 | <10 |
| 106 | 72.28 |
| 107 | <10 |
| 108 | <10 |
| 109 | 51.35 |
| 110 | 15.48 |
| 111 | <10 |
| 112 | <10 |
| 113 | <10 |
| 114 | <10 |
| 115 | 38.99 |
| 116 | <10 |
| 117 | <10 |
| 118 | <10 |
| 119 | 56.56 |
| 120 | 54.98 |
| 121 | <10 |
| 122 | 65.26 |
| 123 | <10 |
| 124 | 11.32 |
| 125 | 11.04 |
| 126 | 10.02 |
| 127 | 74.01 |
| 128 | <10 |
| 129 | <10 |
| 130 | <10 |
| 131 | 33.39 |
| 132 | <10 |
| 133 | <10 |
| 134 | 18.39 |
| 135 | <10 |
| 136 | <10 |
| 137 | <10 |
| 138 | 66.16 |
| 139 | 65.52 |
| 140 | 38.70 |
| 141 | <10 |
| 142 | 53.87 |
| 143 | 65.81 |
| 144 | 16.19 |
| 145 | <10 |
| 146 | 28.31 |
| 147 | <10 |
| 148 | <10 |
| 149 | <10 |
| 150 | <10 |
| 151 | <10 |
| 152 | <10 |
| 153 | <10 |
| 154 | 77.73 |
| 155 | <10 |
| 156 | <10 |
| 157 | <10 |
| 158 | 25.90 |
| 159 | 10.14 |
| 160 | 34.22 |
| 161 | 51.45 |
| 162 | 75.35 |
| 163 | 62.44 |
| 164 | 55.18 |
| 165 | 28.67 |
| 166 | <10 |
| 167 | 40.01 |
| 168 | 39.14 |
| 169 | 21.31 |
| 170 | 78.57 |
| 171 | 62.23 |
| 172 | 86.90 |
| 173 | 36.94 |
| 174 | 32.24 |
| 175 | <10 |
| 176 | <10 |
| 177 | 63.60 |
| 178 | <10 |
| 179 | <10 |
| 180 | 91.96 |
| 181 | <10 |
| 182 | 69.81 |
| 183 | <10 |
| 184 | 21.20 |
| 185 | 73.94 |
| 186 | 18.83 |
| 187 | 57.76 |
| 188 | 50.76 |
| 189 | 15.06 |
| 190 | 27.56 |
| 191 | 55.66 |
| 192 | 63.94 |
| 193 | 38.28 |
| 194 | 89.85 |
| 195 | 91.77 |
| 196 | 73.11 |
| 197 | <10 |
| 198 | 75.78 |
| 199 | 50.73 |
| 200 | 72.66 |
| 201 | 87.14 |
| 202 | 25.39 |
| 203 | 52.22 |
| 204 | 70.04 |
| 205 | 11.72 |
| 206 | 47.89 |
| 207 | 60.34 |
| 208 | 22.39 |
| 209 | 56.80 |
| 210 | 52.45 |
| 211 | 24.65 |
| 212 | 60.24 |
| 213 | 19.73 |
| 214 | 71.74 |
| 215 | 74.72 |
| 216 | 65.43 |
| 217 | 37.08 |
| 218 | <10 |
| 219 | 15.15 |
| 220 | 65.49 |
| 221 | 44.70 |
| 222 | <10 |
| 223 | 68.21 |
| 224 | <10 |
| 225 | 26.39 |
| 226 | 46.34 |
| 227 | 77.28 |
| 228 | 76.08 |
| 229 | 75.79 |
| 230 | 75.15 |
| 231 | 78.10 |
| 232 | 76.92 |
| 233 | 76.94 |
| 234 | 76.65 |
| 235 | 145.92 |
| 236 | 79.42 |
| 237 | 57.13 |
| 238 | 12.0 |
| 239 | <10 |
| 240 | 10.3 |
| 241 | <10 |
| 242 | <10 |
| 243 | <10 |
| 244 | <10 |
| 245 | 16.1 |
| 246 | <10 |
| 247 | 14.8 |
| 248 | 11.4 |
| 249 | <10 |
| 250 | <10 |

| | |
|---|---|
| ^{a} "<10" means percent inhibition was >0 µm, but < 10 µm. | |

As shown by cell-based assay data, the sulfonylaminocarbonyl derivatives in Table 3 are inhibitors of NF-κB mediated transcription that are able to cross cell membranes and reach a target in a NF-κB signal pathway. Accordingly, the sulfonylaminocarbonyl derivatives are useful in the present invention for treating a disease or a disorder responsive to the inhibition of NF-κB such as, for example, rheumatoid arthritis and osteoarthritis, autoimmune diseases, psoriasis, asthma, cardiovascular diseases such as, for example, atherosclerosis, acute coronary syndromes including myocardial infarction and unstable angina, and congestive heart failure, Alzheimer's disease, multiple sclerosis, cancer, type II diabetes, metabolic syndrome X, and inflammatory bowel disease.

In carrying out the methods for treating a disease or a disorder responsive to the inhibition of NF-κB of the present invention, sulfonylaminocarbonyl derivatives useful in the present invention may be administered in a number of pharmaceutically acceptable oral and parenteral forms. Thus, the sulfonylaminocarbonyl derivatives can be administered by injection, that is, intravenously, intramuscularly, intracutaneously, subcutaneously, intraduodenally, or intraperitoneally. Also, the sulfonylaminocarbonyl derivatives can be administered by inhalation, for example, intranasally. Additionally, the sulfonylaminocarbonyl derivatives can be administered transdermally. The following dosage forms may comprise as the active component a compound of Formula I or Formula II, or another sulfonylaminocarbonyl derivative useful in the present invention, or a pharmaceutically acceptable salt thereof.

For preparing pharmaceutical compositions from the compounds of the present invention, pharmaceutically acceptable carriers can be either solid or liquid. Solid form preparations include powders, tablets, pills, capsules, cachets, suppositories, and dispersible granules. A solid carrier can be one or more substances, which may also act as diluents, flavoring agents, binders, preservatives, tablet disintegrating agents, or an encapsulating material.

In powders, the carrier is a finely divided solid, which is in a mixture with the finely divided active component.

In tablets, the active component is mixed with the carrier having the necessary binding properties in suitable proportions and compacted in the shape and size desired.

The powders and tablets preferably contain from five or ten to about seventy percent of the active compound. Suitable carriers are magnesium carbonate, magnesium stearate, talc, sugar, lactose, pectin, dextrin, starch, gelatin, tragacanth, methylcellulose, sodium carboxymethylcellulose, a low melting wax, cocoa butter, and the like. The term "preparation" is intended to include the formulation of the active compound with encapsulating material as a carrier providing a capsule in which the active component with or without other carriers, is surrounded by a carrier, which is thus in association with it. Similarly, cachets and lozenges are included. Tablets, powders, capsules, pills, cachets, and lozenges can be used as solid dosage forms suitable for oral administration.

For preparing suppositories, a low melting wax, such as a mixture of fatty acid glycerides or cocoa butter, is first melted, and the active component is dispersed homogeneously therein, as by stirring. The molten homogenous mixture is then poured into convenient sized molds, allowed to cool, and thereby to solidify.

Liquid form preparations include solutions, suspensions, and emulsions, for example, water or water propylene glycol solutions. For parenteral injection, liquid preparations can be formulated in solution in aqueous polyethylene glycol solution.

Aqueous solutions suitable for oral use can be prepared by dissolving the active component in water and adding suitable colorants, flavors, stabilizing and thickening agents as desired.

Aqueous suspensions suitable for oral use can be made by dispersing the finely divided active component in water with viscous material, such as natural or, synthetic gums, resins, methylcellulose, sodium carboxymethylcellulose, and other well-known suspending agents.

Also included are solid form preparations, which are intended to be converted, shortly before use, to liquid form preparations for oral administration. Such liquid forms include solutions, suspensions, and emulsions. These preparations may contain, in addition to the active component, colorants, flavors, stabilizers, buffers, artificial and natural sweeteners, dispersants, thickeners, solubilizing agents, and the like.

The pharmaceutical preparation is preferably in unit dosage form. In such form the preparation is divided into unit doses containing appropriate quantities of the active component. The unit dosage form can be a packaged preparation, the package containing discrete quantities of preparation, such as packeted tablets, capsules, and powders in vials or ampoules. Also, the unit dosage form can be a capsule, tablet, cachet, or lozenge itself, or it can be the appropriate number of any of these in packaged form.

The quantity of active component in a unit dose preparation may be varied or adjusted from 0.1 mg to 100 mg preferably 0.5 mg to 100 mg according to the particular application and the potency of the active component. The composition can, if desired, also contain other compatible therapeutic agents.

In therapeutic use as antagonists or as agents for the treatment of diseases, the compounds utilized in the pharmaceutical method of this invention are administered at the initial dosage of about 0.01 mg to about 100 mg/kg daily. A daily dose range of about 0.01-mg to about 10 mg/kg is preferred. The dosages, however, may be varied depending upon the requirements of the patient, the severity of the condition being treated, the compound being employed. Determination of the proper dosage for a particular situation is within the skill of the art. Generally, treatment is initiated with smaller dosages, which are less than the optimum dose of the compound. Thereafter, the dosage is increased by small increments until the optimum effect under the circumstances is reached. For convenience, the total daily dosage may be divided and administered in portions during the day, if desired.

Examples of pharmaceutical preparations of the sulfonylaminocarbonyl derivatives useful in the present invention are described below. Such preparations can be administered to a patient, including a human, from 1 to 6 times a day for treatment of diseases and disorders responsive to inhibition of NF-κB mediated transcription.

### FORMULATION EXAMPLE 1

| Tablet Formulation: | |
|---|---|
| Ingredient | Amount (mg) |
| Compound of Example 199 | 25 |
| Lactose | 50 |
| Cornstarch (for mix) | 10 |
| Cornstarch (paste) | 10 |
| Magnesium stearate (1%) | 5 |
| Total | 100 |

The compound of Example 199, lactose, and cornstarch (for mix) are blended to uniformity. The cornstarch (for paste) is suspended in 200 mL of water and heated with stirring to form a paste. The paste is used to granulate the mixed powders. The wet granules are passed through a No. 8 hand screen and dried at 80°C. The dry granules are lubricated with the 1% magnesium stearate and pressed into a tablet.

### FORMULATION EXAMPLE 2

### Coated Tablets:

The tablets of formulation Example 1 are coated in a customary manner with a coating of sucrose, potato starch, talc, tragacanth, and colorant.

### FORMULATION EXAMPLE 3

### Infection vials:

The pH of a solution of 500 g of the compound of Example 3 and 5 g of disodium hydrogen phosphate is adjusted to pH 6.5 in 3 L of double-distilled water using 2 M hydrochloric acid. The solution is sterile filtered, and the filtrate is filled into injection vials, lyophilized under sterile conditions, and aseptically sealed. Each injection vial contains 25 mg of the compound of Example 3.

### FORMULATION EXAMPLE 4

### Suppositories:

A mixture of 25 g of the compound of Example 31, 100 g of soya lecithin, and 1400 g of cocoa butter is fused, poured into molds, and allowed to cool. Each suppository contains 25 mg of the compound of Example 31.

### FORMULATION EXAMPLE 5

### Solution:

A solution is prepared from 1 g of the compound of Example 55, 9.38 g of NaH₂PO₄·2H₂O, 28.48 g of Na₂HPO₄·12H₂O, and 0.1 g benzalkonium chloride in 940 mL of double-distilled water. The pH of the solution is adjusted to pH 6.8 using 2 M hydrochloric acid. The solution is diluted to 1.0 L with double-distilled water, and sterilized by irradiation. A 25 mL volume of the solution contains 25 mg of the compound of Example 55.

### FORMULATION EXAMPLE 6

### Ointment:

500 mg of the compound of Example 119 is mixed with 99.5 g of petroleum jelly under aseptic conditions. A 5 g portion of the ointment contains 25 mg of the compound of Example 119.

### FORMULATION EXAMPLE 7

### Capsules:

2 kg of the compound of Example 180 are filled into hard gelatin capsules in a customary manner such that each capsule contains 25 mg of the invention compound.

### FORMULATION EXAMPLE 8

### Ampoules:

A solution of 2.5 kg of the compound of Example 231 is dissolved in 60 L of double-distilled water. The solution is sterile filtered, and the filtrate is filled into ampoules. The ampoules are lyophilized under sterile conditions and aseptically sealed. Each ampoule contains 25 mg of the compound of

### Example 231.

Having described the methods of the present invention above, embodiments of the present invention are hereupon claimed.

## Claims

1. The use of a sulfonylaminocarbonyl derivative, or a pharmaceutically acceptable salt thereof for the preparation of a pharmaceutical composition for the treatment of a disease or a disorder responsive to inhibition of nuclear factor-κB (NF-κB) transcription factors.

2. The use according to Claim 1, wherein the sulfonylaminocarbonyl derivative is a compound of Formula I 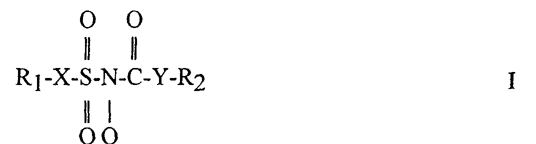 or a pharmaceutically acceptable salt thereof, wherein:
X and Y are selected from oxygen, sulfur and (CR'R")ₙ, wherein n is an integer of from I to 4 and R' and R" are each independently hydrogen, alkyl, alkoxy, halogen, hydroxy, acyloxy, cycloalkyl, phenyl optionally substituted or R' and R" together form a spirocycloalkyl or a carbonyl;
with the proviso at least one of X and Y is -(CR'R")ₙ- and with the further proviso when X and Y are both (CR'R")ₙ and R' and R" are hydrogen and n is one, R₁ and R₂ are aryl;
R is hydrogen, a straight or branched alkyl of from I to 8 carbon atoms or benzyl;
R₁ and R₂ are each independently selected from:
(a) phenyl or phenoxy each of which is unsubstituted or is substituted with from 1 to 5 substituents selected from:
phenyl,
an alkyl group having from I to 6 carbon atoms and which is straight or branched,
an alkoxy group having from 1 to 6 carbon atoms and which is straight or branched;
phenoxy,
hydroxy,
fluorine,
chlorine,
bromine,
nitro,
trifluoromethyl,
- COOH,
- COOalkyl wherein alkyl has from 1 to 4 carbon atoms and is straight or branched, and
- (CH₂)ₚNR₃R₄, wherein p is zero or one, and each of R₃ and R₄ is selected from hydrogen or a straight or branched alkyl group having 1 to 4 carbon atoms;
(b) 1- or 2-naphthyl unsubstituted or substituted with from 1 to 3 substituents selected from:
phenyl,
an alkyl group having from 1 to 6 carbon atoms and which is straight or branched,
an alkoxy group having from 1 to 6 carbon atoms and which is straight or branched;
hydroxy,
phenoxy,
fluorine,
chlorine,
bromine,
nitro,
trifluoromethyl,
- COOH,
- COOalkyl wherein alkyl has from 1 to 4 carbon atoms and is straight or branched, and
- (CH₂)ₚNR₃R₄, wherein p, R₃ and R₄ have the meanings defined above;
(c) arylalkyl;
(d) a straight or branched alkyl chain having from 1 to 20 carbon atoms and which is saturated or contains from 1 to 3 double bonds; and
(e) adamantyl or a cycloalkyl group wherein the cycloalkyl moiety has from 3 to 6 carbon atoms;
with the provisos:
(i) where X is (CH₂)ₙ, Y is oxygen, and R₁ is a substituted phenyl, then R₂ is a substituted phenyl;
(ii) where Y is oxygen, X is (CH₂)ₙ, R₂ is phenyl or naphthyl, then R₁ is not a straight or branched alkyl chain; and
(iii) the following compounds are excluded: 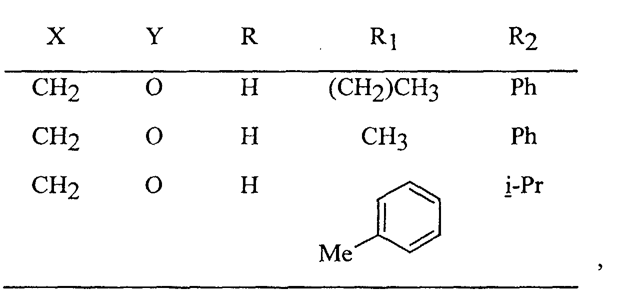 with the further proviso that compounds selected from the group consisting of:
Sulfamic acid [1-oxo-3-[2,4,6-tris(1-methylethyl)phenyl]propyl]-2,6-bis(1-methylethyl)phenyl ester,
Sulfamic acid [fluoro[2,4,6-tris(1-methylethyl)phenyl]acetyl]-2,6-bis(1-methylethyl)phenyl ester, and
Sulfamic acid [[2,4,6-tris(1-methylethyl)phenyl]acetyl]-2,6-bis(phenyl)phenyl ester are excluded.

3. The use according to Claim 2, wherein the sulfonylaminocarbonyl derivative is a compound of Formula I, or a pharmaceutically acceptable salt thereof, selected from:
(1,2,3,4-Tetrahydro-naphthalene-2-carbonyl)-sulfamic acid 2,6-diisopropyl-phenyl ester;
[Bis-(4-chloro-phenyl)-acetyl]-sulfamic acid 2,6-diisopropyl-phenyl ester;
(Bromo-phenyl-acetyl)-sulfamic acid 2,6-diisopropyl-phenyl ester; [(2,4,6-Triisopropyl-phenyl)-acetyl]-sulfamic acid 4-hydroxy-2,6-diisopropyl-phenyl ester;
Methyl-[(2,4,6-triisopropyl-phenyl)-acetyl]-sulfamic acid 2,6-diisopropyl-phenyl ester;
[(2,4,6-Triisopropyl-phenyl)-acetyl]-sulfamic acid 2,6-diisopropyl-4-nitro-phenyl ester;
[(2,4,6-Triisopropyl-phenyl)-acetyl]-sulfamic acid 4-fluoro-2,6-diisopropyl-phenyl ester;
[(2,4,6-Triisopropyl-phenyl)-acetyl]-sulfamic acid 2,6-dimethoxyphenyl ester;
[(2,4,6-Triisopropyl-phenyl)-acetyl]-sulfamic acid 4-amino-2,6-diisopropyl-phenyl ester;
[(2,4,6-Triisopropyl-phenyl)-acetyl]-sulfamic acid 2,4,6-trimethoxy-phenyl ester;
[(2,4,6-Triisopropyl-phenyl)-acetyl]-sulfamic acid 4-tert-butyl-2,6-diisopropyl-phenyl ester;
[(2,4,6-Triisopropyl-phenyl)-acetyl]-sulfamic acid 4-acetyl-2-isopropyl-phenyl ester;
[(2,4,6-Triisopropyl-phenyl)-acetyl]-sulfamic acid 2,6-diisopropyl-4-methoxy-phenyl ester;
[(2,4,6-Triisopropyl-phenyl)-acetyl]-sulfamic acid 2,6-dichloro-phenyl ester;
[(2,4,6-Triisopropyl-phenyl)-acetyl]-sulfamic acid dodecyl ester;
[(2,4,6-Triisopropyl-phenyl)-acetyl]-sulfamic acid 4-bromo-2,6-diisopropyl-phenyl ester;
[(2,4,6-Triisopropyl-phenyl)-acetyl]-sulfamic acid 2,6-diisopropyl-4-methyl-phenyl ester;
[1-(4-Dimethylamino-phenyl)-cyclopentanecarbonyl]-sulfamic acid 2,6-diisopropyl-phenyl ester;
[1-(4-Nitro-phenyl)-cyclopentanecarbonyl]-sulfamic acid 2,6-diisopropyl-phenyl ester;
3,5-Diisopropyl-4-{[(2,4,6-triisopropyl-phenyl)-acetyl]sulfamoyloxy}-benzoic acid methyl ester;
Sulfamic acid (phenylacetyl)-2,6-bis(1-methylethyl)phenyl ester;
Sulfamic acid[[2,4,6-tris(1-methylethyl)phenyl]acetyl-2,6-bis(1-methylethyl)phenyl ester;
Sulfamic acid[[2,6-bis(1-methylethyl)phenyl]acetyl]-2,6-bis(1-methylethyl)phenyl ester;
Sulfamic acid [[2,4,6-tris(1-methylethyl)phenyl]acetyl-2,4,6-tris( 1-methylethyl)phenyl ester;
Sulfamic acid[[2,6-bis(1-methylethyl)phenyl]acetyl]-2,4,6-tris( 1-methylethyl)phenyl ester;
Sulfamic acid[adamantaneacetyl]-2,6-bis[1-methylethyl)phenyl ester;
Sulfamic acid[[2,6-bis(1-methylethyl)phenyl]acetyl]-2,6-bis(1-methylethyl)phenyl ester-sodium salt;
Sulfamic acid[[2,4,6-tris(1-methylethyl)phenyl]acetyl]-2,6-bis(1-methylethyl)phenyl ester-sodium salt;
Sulfamic acid (decanoyl)-2,6-bis-(1-methylethyl)phenyl ester;
Sulfamic acid (dodecanoyl)-2,6-bis-(1-methylethyl)phenyl ester; 2,6-Bis(1-methylethyl)-N-[[[2,4,6-tris(1-methylethyl)phenyl]-methyl]sulfonyl]benzeneacetamide;
2,6-Bis(1-methylethyl)-N-[[[2,4,6-tris( 1-methylethyl)phenyl]-methyl]sulfonyl]benzeneacetamide-sodium salt;
2,6-Bis(1-methylethyl)phenyl[[[2,4,6-tris(1-methylethyl)phenyl]-methyl]sulfonyl]carbamate;
2,6-Bis(I -methylethyl)phenyl[[[2,4,6-tris(1-methylethyl)phenyl]-methyl]sulfonyljcarbamate-sodium salt;
Sulfamic acid (1-oxo-3,3-diphenylpropyl)-2,6-bis(1-methylethyl)phenyl ester;
Sulfamic acid [2,6-dichlorophenyl(acetyl)]-2,6-bis(1-methylethyl)phenyl ester;
Sulfamic acid [2,6-dichlorophenyl(acetyl)]-2,6-bis(1-methylethyl)phenyl ester sodium salt;
Sulfamic acid trans-[(2-phenylcyclopropyl)carbonyl]-2,6-bis(1-methylethyl)phenyl ester;
Sulfamic acid [2,5-dimethoxyphenyl(acetyl)]-2,6-bis(1-methylethyl)phenyl ester;
Sulfamic acid [2,4,6-trimethoxyphenyl(acetyl)]-2,6-bis(1-methylethyl)phenyl ester;
Sulfamic acid [2,4,6-trimethylphenyl(acetyl)]-2,6-bis(1-methylethyl)phenyl ester;
Sulfamic acid [2-thiophenyl(acetyl)]-2,6-bis(1-methylethyl)phenyl ester;
Sulfamic acid [3-thiophenyl(acetyl)]-2,6-bis(1-methylethyl)phenyl ester;
Sulfamic acid [2-methoxyphenyl(acetyl)]-2,6-bis(1-methylethyl)phenyl ester;
Sulfamic acid (oxophenylacetyl)-2,6-bis(1-methylethyl)phenyl ester;
Sulfamic acid [2-trifluoromethylphenyl(acetyl)]-2,6-bis(1-methylethyl)phenyl ester;
Sulfamic acid (1-oxo-2-phenylpropyl)-2,6-bis(1-methylethyl)phenyl ester;
Sulfamic acid (cyclopentylphenylacetyl)-2,6-bis(1-methylethyl)phenyl ester;
Sulfamic acid (cyclohexylacetyl)-2,6-bis(1-methylethyl)phenyl ester;
Sulfamic acid (diphenylacetyl)-2,6-bis(1-methylethyl)phenyl ester;
Sulfamic acid (triphenylacetyl)-2,6-bis(1-methylethyl)phenyl ester;
Sulfamic acid [(1-phenylcyclopentyl)carbonyl]-2,6-bis(1-methylethyl)phenyl ester;
Sulfamic acid (3-methyl-1-oxo-2-phenylpentyl)-2,6-bis(1-methylethyl)phenyl ester;
Sulfamic acid (1-oxo-2-phenylbutyl)-2,6-bis(1-methylethyl)phenyl ester;
Sulfamic acid (cyclohexylphenyl-acetyl)-2,6-bis(1-methylethyl)phenyl ester;
Sulfamic acid (1-oxo-2,2-diphenylpropyl)-2,6-bis(1-methylethyl)phenyl ester;
Sulfamic acid [(9H-fluoren-9-yl)carbonyl]-2,6-bis(1-methylethyl)phenyl ester;
Sulfamic acid (1-oxo-3-phenylpropyl)-2,6-bis(1-methylethyl)phenyl ester;
Sulfamic acid [1-oxo-3-[2,4,6-tris(1-methylethyl)phenyl]-2-propenyl]-2,6-bis(1-methylethyl)phenyl ester;
Sulfamic acid [(acetyloxy)[2,4,6-tris(1-methylethyl)phenyl]acetyl]-2,6-bis(1-methylethyl)phenyl ester;
Sulfamic acid [hydroxy[2,4,6-tris(1-methylethyl)phenyl]acetyl]-2,6-bis(1-methylethyl)phenyl ester;
Sulfamic acid (3-methyl-1-oxo-2-phenylpentyl)-2,6-bis(1-methylethyl)phenyl ester sodium salt;
Sulfamic acid [[2,4,6-tris(1-methylethyl)phenoxy]acetyl]-2,6-bis(1-methylethyl)phenyl ester; and
Sulfamic acid [[2,6-bis(1-methylethyl)phenoxy]acetyl]-2,6-bis(1-methylethyl)phenyl ester.

4. The use according to Claim 2, wherein the sulfonylaminocarbonyl derivative is sulfamic acid [[2,4,6-tris(1-methylethyl)phenyl]acetyl-2,6-bis(1-methylethyl)phenyl ester, or a pharmaceutically acceptable salt thereof.

5. The use according to Claim 1, wherein the sulfonylaminocarbonyl derivative is a compound of Formula II 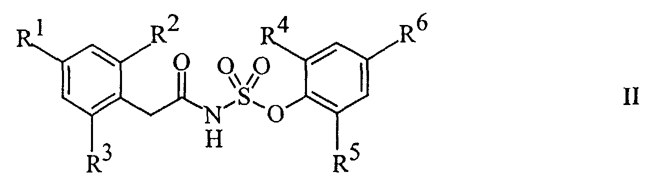 or a pharmaceutically acceptable salt thereof wherein:
R¹ is hydrogen, alkyl, or alkoxy;
R² to R⁵ are alkyl, alkoxy, or unsubstituted or substituted phenyl; and
R⁶ is -CN,
- (CH₂)₀₋₁-NR⁷R⁸,
- O-(CH₂)₁₋₁₀-Z, wherein Z is -NR⁹R¹⁰, OR¹, or CO₂R¹,
- OC(=O)R¹¹,
- SR¹¹,
- SCN,
- S(CH₂)₁₋₁₀Z,
- S(O)₁₋₂R¹², wherein R¹² is hydroxy, alkoxy, alkyl, (CH₂)₁₋₁₀Z or NR⁷R⁸,
- C(=O)XR¹¹, or
- CH₂-R¹³, wherein R¹³ is (CH₂)₀₋₅-Y-(CH₂)₀₋₅Z, or alkyl of from 1 to 20 carbons with from 1-3 double bonds, which alkyl is optionally substituted by one or more moieties selected from -CN, NO₂, halogen, OR¹, NR⁹R¹⁰, and CO₂R¹;
wherein R⁷ and R⁸ are each independently selected from:
- hydrogen, at least one of R⁷ and R⁸ is other than hydrogen,
- (CH₂)₁₋₁₀Z, wherein Z is as defined above and R⁹ and R¹⁰ are each independently selected from hydrogen, alkyl, and unsubstituted or substituted phenyl, or
R⁹ and R¹⁰ are taken together with the nitrogen to which they are attached to form a ring selected from: 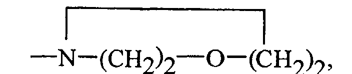 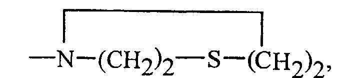 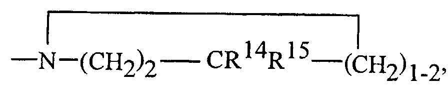 and 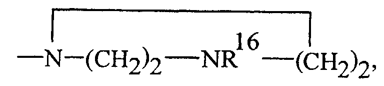 wherein R¹⁴, R¹⁵, and R¹⁶ are each independently selected from hydrogen, alkyl, and unsubstituted or substituted phenyl;
- C(=Q)XR¹¹, wherein X is a bond or NH wherein Q is O or S, R¹¹ is hydrogen, alkyl, unsubstituted or substituted phenyl,
- (CH₂)₀₋₅-Y-(CH₂)₀₋₅Z, wherein Z is as defined above and Y is phenyl or a bond;
- C(=O)-CR¹⁷R¹⁸Z;
- C(=O)NHCR¹⁷R¹⁸Z, wherein R¹⁷ and R¹⁸ are each independently hydrogen, alkyl, phenyl, substituted phenyl, or the side chain of a naturally occurring amino acid;
- S(O)₁₋₂R¹⁹, wherein R¹⁹ is alkyl, unsubstituted or substituted phenyl, naphthyl, or a heteroaromatic ring, or NR⁹R¹⁰ or
R⁷ and R⁸ are taken together with the nitrogen to which they are attached to form a ring selected from: 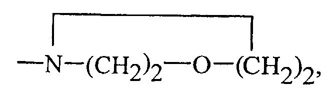 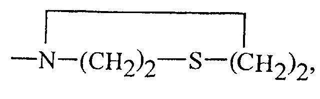 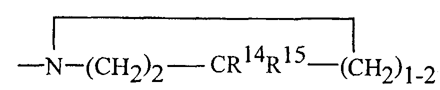 and 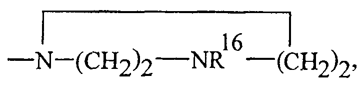
wherein R¹⁴, R¹⁵, and R¹⁶ are as above, with the proviso that compounds selected from the group consisting of:
[(2,4,6-Triisopropyl-phenyl)-acetyl]-sulfamic acid 4-formyl-2,6-diisopropyl-phenyl ester;
[(2,4,6-Triisopropyl-phenyl)-acetyl]-sulfamic acid 4-(2-cyano-vinyl)-2,6-diisopropyl-phenyl ester;
[(2,4,6-Triisopropyl-phenyl)-acetyl]-sulfamic acid 2,6-diisopropyl-4-(4-methyl-piperazin-1-ylmethyl)-phenyl ester, dihydrochloride;
[(2,4,6-Triisopropyl-phenyl)-acetyl]-sulfamic acid 4-[bis-(2-hydroxyethyl)-amino]-2,6-diisopropyl-phenyl ester;
[(2,4,6-Triisopropyl-phenyl)-acetyl]-sulfamic acid 2,6-diisopropyl-4-(3-phenyl-thioureido]-phenyl ester; and
[(2,4,6-Triisopropyl-phenyl)-acetyl]-sulfamic acid 2,6-diisopropyl-4-sulfamoyl-phenyl ester
are excluded.

6. The use according to Claim 5, wherein the sulfonylaminocarbonyl derivative is a compound of Formula II, or a pharmaceutically acceptable salt thereof, selected from:
6-(3,5-Diisopropyl-4-{[(2,4,6-triisopropyl-phenyl)-acetyl]sulfamoyloxy}-phenyl)-hexanoic acid ethyl ester;
3-[3-(3,5-Diisopropyl-4-{[(2,4,6-triisopropyl-phenyl)-acetyl]sulfamoyloxy}-phenyl)-ureido]-propionic acid ethyl ester;
{[4-(1-Hydroxy-1-methyl-ethyl)-2,6-diisopropyl-phenyl]-acetyl}-sulfamic acid 2,6-diisopropyl-phenyl ester;
[2-(2,4,6-Triisopropyl-phenyl)-acetyl]-sulfamic acid 4-((S)-2-amino-4-methyl-pentanoylamino)-2,6-diisopropyl-phenyl ester; compound with trifluoro-acetic acid;
[(2,4,6-Triisopropyl-phenyl)-acetyl]-sulfamic acid 4-(3-tert-butyl-ureido)-2,6-diisopropyl-phenyl ester;
[2-(2,4,6-Triisopropyl-phenyl)-acetyl]-sulfamic acid 4-(3-amino-propionylamino)-2,6-diisopropyl-phenyl ester; compound with trifluoro-acetic acid;
[(2,4,6-Triisopropyl-phenyl)-acetyl]-sulfamic acid 4-(2-cyanovinyl)-2,6-diisopropyl-phenyl ester;
[2-(2,4,6-Triisopropyl-phenyl)-acetyl]-sulfamic acid 4-((S)-2-amino-3-hydroxy-propionylamino)-2,6-diisopropyl-phenyl ester; compound with trifluoro-acetic acid;
[2-(2,4,6-Triisopropyl-phenyl)-acetyl]-sulfamic acid 4-((S)-2-amino-4-carbamoyl-butyrylamino)-2,6-diisopropyl-phenyl ester; compound with trifluoro-acetic acid;
[2-(2,4,6-Triisopropyl-phenyl)-acetyl]-sulfamic acid 4-((S)-2-amino-3-methyl-butyrylamino)-2,6-diisopropyl-phenyl ester; compound with trifluoro-acetic acid;
[(2,4,6-Triisopropyl-phenyl)-acetyl]-sulfamic acid 4-[3-(3,5-dichloro-phenyl)-thioureido]-2,6-diisopropyl-phenyl ester;
(S)-[5-tert-Butoxycarbonylamino-5-(3,5-diisopropyl-4-{[(2,4,6-triisopropyl-phenyl)-acetyl]sulfamoyloxy}-phenylcarbamoyl)-pentyl]-carbamic acid tert-butyl ester;
(S)-[(2,4,6-Triisopropyl-phenyl)-acetyl]-sulfamic acid 4-(2,6-diamino-hexanoylamino)-2,6-diisopropyl-phenyl ester dihydrochloride;
[(2,4,6-Triisopropyl-phenyl)-acetyl]-sulfamic acid 4-(2-t-butoxycarbonylamino-acetylamino)-2,6-diisopropyl-phenyl ester;
[(2,4,6-Triisopropyl-phenyl)-acetyl]-sulfamic acid 4-(2-amino-acetylamino)-2,6-diisopropyl-phenyl ester;
[(2,4,6-Triisopropyl-phenyl)-acetyl]-sulfamic acid 4-(2-t-butoxycarbonylamino-4-methylsulfanyl-butyrylamino)-2,6-diisopropyl-phenyl ester;
[(2,4,6-Triisopropyl-phenyl)-acetyl]-sulfamic acid 4-(2-amino-4-methylsulfanyl-butyrylamino)-2,6-diisopropyl-phenyl ester trifluoroacetate;
3-[3-(3,5-Diisopropyl-4- { [(2,4,6-triisopropyl-phenyl)-acetyl]sulfamoyloxy}-phenyl)-ureido]-propionic acid ethyl ester;
3-[3-(3,5-Diisopropyl-4-{[(2,4,6-triisopropyl-phenyl)-acetyl]sulfamoyloxy}-phenyl)-ureido]-propionic acid;
[(2,4,6-Triisopropyl-phenyl)-acetyl]-sulfamic acid 4-[2-amino-3-( 1H-indol-3-yl)-propionylamino]-2,6-diisopropyl-phenyl ester;
[(2,4,6-Triisopropyl-phenyl)-acetyl]-sulfamic acid 4-(5-amino-pentanoylamino)-2,6-diisopropyl-phenyl ester trifluoroacetate(1:1)(salt);
(D)-[(2,4,6-Triisopropyl-phenyl)-acetyl]-sulfamic acid 4-(2-amino-propionylamino)-2,6-diisopropyl-phenyl ester trifluoroacetate(1:1)(salt);
(L)-[(2,4,6-Triisopropyl-phenyl)-acetyl]-sulfamic acid 4-(2-amino-propionylamino)-2,6-diisopropyl-phenyl ester;
[(2,4,6-Triisopropyl-phenyl)-acetyl]-sulfamic acid 4-(2-amino-2-methyl-propionylamino)-2,6-diisopropyl-phenyl ester;
[(2,4,6-Triisopropyl-phenyl)-acetyl]-sulfamic acid 4-(3-dimethylamino-propoxy)-2,6-diisopropyl-phenyl ester;
[(2,4,6-Triisopropyl-phenyl)-acetyl]-sulfamic acid 4-(3-dimethylamino-propoxy)-2,6-diisopropyl-phenyl ester hydrochloride salt;
[(2,4,6-Triisopropyl-phenyl)-acetyl]-sulfamic acid 4-(3-amino-propoxy)-2,6-diisopropyl-phenyl ester hydrochloride salt;
[(2,4,6-Triisopropyl-phenyl)-acetyl]-sulfamic acid 2,6-diisopropyl-4-thiocyanato-phenyl ester;
[(2,4,6-Triisopropyl-phenyl)-acetyl]-sulfamic acid 4-cyano-2,6-diisopropyl-phenyl ester;
[(2,4,6-Triisopropyl-phenyl)-acetyl]-sulfamic acid 4-[(2-amino-acetylamino)-methyl]-2,6-diisopropyl-phenyl ester;
[(2,4,6-Triisopropyl-phenyl)-acetyl]-sulfamic acid 4-(benzylaminomethyl)-2,6-diisopropyl-phenyl ester mono hydrochloride;
[(2,4,6-Triisopropyl-phenyl)-acetyl]-sulfamic acid 4-carbamoyl-2,6-diisopropyl-phenyl ester;
[(2,4,6-Triisopropyl-phenyl)-acetyl]-sulfamic acid 4-hydroxymethyl-2,6-diisopropyl-phenyl ester;
[(2,4,6-Triisopropyl-phenyl)-acetyl]-sulfamic acid 4-acetylamino-2,6-diisopropyl-phenyl ester;
[(2,4,6-Triisopropyl-phenyl)-acetyl]-sulfamic acid 4-(2-hydroxyethylamino)-2,6-diisopropyl-phenyl ester;
[(2,4,6-Triisopropyl-phenyl)-acetyl]-sulfamic acid 4-[3-(2,6-diisopropyl-phenyl)-ureido]-2,6-diisopropyl-phenyl ester;
[(2,4,6-Triisopropyl-phenyl)-acetyl]-sulfamic acid 2,6-diisopropyl-4-(3-phenyl-ureido]-phenyl ester;
[(2,4,6-Triisopropyl-phenyl)-acetyl]-sulfamic acid 2,6-diisopropyl-4-(thiophene-2-sulfonylamino)-phenyl ester;
[(2,4,6-Triisopropyl-phenyl)-acetyl]-sulfamic acid 4-(5-dimethylamino-naphthalene-1-sulfonylamino)- 2,6-diisopropyl-phenyl ester;
[(2,4,6-Triisopropyl-phenyl)-acetyl]-sulfamic acid 2,6-diisopropyl-4-methanesulfonylamino-phenyl ester;
6-(3,5-Diisopropyl-4-{[(2,4,6-triisopropyl-phenyl-acetyl]sulfamoyloxy}-phenyl)-hexanoic acid ethyl ester; and
6-(3,5-Diisopropyl-4-{[(2,4,6-triisopropyl-phenyl-acetyl]sulfamoyloxy}-phenyl)-hexanoic acid.

7. The use according to Claim 1, wherein the sulfonylaminocarbonyl derivative is a compound, or a pharmaceutically acceptable salt thereof, selected from:
(9H-Xanthene-9-carbonyl)-sulfamic acid 2,6-diisopropyl-phenyl ester;
((E)-2-Methyl-3-phenyl-acryloyl)-sulfamic acid 2,6-diisopropyl-phenyl ester; and
(2-Oxo-2H-chromene-3-carbonyl)-sulfamic acid 2,6-diisopropyl-phenyl ester.

8. The use according to Claim 1, wherein the sulfonylaminocarbonyl derivative is a compound, or a pharmaceutically acceptable salt thereof, selected from:
Carbamic acid, [(phenylamino)sulfonyl]-, 2,6-bis(1-methylethyl)phenyl ester;
Carbamic acid, [(phenylamino)sulfonyl]-, 2,6-bis(1,1-dimethylethyl)-4-hydroxyphenyl ester;
Carbamic acid, [(phenylamino)sulfonyl]-, 2,6-bis(1,1-dimethylethyl)phenyl ester;
Carbamic acid, [(didecylamino)sulfonyl]-, 2,6-bis(1,1-dimethylethyl)-4-methylphenyl ester;
Carbamic acid, [[bis(1-methylethyl)amino]sulfonyl]-, 2,6-bis(1-methylethyl)phenyl ester;
Carbamic acid, [(dipentylamino)sulfonyl]-, 2,6-bis(1-methylethyl)phenyl ester;
Carbamic acid, [[(diphenylmethyl)amino]sulfonyl]methyl-, 2,6-bis(1,1-dimethylethyl)phenyl ester;
DL-Tryptophan, α-methyl-N-[[[(tricyclo[3.3.1.1^{3,7}]dec-2-yloxy)carbonyl]amino]sulfonyl]-, methyl ester;
Carbamic acid, sulfonylbis-, bis[2,6-bis(1-methylethyl)phenyl] ester;
Carbamic acid, [[[2-(phenylmethyl)phenyl]amino]sulfonyl]-, 2,6-bis(1,1-dimethylethyl)phenyl ester; Methyl[[2,6-bis(1-methylethyl)phenyl amino]sulfonyl]carbamate; Dodecyl[[2,6-bis(1-methylethyl)phenyl]amino]sulfonyl]carbamate; 2,6-Bis(1,1-dimethylethyl)-4-methoxyphenyl [[(2,2-diphenylethyl)-amino]sulfonyl]carbamate;
2,6-Bis(1,1-dimethylethyl)-4-methoxy phenyl [[[2,6-bis(1-methylethyl)phenyl]amino]sulfonyl]carbamate;
2,6-Bis(1,1-dimethylethyl)phenyl-[[(diphenylmethyl)amino]-sulfonyl]carbamate;
2,6-Bis(1,1-dimethylethyl)phenyl [[[2,6-bis(1-methylethyl)phenyl]-amino]sulfonyl] carbamate;
2,6-Bis(1,1-dimethylethyl)phenyl [[(2,2-diphenylethyl)amino]-sulfonyl]carbamate;
2,6-Bis(1,1-dimethylethyl)phenyl [[bis(phenylmethyl)amino]-sulfonyl]carbamate;
2,6-bis(1-methylethyl)phenyl[(diphenyl-amino)sulfonyl]carbamate;
2,6-Bis(1-methylethyl)phenyl[(dibutyl-amino)sulfonyl]carbamate;
2,6-Bis(1-methylethyl)phenyl[[bis(phenyl-methyl)amino]sulfonyl]-carbamate;
2,6-Bis(1-methylethyl)phenyl[(1H-benzimidazol-2-ylamino)-sulfonyl]carbamate;
2,6-Bis(1-methylethyl)phenyl [[2,2-diphenylethyl)amino]sulfonyl]-carbamate;
2,6-Bis(1-methylethyl)phenyl[[[2,6-bis( 1-methylethyl)phenyl]-amino]sulfonyl]carbamate;
2,6-Bis(1-methylethyl)phenyl[[(diphenyl-methyl)amino]sulfonyl]-carbamate;
2,6-Bis(1,1-dimethylethyl)-4-methyl-phenyl[[(diphenylmethyl)-amino]sulfonyl]carbamate;
2,6-Bis(1,1-dimethylethyl)-4-methyl-phenyl[[[bis(2,6-bis(1-methylethyl)phenyl]amino]sulfonyl]carbamate;
2,6-Bis(1,1-dimethylethyl)-4-methyl-phenyl[[(2,2-diphenylethyl)-amino] sulfonyl]-carbamate;
2,6-Bis(1,1-dimethylethyl)-4-methyl-phenyl[(dibutylamino)-sulfonyl]carbamate;
2,6-Bis(1,1-dimethylethyl)-4-methyl-phenyl [(dipentylamino)-sulfonyl]carbamate;
2,6-Bis(1,1-dimethylethyl)-4-methyl-phenyl [[bis( 1-methylethyl)amino]sulfonyl]carbamate;
2,6-Bis( 1,1-dimethylethyl)-4-methyl-phenyl [(dihexylamino)-sulfonyl]carbamate;
2,6-Bis(1,1-dimethylethyl)-4-methyl-phenyl [(hexylamino)-sulfonyl]carbamate;
2,6-Bis(1,1-dimethylethyl)-4-methyl-phenyl[[methyl(2-phenylethyl)amino]sulfonyl]carbamate;
2,6-Bis( 1,1-dimethylethyl)-4-methyl-phenyl[[[bis-3-(dimethylamino)propyl]amino]-sulfonyl]carbamate;
2,6-Bis(1,1-dimethylethyl)-4-methyl-phenyl[(methyl octyl amino)-sulfonyl]carbamate;
2,6-Bis( 1,1-dimethylethyl)-4-methyl-[[bis[(tetrahydro-2-furanyl)methyl]amino]sulfonyl]carbamate;
2,6-Bis(1,1-dimethylethyl)-4-methyl -phenyl[(dioctylamino)-sulfonyl]carbamate;
2,6-Bis(1,1-dimethylethyl)-4-methyl-phenyl[[[methyl 2-(2-pyridinyl)ethyl]amino]sulfonyl]carbamate, hydrochloride salt;
2,6-Bis(1,1-dimethylethyl)-4-methyl-phenyl[[[methyl 2-(2-pyridinyl)ethyl]amino]-sulfonyl]carbamate, sodium salt;
2,6-Bis(1,1-dimethylethyl)-4-methyl-phenyl[(dodecylamino)-sulfonyl]carbamate;
2,6-Bis(1-methylethyl)phenyl [[bis(1 -methylethyl)amino]sulfonyl]-carbamate;
2,6-Bis( 1-methylethyl)phenyl[[( 1-methylethyl)phenylmethyl)-amino]sulfonyl]carbamate;
2,6-Bis( 1-methylethyl)phenyl[(hexyl-amino)sulfonyl]carbamate;
2,6-Bis(1-methylethyl)phenyl [(dioctyl-amino)sulfonyl]carbamate;
2,6-Bis( 1-methylethyl)phenyl[[cyclo-hexyl(1-methylethyl)amino]-sulfonyl]carbamate;
2,6-Bis( 1-methylethyl)phenyl[(methyl-octylamino)sulfonyl]-carbamate;
2,6-Bis(1-methylethyl)phenyl [(dihexyl-amino)sulfonyl]carbamate;
Dodecyl [[(2,4,6-trimethoxyphenyl)amino]-sulfonyl]carbamate;
2,6-Bis(1-methylethyl)phenyl ester(4-morpholinylsulfonyl)-carbamic acid;
2,6-Bis(1-methylethyl)phenyl ester( 1-piperidinylsulfonyl)carbamic acid;
2,6-Bis(1-methylethyl)phenyl ester(1-pyrrolidinylsulfonyl)-carbamic acid;
2,6-Bis(1-methylethyl)phenyl ester[(2,3-dihydro-1H-indol-1-yl)sulfonyl]carbamic acid;
2,6-Bis(1-methylethyl)phenyl[(dibutylamino)sulfonyl]carbamate monosodium salt; and
2,6-Bis(1,1-dimethylethyl)phenyl[[(diphenylmethyl)amino]-sulfonyl]methyl carbamate .

9. The use according to Claim 1, wherein the sulfonylaminocarbonyl derivative is a compound, or a pharmaceutically acceptable salt thereof, selected from:
Urea, N-[2,6-bis(1-methylethyl)phenyl]-N'-[(dipropylamino)-sulfonyl]-;
Urea, N-(2,2-dimethyl-4-phenyl-1,3-dioxan-5-yl)-N'-[[(tricyclo[3.3.1.1^{3,7}]dec- 1-ylmethyl)amino]sulfonyl]-, (4S-*cis*)-;
Urea, N-(2,2-dimethyl-4-phenyl-1,3-dioxan-5-yl)-N'-[[(2,2-dimethyl-4-phenyl-1,3-dioxan-5-yl)amino]sulfonyl]-, stereoisomer;
N-[2,6-bis(1-methylethyl)phenyl]-N'-[[bis(1-methylethyl)amino]-sulfonyl]urea;
N-[2,6-bis(1-methylethyl)phenyl]-N'-[[(diphenylmethyl)amino]-sulfonyl]urea;
N-[2,6-bis(1-methylethyl)phenyl]-N'-[(diphenylamino)-sulfonyl]urea;
N-[2,6-bis(1-methylethyl)phenyl]-N'-[(dibutylamino)sulfonyl]urea;
N-[[[2,6-bis(1-methylethyl)phenyl]amino]-sulfonyl]-N'-(diphenylmethyl)urea;
N-[2,6-bis(1-methylethyl)phenyl]-N'-[[[2,6-bis(1-methylethyl)-phenyl]amino]sulfonyl]urea;
N-[2,6-bis(1-methylethyl)phenyl]-N'-[[(2,2-diphenylethyl)-amino]sulfonyl]urea;
N-[2,6-bis( 1-methylethyl)phenyl]-N'-[(9H-fluoren-9-ylamino)-sulfonyl]urea;
N-[2,6-bis( 1-methylethyl)phenyl]-N'-[[bis(phenylmethyl)amino]-sulfonyl]urea;
N-[2,6-bis( 1-methylethyl)phenyl]-N'-[[( 1-methylethyl)-(phenylmethyl)amino]sulfonyl]urea;
N-[2,6-bis(1-methylethyl)phenyl]-N'-[(dioctylamino)sulfonyl]urea;
N-[2,6-bis( 1-methylethyl)phenyl]-N'-[(4-phenyl-1-piperidinyl)-sulfonyl]urea;
N-[2,6-bis(1-methylethyl)phenyl]-N'-[(dihexylamino)sulfonyl]-urea;
N-[[bis[3-(dimethylamino)propyl]amino]-sulfonyl]-N'-[2,6-bis(1-methylethyl)phenyl]urea;
N-[2,6-bis(1-methylethyl)phenyl]-N'-[(hexylamino)sulfonyl]urea;
N-[2,6-bis(1-methylethyl)phenyl]-N'-[[bis-[(tetrahydro-2-furanyl)methyl]amino]sulfonyl]-urea;
N-[2,6-bis(1-methylethyl)phenyl]-N'-[(diethylamino)sulfonyl]urea;
N-[2,6-bis( 1-methylethyl)phenyl]-N'-[(methyloctyl amino)sulfonyl]urea;
N-[2,6-bis(1-methylethyl)phenyl]-N'-[[cyclohexyl(1-methylethyl)amino] sulfonyl]urea;
N-[2,6-bis(1-methylethyl)phenyl]-N'-[(dipentylamino)sulfonyl]-urea;
N-[2,6-bis(1-methylethyl)phenyl]-N'-[[bis(2-methylpropyl)amino]-sulfonyl]urea;
N-[2,6-bis(1-methylethyl)phenyl]-N'-[[ethyl(2-propenyl)amino]-sulfonyl]urea;
N-[[bis(3-methylbutyl)amino]sulfonyl]-N'-[2,6-bis( 1-methylethyl)-phenyl]urea;
N-[2,6-bis(1-methylethyl)phenyl]-N'-[(didecylamino)sulfonyl]urea;
N-[2,6-bis(1-methylethyl)phenyl]-N'-[(didodecylamino)-sulfamoyl]urea;
N-[2,6-bis(1-methylethyl)phenyl]-N'-[(diisopropylamino)-sulfonyl]urea;
N-[2,6-bis(1-methylethyl)phenyl]-N'-[(dicyclohexylamino)-sulfonyl]urea;
N-[2,6-bis(1-methylethyl)phenyl]-N'-[(methyloctadecylamino)-sulfonyl]urea;
N-[2,6-bis(1-methylethyl)phenyl]-N'-[(di-2-propenylamino)-sulfonyl]urea;
N-[2,6-bis(1-methylethyl)phenyl]-N'-[[(1,1-dimethylethyl)(1-methylethyl)amino]sulfonyl]-urea;
N-[2,6-bis(1-methylethyl)phenyl]-N'-[[bis( 1-methylpropyl)amino]-sulfonyl]urea;
N-[2,6-bis(1-methylethyl)phenyl]-N'-[(methyltetradecylamino)-sulfonyl]urea;
N-[2,6-bis(1-methylethyl)phenyl]-N'-(1-pyrrolidinylsulfonyl)urea;
N-[2,6-bis(1-methylethyl)phenyl]-N'-(1-piperidinylsulfonyl)urea;
N'-[[[2,6-bis(1-methylethyl)phenyl]amino]sulfonyl]-N,N-bis(phenylmethyl)urea;
N-[2,6-bis(1-methylethyl)phenyl]-N'-[(dibutylamino)sulfonyl]urea, monosodium salt; and
N'-[2,6-bis(1-methylethyl)phenyl]-N-methyl-[(dibutylamino)sulfonyl]urea.

10. The use according to Claim 1, wherein the sulfonylaminocarbonyl derivative is a compound, or a pharmaceutically acceptable salt thereof, selected from:
Sulfamic acid, [[[2,4,6-tris(1-methylethyl)phenyl]amino]-carbonyl]-, 2,6-bis(1-methylethyl)phenyl ester;
Sulfamic acid, [[[[1-[4-(dimethylamino)phenyl]cyclopentyl]-methyl]amino]carbonyl], 2,6-bis(1-methylethyl)phenyl ester;
(2,3-Dihydro-indole-1-carbonyl)-sulfamic acid 2,6-diisopropyl-phenyl ester;
Sulfamic acid, [[(triphenylmethyl)amino]carbonyl]-, 2,6-bis(1-methylethyl)phenyl ester;
Octadecyl [[[2,6-bis(1-methylethyl)phenyl]-amino]carbonyl]-sulfamate;
Dodecyl-N-[[[2,6-bis(1-methylethyl)phenyl]-amino]carbonyl]-sulfamate;
Decyl [[[2;6-bis(1-methylethyl)phenyl]amino]carbonyl]sulfamate;
(±) 1-Methylheptyl [[[2;6-bis(1-methylethyl)phenyl]amino]-carbonyl]sulfamate;
2;6-Bis(1-methylethyl)phenyl [[[2;6-bis(1-methylethyl)-phenyl]amino]carbonyl]sulfamate;
(±) 1-Methylundecyl [[[2;6-bis(1-methylethyl)phenyl]amino]-carbonyl]sulfamate; and
Dodecyl [[[2;6-bis(1-methylethyl)phenyl]amino]carbonyl]-sulfamate; sodium salt.

11. The use according to Claim 1, wherein the sulfonylaminocarbonyl derivative is a compound, or a pharmaceutically acceptable salt thereof, selected from:
Carbamic acid, [(dodecyloxy)sulfonyl]-, dodecyl ester;
Carbamic acid, [(dodecyloxy)sulfonyl]-,[1,1':3',1"-terphenyl]-2'-yl ester;
Carbamothioic acid, [(dodecyloxy)sulfonyl]-, S-[2,6-bis(1-methylethyl)-phenyl] ester;
Carbamic acid, (phenoxysulfonyl)-, 2,6-bis(1-methylethyl)phenyl ester;
Carbamic acid, [(2,6-dimethylphenoxy)sulfonyl]-, 2,6-bis(1-methylethyl)phenyl ester;
Carbamic acid, [[2,6-bis(1,1-dimethylethyl)phenoxy]sulfonyl]-, 2,6-bis(1,1-dimethylethyl)phenyl ester;
Carbamic acid, [[2,6-bis(1,1-dimethylethyl)phenoxy]sulfonyl]-, 2,6-bis(1-methylethyl)phenyl ester;
Carbamic acid, [(2,6-difluorophenoxy)sulfonyl]-, 2,6-bis(1-methylethyl)phenyl ester;
Carbamic acid, [(hexadecyloxy)sulfonyl]-, 2,6-bis(1-methylethyl)phenyl ester;
Carbamic acid, [[2,6-bis(1-methylethyl)phenoxy]sulfonyl]-, 2,6-dimethoxyphenyl ester;
Carbamic acid, [[2,6-bis(1-methylethyl)phenoxy]sulfonyl]-, 1-methylheptyl ester;
Carbamic acid, [[2,6-bis(1-methylethyl)phenoxy]sulfonyl]-, 2,6-bis(1-methylethyl)-4-nitrophenyl ester;
Carbamic acid, [[2,6-bis(1-methylethyl)phenoxy]sulfonyl]-, 1,2-ethanediyl ester;
Carbamic acid, [[2,6-bis(1-methylethyl)phenoxy]sulfonyl]-, 1,2,3-propanetriyl ester;
Carbamic acid, [[2,6-bis(1-methylethyl)phenoxy]sulfonyl]-, 4-bromo-2,6-bis(1-methylethyl)phenyl ester;
Carbamic acid, [[2,6-bis(1-methylethyl)phenoxy]sulfonyl]-, [1,1':3',1"-terphenyl]-2'-yl ester;
Carbamic acid, [[2,6-bis(1-methylethyl)phenoxy]sulfonyl]-, 2,6-bis(1,1-dimethylethyl)-4-methoxyphenyl ester;
Carbamic acid, [[2,6-bis(1-methylethyl)phenoxy]sulfonyl]-, 4-fluoro-2,3,5,6-tetrakis(1-methylethyl)phenyl ester;
Carbamic acid, [[2,6-bis(1-methylethyl)phenoxy]sulfonyl]-, 4-chloro-2,6-bis-(1-methylethyl)phenyl ester;
Stigmasta-5,22-dien-3-ol, [[2,6-bis(1-methylethyl)phenoxy]-sulfonyl]-carbamate, (3α)-;
Carbamic acid, [[2,6-bis(1-methylethyl)phenoxy]sulfonyl]-, 2,6-bis( 1,1dimethylethyl)-4-methylphenyl ester;
Stigmastan-3-ol, [[2,6-bis(1-methylethyl)phenoxy]sulfonyl]-carbamate, (3α)-;
Carbamic acid, [[2,6-bis(1-methylethyl)phenoxy]sulfonyl]-, 4-methoxy-2, 6-bis(1-methylethyl)phenyl ester;
Carbamic acid, [[2,6-bis(1-methylethyl)phenoxy]sulfonyl]-, 2,4,6-tris(1-methylethyl)phenyl ester;
Carbamic acid, [[2,4,6-tris(1-methylethyl)phenoxy]sulfonyl]-, 2,6-bis(1-methylethyl)phenyl ester;
Carbamic acid, [[2,4,6-tris(1-methylethyl)phenoxy]sulfonyl]-, 2,4,6-tris(1-methylethyl)phenyl ester;
Carbamic acid, [[2,6-bis(1-methylethyl)phenoxy]sulfonyl]-, 2,4,6-tris(1,1-dimethylethyl)phenyl ester;
Carbamic acid, [[2,6-bis(1-methylethyl)phenoxy]sulfonyl]-, 4-[[3,5-bis(1,1-dimethylethyl)-4-hydroxyphenyl]dithio]-2,6-bis( 1,1-dimethylethyl)phenyl ester;
Carbamic acid, [[2,6-bis(1-methylethyl)phenoxy]sulfonyl]-, 2,4-bis(1-methylethyl)phenyl ester;
Carbamic acid, [[2,6-bis(1-methylethyl)phenoxy]sulfonyl]-, 4-[(dimethylamino)methyl]-2,6-bis(1-methylethyl)phenyl ester;
Carbamic acid, [[2,6-bis(1-methylethyl)phenoxy]sulfonyl]-, tricyclo[3.3.1.1^{3,7}]dec-2-yl ester;
Carbamic acid, [[2,6-bis(1-methylethyl)phenoxy]sulfonyl]-, 4-hydroxy-2,6-bis(1-methylethyl)phenyl ester;
Carbamic acid, [[2,6-bis(1-methylethyl)phenoxy]sulfonyl]-, cyclohexyl ester;
Carbamic acid, [[2,6-bis(1-methylethyl)phenoxy]sulfonyl]-, 3,3',5,5'-tetrakis(1-methylethyl)[1,1'-biphenyl]-4,4'-diyl ester;
Carbamic acid, [[4-hydroxy-2,6-bis(1-methylethyl)phenoxy]-sulfonyl]-, 2,6-bis(1-methylethyl)phenyl ester;
Carbamic acid, [[2,6-bis(1-methylethyl)phenoxy]sulfonyl]-, tricyclo[3.3.1.1^{3,7}]dec-1-yl ester;
Carbamic acid, [[2,6-bis(1-methylethyl)phenoxy]sulfonyl]-, 2-(1,1-dimethylethyl)-6-methylphenyl ester;
Carbamic acid, [[2,6-bis(1-methylethyl)phenoxy]sulfonyl]-, 5-methyl-2-(1-methylethyl)cyclohexyl ester;
Carbamothioic acid, [[2,6-bis(1-methylethyl)phenoxy]sulfonyl]-, *S*-[2,6-bis(1-methylethyl)phenyl] ester;
Carbamic acid, [[2,6-bis(1-methylethyl)phenoxy]sulfonyl]-, (2,6-diethylphenyl)methyl ester;
Carbamic acid, [[2,6-bis(1-methylethyl)phenoxy]sulfonyl]-, (2*S*,6*S*)-2,6-bis(1-methylethyl)cyclohexyl ester;
Carbamic acid, [[2,6-bis(1-methylethyl)phenoxy]sulfonyl]-, 4-(1,1-dimethylethyl)-2, 6-bis(1-methylethyl)phenyl ester;
Carbamic acid, [[2,6-bis(1-methylethyl)phenoxy]sulfonyl]-, 4-fluorophenyl ester;
Carbamic acid, [[2,6-bis(1-methylethyl)phenoxy]sulfonyl]-, 2,4-difluorophenyl ester;
Carbamic acid, [[2,6-bis(1-methylethyl)phenoxy]sulfonyl]-, pentafluorophenyl ester;
Carbamic acid, [[2,6-bis(1-methylethyl)phenoxy]sulfonyl]-, 2,6-difluorophenyl ester;
Carbamic acid, [[2,6-bis(1-methylethyl)phenoxy]sulfonyl]-, (2*R*,6*S*)-2,6-bis(1-methylethyl)cyclohexyl ester;
Carbamic acid, [[2,6-bis(1-methylethyl)phenoxy]sulfonyl]-, 2,3,5,6-tetramethylphenyl ester;
Carbamic acid, [[2,6-bis(1-methylethyl)phenoxy]sulfonyl]-, 3-pyridinyl ester;
Carbamic acid, [[2,6-bis(1-methylethyl)phenoxy]sulfonyl]-, 2,6-dimethylphenyl ester;
Carbamic acid, [[2,6-bis(1-methylethyl)phenoxy]sulfonyl]-, 4-acetyl-2,6-bis(1-methylethyl)phenyl ester;
Carbamic acid, [[2,6-bis(1-methylethyl)phenoxy]sulfonyl]-, 4-fluoro-2,6-bis(1-methylethyl)phenyl ester;
2,6-Bis(1-methylethyl)phenyl[[2,6-bis(1-methylethyl)phenoxy]-sulfonyl]carbamate;
2,6-Bis( 1;I -dimethylethyl)-4-methylphenyl (phenoxysulfonyl)-carbamate;
2,6-Bis(1,1-dimethylethyl)-4-methylphenyl [(hexyloxy)-sulfonyl]carbamate;
2,6-Bis(1,1-dimethylethyl)-4-methylphenyl[(dodecyloxysulfonyl]carbamate;
Dodecyl[[2,6-bis(1-methylethyl)phenoxy]-sulfonyl]carbamate;
Methyl[[2,6-bis(1-methylethyl)phenoxy]-sulfonyl]carbamate;
2,6-Bis(1-methylethyl)phenyl[(hexyloxy)-sulfonyl]carbamate;
2,6-Bis(1-methylethyl)phenyl](dodecyloxy)-sulfonyl]carbamate; and
2,6-Bis(1,1-dimethylethyl)phenyl[[2,6-bis(1-methylethyl)-phenoxy]sulfonyl]carbamate .

12. The use according to Claim 1, wherein the sulfonylaminocarbonyl derivative is a compound, or a pharmaceutically acceptable salt thereof, selected from:
N-[2,6-bis(1-methylethyl)phenyl]-N'-(6-ethoxy-2-benzothiazolyl)-sulfonyl]-urea;
N-[2,6-bis(1-methylethyl)phenyl]-N'-(2-octadecylsulfonyl)urea;
N-[2,4,6-trimethoxyphenyl]-N'-(2-octadecylsulfonyl)urea;
N-[2,6-bis( 1-methylethyl)phenyl]-N'-(tetradecylsulfonyl)urea;
N-[2,6-bis(1-methylethyl)phenyl]-N'-(dodecylsulfonyl)urea;
N-[2,6-bis( 1-methylethyl)phenyl]-N'-(hexadecylsulfonyl)urea;
N-[2,6-bis(1-methylethyl)phenyl]-N'-methyl-N'-(dodecylsulfonyl)urea;
N-[2,6-bis( 1-methylethyl)phenyl]-N'-(tridecylsulfonyl)urea;
N-[2,4,6-trimethoxyphenyl]-N'-(hexadecylsulfonyl)urea;
N-[2,6-bis(1-methylethyl)phenyl]-N'-(2-methyl-2-pentadecylsulfonyl)urea;
N-[2,6-bis( 1-methylethyl)phenyl]-N'-( 1-phenyl-1-tetradecylsulfonyl)urea;
N-2,6-bis(1-methylethyl)phenyl-N'-(dodecylsulfonyl)urea;
N-[2,6-bis( 1-methylethyl)phenyl]-N'-( 1-phenyl-1-nonylsulfonyl)urea; and
N-[2,6-bis(1-methylethyl)phenyl]-N'-(2-decylsulfonyl)urea.

13. The use of Claim 1, wherein the disease or disorder being treated is rheumatoid arthritis, osteoarthritis, an autoimmune disease, psoriasis, asthma, a cardiovascular disease, an acute coronary syndrome, congestive heart failure, Alzheimer's disease, multiple sclerosis, cancer, type II diabetes, metabolic syndrome X, or inflammatory bowel disease.

14. The use according to Claim 13, wherein the disease or disorder being treated is selected from the group consisting of: systemic lupus erythematosus, Grave's disease, myasthenia gravis, insulin resistance, autoimmune hemolytic anemia, scleroderma with anticollagen antibodies, pernicious anemia, and diabetes mellitus.

15. The use according to Claim 13, wherein the disease or disorder being treated is rheumatoid arthritis .

16. The use according to Claim 13, wherein the disease or disorder being treated is osteoarthritis.

17. The use according to Claim 13, wherein the disease or disorder being treated is insulin resistance.

18. The use according to Claim 13, wherein the disease or disorder being treated is asthma.

19. The use according to Claim 13, wherein the disease or disorder being treated is atherosclerosis.

20. The use according to Claim 13, wherein the disease or disorder being treated is myocardial infarction ·

21. The use according to Claim 13, wherein the disease or disorder being treated is unstable angina.

22. The use according to Claim 13, wherein the disease or disorder being treated is congestive heart failure.

23. The use according to Claim 13, wherein the disease or disorder being treated is Alzheimer's disease,

24. The use according to Claim 13, wherein the disease or disorder being treated is cancer.

25. The use according to Claim 13, wherein the disease or disorder being treated is inflammatory bowel disease.

26. The use according to Claim 13, wherein the disease or disorder being treated is multiple sclerosis.

27. The use according to Claim 13, wherein the disease or disorder being treated is psoriasis.

28. The method according to Claim 13, wherein the disease or disorder being treated is type II diabetes.

29. The method according to Claim 13,wherein the disease or disorder being treated is metabolic syndrome X.

30. Use of a sulfonylaminocarbonyl derivative or a pharmaceutically acceptable salt thereof for preparing a pharmaceutical composition for inhibiting NF-κB transcription factors in an animal.

31. A pharmaceutical composition, comprising a nuclear factor-κB transcription factor inhibiting amount of a sulfonylaminocarbonyl derivative, or a pharmaceutically acceptable salt thereof and a pharmaceutically acceptable carrier.
